# EUROPÄISCHE PATENTSCHRIFT

(11) **EP 0 350 805 B1**
(45) Veröffentlichungstag und Bekanntmachung des Hinweises auf die Patenterteilung: **16.04.1997**
(21) Anmeldenummer: 89112429.9
(22) Anmeldetag: 07.07.1989
(51) Int. Cl.: C07D 311/08, C07D 311/22, C07D 311/68, C07D 405/04, C07D 413/04, C07D 417/04, C07D 493/04, A61K 31/50

(54) **Neue Benzopyran-Derivate, Verfahren zu ihrer Herstellung und ihre Verwendung sowie die Verbindungen enthaltenden Zubereitungen**
Benzopyran derivatives, processes for their preparation and their use and preparations containing the compounds
Dérivés benzopyraniques, procédé pour leur préparation et leur application, aussi bien que les préparations les contenant

(30) Priorität: 12.07.1988 DE 3823533
(43) Veröffentlichungstag der Anmeldung: 17.01.1990
(73) Patentinhaber: Beiersdorf-Lilly GmbH, 20253 Hamburg (DE)
(72) Erfinder: Stenzel, Wolfgang, Dr., D-2057 Reinbek (DE); Schotten, Theo, Dr., D-2000 Hamburg 20 (DE); Armah, Ben, Dr., D-2000 Hamburg 52 (DE)
(74) Vertreter: Hudson, Christopher Mark

(56) Entgegenhaltungen:
- EP-A- 0 028 064
- EP-A- 0 093 535
- EP-A- 0 107 423
- EP-A- 0 273 262
- JOURNAL OF MEDICINAL CHEMISTRY, Band 27, nr 9, 1984, Seiten 1127-1131.
- JOURNAL OF MEDICINAL CHEMISTRY, Band 29,Nr.11, 1986, Seiten2194-2201.

## Beschreibung

Einige 6-Nitril-substituierte Benzopyran-Derivate sind in EPA 0 344 747 beschrieben, und verwandte 6-Trifluormethoxy und 6-Trifluorethoxy Benzopyran-Derivate sind in EPA 0 314 446 beschrieben.

Gegenstand der Erfindung sind neue substituierte Benzopyran-Derivate der allgemeinen Formel I, 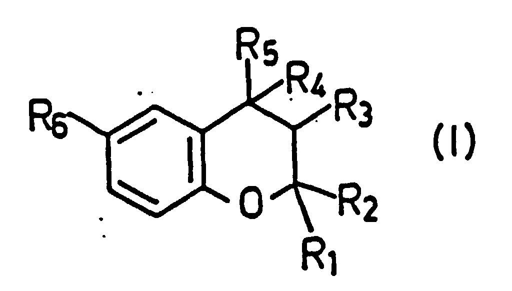 in der
R₁ und R₂, die gleich oder verschieden sein können, Wasserstoff, C₁₋₆-Alkyl, C₃₋₆-verzweigt-Alkyl,
C₃₋₇-Cycloalkyl oder
zusammen mit dem von ihnen eingeschlossenen
Kohlenstoffatom C₃₋₇-Spiroalkyl bedeuten,
R₃ Hydroxy, C₁₋₈-Alkoxy, Formyloxy, C₁₋₈-Alkylcarbonyloxy, C₁₋₈-Alkoxycarbonyloxy, C₁₋₈-Mono-alkylaminocarbonyloxy oder C₁₋₈-Dialkylamino-carbonyloxy bedeutet, wobei die C₁₋₈-Alkyl- oder -Alkoxygruppen sowohl linear als auch verzweigt sein können, und
R₄ für Wasserstoff steht oder
R₃ und R₄ zusammen eine Bindung bilden,
R₅ einen Heterocyclus der Formel A 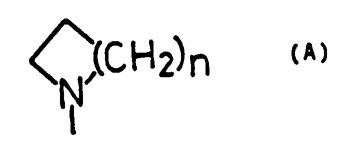 bedeutet, wobei n für 1, 2, 3 oder 4 steht,
oder einen Heterocyclus der Formel B 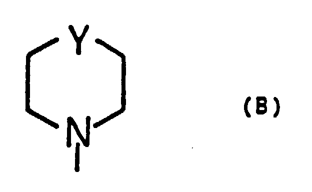 bedeutet, wobei Y für Sauerstoff, Schwefel, unsubstituiertes Amino -NH-, substituiertes Amino -NR₇- steht und R₇ geradkettiges C₁₋₉-Alkyl, verzweigtes C₃₋₇-Alkyl, C₃₋₇-Cycloalkyl, durch C₃₋₇-Cycloalkyl substituiertes geradkettiges oder verzweigtes C₁₋₉-Alkyl, C₁₋₈-Alkylcarbonyl, C₁₋₈-Alkoxycarbonyl, Benzyl, Triphenylmethyl, Phenyl, Benzyloxycarbonyl, Phenylcarbonyl oder Benzylcarbonyl bedeutet,
oder R₅ einen Heterocyclus der Formel C 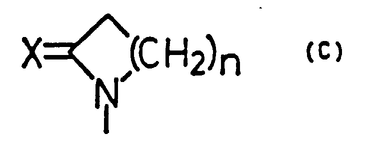 bedeutet, wobei X für Sauerstoff oder Schwefel steht und n für 1, 2, 3 oder 4 steht,
oder einen Heterocyclus der Formel D 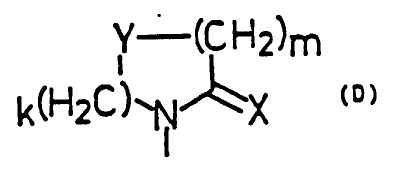 bedeutet, wobei m 0, 1 oder 2 und k 1, 2 oder 3 bedeutet, jedoch so, daß (m + k) 1, 2 oder 3 ist und weiterhin X und Y die für die Formeln B und C angegebene Bedeutung haben,
oder einen Heterocyclus der Formel E 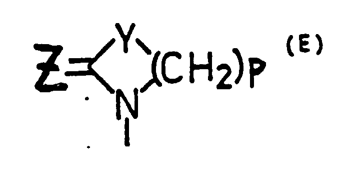 bedeutet, wobei Z für Cyanimino N-CN, cis oder trans Nitromethyliden (c/t) CH-NO₂ oder Nitroimino N-NO₂ steht, Y die oben angegebene Bedeutung hat und p 2 oder 3 bedeutet, und
R₆ in Abhängigkeit von R₅ für die zwei Klassen von Substituenten R₆' und R₆" steht, wobei, falls R₅ einen Heterocyclus A, B, C oder D bedeutet, R₆ für die Substituentenklasse R₆' steht und
R₆' Difluormethoxy, Trifluormethoxy, Trifluorethoxy, Tetrafluorethoxy, Difluormethylthio, Difluormethylsulfinyl, Difluormethylsulfonyl, Trifluormethylthio, Trifluormethylsulfinyl, Trifluormethylsulfonyl, Trifluorethylthio, Trifluorethylsulfinyl oder Trifluorethylsulfonyl bedeutet,
oder, falls R₅ den Heterocyclus E bedeutet, R₆ für die vorstehende Substituentenklasse R₆' und für die Substituentenklasse R₆'' steht und
R₆'' Cyano, Nitro, C₁₋₆-Alkyl, C₃₋₈-Cycloalkyl, Formyl und C₁₋₆-Alkylcarbonyl bedeutet, wobei sich der Heterocyclus R₅ in trans-Stellung zu dem Rest R₃ befindet, wenn R₃ und R₄ nicht zusammen eine Bindung bedeuten, sondern R₄ für Wasserstoff steht, sowie deren Salze und Säureadditionssalze, Tautomere und optische Isomere,
ausgenommen Verbindungen der Formel (I), bei denen zugleich R₁ und R₂ beide Methyl, R₃ Hydroxyl, R₅ einen heterocyclichen Rest der Formel (C), X Sauerstoff, n 2, 3 oder 4 und R₆ Trifluormethoxy oder Trifluorethoxy bedeuten,
und ausgenommen Verbindungen der Formel (I), bei denen zugleich R₁ und R₂ C₁₋₆-Alkyl, oder C₃₋₆-verzweigtes Alkyl, R₃ Hydroxy, R₄ Wasserstoff oder R₃ und R₄ zusammen eine Bindung, R₅ einen Heterocyclus der Formel (E), worin Y für Schwefel, Amino oder substituiertes Amino mit R₇ in der Bedeutung c₁₋₆-Alkyl oder verzweigtes C₃₋₆-Alkyl und Z für Cyanimino stehen, und R₆ Cyano bedeuten,
Verfahren zu ihrer Herstellung, ihre
Verwendung und Zubereitungen, die diese Verbindungen enthalten.

Der Einfachheit halber sind die erfindungsgemäßen Verbindungen in nur einer durch Formel I wiedergegebenen tautomeren Form definiert. Die Erfindung erstreckt sich jedoch auf alle tautomeren Formen der Verbindungen.

Obgleich pharmazeutisch verträgliche Salze und Säureadditionssalze der neuen Verbindungen der Formeln I und deren tautomere Formen bevorzugt sind, liegen alle Salze innerhalb des Bereichs der Erfindung. Alle Salze sind wertvoll zur Herstellung der Verbindungen, selbst wenn das spezielle Salz nur als Zwischenprodukt gewünscht wird, wie zum Beispiel, wenn das Salz nur für Zwecke der Reinigung oder Identifizierung gebildet wird, oder wenn es als ein Zwischenprodukt bei der Herstellung eines pharmazeutisch verträglichen Salzes, beispielweise durch Ionenaustauschverfahrensweise, verwendet wird.

Verbindungen der allgemeinen Formel I und deren Salze und Säureadditionssalze enthalten asymmetrische Kohlenstoff- und Schwefelatome. Daher sind auch die verschiedenen optischen Isomeren sowie Diasteromeren Gegenstand der Erfindung. Die Racemate können nach an sich bekannten Methoden in ihre optischen Antipoden aufgetrennt werden.

Gegenstand der Erfindung sind auch die neuen Verbindungen der Formeln IIa, IIIa und Va 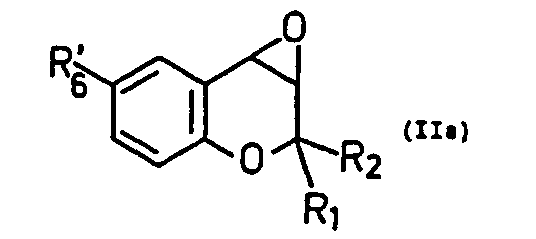 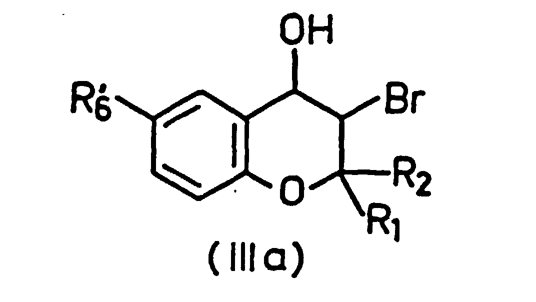 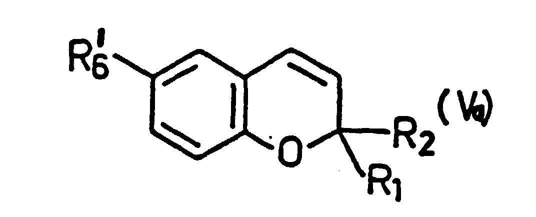 mit den vorstehend angegebenen Bedeutungen für R₁, R₂ und R₆', ausgenommen Verbindungen der Formel (IIa), bei denen zugleich R₁ und R₂ beide Methyl und R₆' Trifluormethoxy oder Trifluorethoxy bedeuten, und die Verfahren zu ihrer Herstellung. Sie dienen als Vorprodukte oder Zwischenprodukte zur Herstellung der erfindungsgemäßen Endprodukte.

Mit den Verbindungen der vorliegenden Erfindung strukturell verwandte Verbindungen sind in dem US-Patent 4,251,537, den Europäischen Patentschriften EP 0 076 075, EP 0 107 423 und im Journal of Medicinal Chemistry 26, 1582 (1983), 27, 1127 (1984) und 29, 2194 (1986) beschrieben. Die Verbindungen der vorliegenden Erfindung werden aber weder spezifisch offenbart noch nahegelegt.

Die erfindungsgemäßen Verbindungen der Formel I zeichnen sich insbesondere durch eine deutlich höhere Wirkstärke bei deutlich verlängerter Wirkdauer gegenüber den bekannten Verbindungen aus.

Soweit nicht anders angegeben , können die erfindungsgemäßen Alkylgruppen und Alkylteile beziehungsweise Alkylenteile von Gruppen geradkettig oder verzweigt sein und sie besitzen jeweils bevorzugt 1 bis 6 Kohlenstoffatome, vorzugsweise 1 bis 4 Kohlenstoffatome, insbesondere 1 oder 2 Kohtenstoffatome. Die verzweigten Alkylgruppen besitzen mindestens 3 Kohlenstoffatome. Bevorzugte Alkyl- oder Alkylenteile sind Methyl, Ethyl, n-Propyl, Isopropyl, Butyl beziehungsweise entsprechend Methylen, Ethylen, n- oder Isopropylen und Butylen.

Vorzugsweise besitzen erfindungsgemäße Cycloalkylgruppen und Cycloalkylteile wie Cycloalkylreste von Cycloalkylalkylgruppen 3 bis 7 Kohlenstoffatome, insbesondere 3 bis 6 Kohlenstoffatome. Besonders bevorzugt sind Cyclopropyl und Cyclohexyl.

Formyl ist HCO-, Formyloxy ist HCOO-, C₁₋₈-Alkylcarbonyloxy ist C₁₋₈-Alkyl-CO-O-, C₁₋₈-Alkyloxycarbonyloxy ist C₁₋₈-Alkyl-O-CO-O-, C₁₋₈-Monoalkylaminocarbonyloxy ist C₁₋₈-Alkyl-NH-CO-O-, C₁₋₈-Dialkylaminocarbonyloxy ist (C₁₋₈-Alkyl)₂-N-CO-O-. C₁₋₈-Alkylcarbonyl ist C₁₋₈-Alkyl-CO-, C₁₋₆-Alkylcarbonyl ist C₁₋₆-Alkyl-CO-, C₁₋₈-Alkoxycarbonyl ist C₁₋₈-Alkoxy-CO-.

Die Trifluorethylgruppe oder Trifluorethyl als Teil anderer erfindungsgemäßer Reste wie Trifluorethoxy ist vorzugsweise 2,2,2-Trifluorethyl.

Die Tetrafluorethylgruppe oder Tetrafluorethyl als Teil anderer erfindungsgemäßer Reste wie Tetrafluorethoxy ist vorzugsweise 2,2',1,1'-Tetrafluorethyl.

C₃₋₇-Cycloalkyl-substituiertes C₁₋₉-Alkyl ist vorzugsweise Cyclopropylmethyl.

R₁ ist bevorzugt Wasserstoff, Methyl oder Ethyl, davon besonders bevorzugt Methyl.

R₂ ist bevorzugt Wasserstoff, Methyl oder Ethyl, davon besonders bevorzugt Methyl.

R₁ und R₂ sind besonders bevorzugt beide zugleich Methyl.

Falls R₁ und R₂ für bevorzugtes verzweigt-Alkyl oder Cycloalkyl steht, sind Isopropyl bzw. Cyclopropyl besonders bevorzugt.

Falls R₁ und R₂ zusammen mit dem von ihnen eingeschlossenen Kohlenstoffatom einen Spiroalkylring bilden, sind Spirocyclopentyl und Spirocyclohexyl bevorzugt.

R₃ steht bevorzugt für Hydroxy oder bildet besonders bevorzugt zusammen mit R₄ eine Bindung, so daß zwischen C₃- und C₄-Position des Benzopyrangerüstes eine Doppelbindung besteht. Die erfindungsgemäßen Verbindungen mit dieser C₃=C₄-Doppelbindung werden ganz besonders bevorzugt.

R₇ ist vorzugsweise C₃₋₇-cycloalkyl-substituiertesgeradkettiges oder verzweigtes C₁₋₉-Alkyl.

Falls R₃ für Alkoxy steht, sind Ethoxy und besonders Methoxy bevorzugt.

Falls R₃ für Alkylcarbonyloxy steht, sind Propionyloxy und besonders Acetoxy und Formyloxy bevorzugt.

R₅ ist bevorzugt ein Heterocyclus der Formel A, C, D und E, davon bevorzugt sind insbesondere C, D, und E, mit den jeweils oben angegebenen Bedeutungen für n, Y, X, m, k, p und Z.

Davon besonders bevorzugt sind Verbindungen in denen:
C für 2-Oxopyrrolidinyl und insbesondere 2-Oxopiperidinyl steht,
D für 2-Oxo-3-oxazolidinyl, 2-Oxomorpholinyl und insbesondere 2-Oxopiperazinyl und
2-Oxohexahydropyrimidinyl steht, wobei das für Y stehende Stickstoffatom sowohl unsubstituiert als auch mit R₇ in der oben angegebenen Bedeutung substituiert sein kann, wobei von allen Resten der Formel D unsubstituiertes oder durch R₇ in der oben angegebenen Bedeutung substituiertes 2-Oxopiperazinyl am meisten bevorzugt ist, und
E für 2-Cyaniminoimidazolyl steht, wobei das für Y stehende Stickstoffatom sowohl unsubstituiert als auch in der oben angegebenen Weise mit R₇ substituiert sein kann, oder
E insbesondere für 2-Cyaniminothiazolyl steht.

Falls R₆ die Bedeutung R₆' hat, sind Difluormethoxy, Trifluormethoxy, Trifluormethylthio, Difluormethylthio, Difluormethylsulfonyl, Trifluormethylsulfonyl, Trifluorethoxy und Tetrafluorethoxy bevorzugt, davon besonders bevorzugt sind Difluormethoxy, Trifluormethoxy, Trifluormethylthio, Difluormethylthio, Difluormethylsulfonyl, Trifluormethylsulfonyl, insbesondere Trifluormethoxy, Trifluormethylthio, Difluormethylsulfonyl, Trifluormethylsulfonyl, Difluormethylthio.

Falls R₆ die Bedeutung R₆" hat, ist Cyano, C₁₋₆-Alkyl, Acetyl, C₃₋₈-Cycloaklyl bevorzugt, davon sind Cyano, Methyl, Ethyl, Propyl und iso-Propyl, Cyclopropyl, Acetyl besonders bevorzugt.

Besonders bevorzugt werden Verbindungen der Formeln Ia, Ia', Ib, Ib', Ic und Ic': 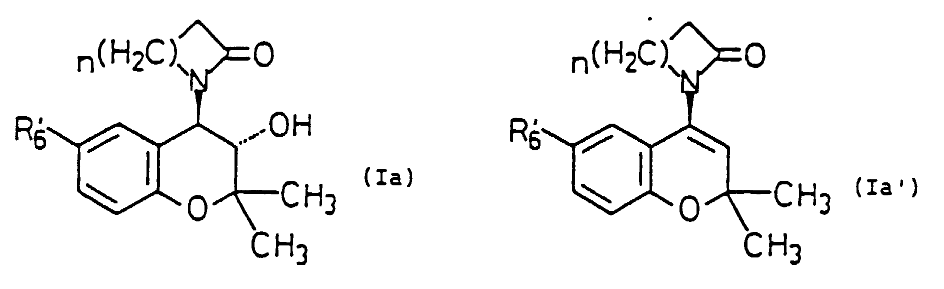 worin n die Zahl 2 oder 3 und R₆' Difluormethoxy, Trifluormethylthio, Difluormethylthio, Trifluormethylsulfonyl, Difluormethylsulfonyl, und Tetrafluorethoxy bedeutet, 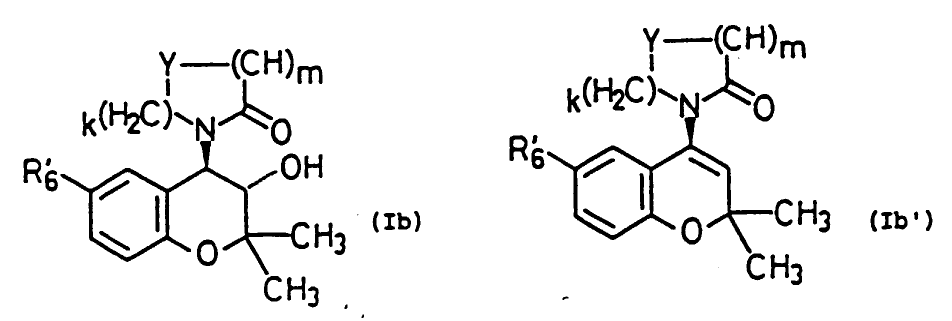 worin m 0 oder 1, k 2, Y O, NH, NR₇ und R₆' Difluormethoxy, Trifluormethoxy, Trifluormethylthio, Difluormethylthio, Trifluormethylsulfonyl, Difluormethysulfonyl, Trifluorethoxy und Tetrafluorethoxy bedeutet, 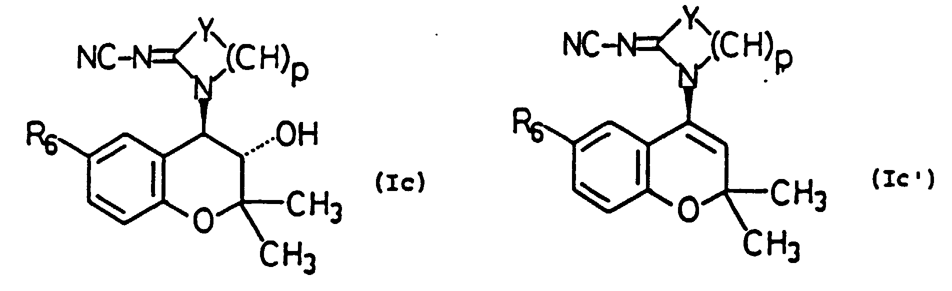 worin p 2, Y NH, NR₇, S und R₆ Difluormethoxy, Trifluormethoxy, Trifluormethylthio, Difluormethylthio, Trifluormethylsulfonyl, Difluormethylsulfonyl, Trifluorethoxy und Tetrafluorethoxy, C₁₋₆-Alkyl, Acetyl, C₃₋₈-Cycloalkyl bedeutet.

Die folgenden erfindungsgemäßen Verbindungen, deren Salze und Säureadditionssalze, Tautomere und optische Isomere werden bevorzugt:
1. 6-Difluormethylthio-3,4-dihydro-2,2-dimethyl-trans-4-(2-oxo-1-pyrrolidinyl)-2H-benzo[b]pyran-3-ol
2. 6-Difluormethylsulfinyl-3,4-dihydro-2,2-dimethyl-trans-4-(2-oxo-1-pyrrolidinyl)-2H-bemo[b]pyran-3-ol
3. 6-Difluormethylsulfonyl-3,4-dihydro-2,2-dimethyl-trans-4-(2-oxo-1-pyrrolidinyl)-2H-benzo[b]pyran-3-ol
4. 6-Trifluormethylthio-3,4-dihydro-2,2-dimethyl-trans-4-(2-oxo-1-pyrrolidinyl)-2H-benzo[b]pyran-3-ol
5. 6-Trifluormethylsulfinyl-3,4-dihydro-2,2-dimethyl-trans-4-(2-oxo-1-pyrrolidinyl)-2H-benzo[b]pyran-3-ol
6. 6-Trifluormethylsulfonyl-3,4-dihydro-2,2-dimethyl-trans-4-(2-oxo-1-pyrrolidinyl)-2H-benzo[b]pyran-3-ol
7. 6-(2,2,2-Trifluorethylthio)-3,4-dihydro-2,2-dimethyl-trans-4-(2-oxo-1-pyrrolidinyl)-2H-benzo[b]pyran-3-ol
8. 6-(2,2,2-Trifluorethylsulfinyl)-3,4-dihydro-2,2-dimethyl-trans-4-(2-oxo-1-pyrrolidinyl)-2H-benzo[b]pyran-3-ol
9. 6-(2,2,2-Trifluorethylsulfonyl)-3,4-dihydro-2,2-dimethyl-trans-4-(2-oxo-1-pyrrolidinyl)-2H-benzo[b]pyran-3-ol
10. 6-Difluormethoxy-3,4-dihydro-2,2-dimethyl-trans-4-(2-oxo-1-pyrrolidinyl)-2H-benzo[b]pyran-3-ol
11. 6-(1,1,2,2-Tetrafluorethoxy)-3,4-dihydro-2,2-dimethyl-trans-4-(2-oxo-1-pyrrolidinyl)-2H-benzo[b]pyran-3-ol
12. 6-Difluormethylthio-3,4-dihydro-2,2-dimethyl-trans-4-(2-oxo-1-piperidinyl)-2H-benzo[b]pyran-3-ol
13. 6-Difluormethylsulfinyl-3,4-dihydro-2,2-dimethyl-trans-4-(2-oxo-1-piperidinyl)-2H-benzo[b]pyran-3-ol
14. 6-Difluormethylsulfonyl-3,4-dihydro-2,2-dimethyl-trans-4-(2-oxo-1-piperidinyl)-2H-benzo[b]pyran-3-ol
15. 6-Trifluormethylthio-3,4-dihydro-2,2-dimethyl-trans-4-(2-oxo-1-piperidinyl)-2H-benzo[b]pyran-3-ol
16. 6-Trifluormethylsulfinyl-3,4-dihydro-2,2-dimethyl-trans-4-(2-oxo-1-piperidinyl)-2H-benzo[b]pyran-3-ol
17. 6-Trifluormethylsulfonyl-3,4-dihydro-2,2-dimethyl-trans-4-(2-oxo-1-piperidinyl)-2H-benzo[b]pyran-3-ol
18. 6-(2,2,2-Trifluorethylthio)-3,4-dihydro-2,2-dimethyl-trans-4-(2-oxo-1-piperidinyl)-2H-benzo[b]pyran-3-ol
19. 6-(2,2,2-Trifluorethylsulfinyl)-3,4-dihydro-2,2-dimethyl-trans-6-(2-oxo-1-piperidinyl)-2H-benzo[b]pyran-3-ol
20. 6-(2,2,2-Trifluorethylsulfonyl)-3,4-dihydro-2,2-dimethyl-trans-4-(2-oxo-1-piperidinyl)-2H-benzo[b]pyran-3-ol
21. 6-Difluormethoxy-3,4-dihydro-2,2-dimethyl-trans-4-(2-oxo-1-piperidinyl)-2H-benzo[b]pyran-3-ol
22. 6-(1,1,2,2-Tetrafluorethoxy)-3,4-dihydro-2,2-dimethyl-trans-4-(2-oxo-1-piperidinyl)-2H-benzo[b]pyran-3-ol
23. 6-Difluormethylthio-2,2-dimethyl-4-(2-oxo-1-pyrrolidinyl)-2H-benzo[b]pyran
24. 6-Difluormethylsulfinyl-2,2-dimethyl-4-(2-oxo-1-pyrrolidinyl-)-2H-benzo[b]pyran
25. 6-Difluormethylsulfonyl-2,2-dimethyl-4-(2-oxo-1-pyrrolidinyl)-2H-benzo[b]pyran
26. 6-Trifluormethylthio-2,2-dimethyl-4-(2-oxo-1-pyrrolidinyl)-2H-benzo[b]pyran
27. 6-Trifluormethylsulfinyl-2,2-dimethyl-4-(2-oxo-1-pyrrolidinyl)-2H-benzo[b]pyran
28. 6-Trifluormethylsulfonyl-2,2-dimethyl-4-(2-oxo-1-pyrrolidinyl)-2H-benzo[b]pyran
29. 6-(2,2,2-Trifluorethylthio)-2,2-dimethyl-4-(2-oxo-1-pyrrolidinyl)-2H-benzo[b]pyran
30. 6-(2,2,2-Trifluorethylsulfinyl)-2,2-dimethyl-4-(2-oxo-1-pyrrolidinyl)-2H-benzo[b]pyran
31. 6-(2,2,2-Trifluorethylsulfonyl)-2,2-dimethyl-4-(2-oxo-1-pyrrolidinyl)-2H-benzo[b]pyran
32. 6-Difluormethoxy-2,2-dimethyl-4-(2-oxo-1-pyrrolidinyl)-2H-benzo[b]pyran
33. 6-Trifluormethoxy-2,2-dimethyl-4-(2-oxo-1-pyrrolidinyl)-2H-benzo[b]pyran
34. 6-(2,2,2-Trifluorethoxy)-2,2-dimethyl-4-(2-oxo-1-pyrrolidinyl)-2H-benzo[b]pyran
35. 6-(1,1,2,2-Tetrafluorethoxy)-2,2-dimethyl-4-(2-oxo-1-pyrrolidinyl)-2H-benzo[b]pyran
36. 6-Difluormethylthio-2,2-dimethyl-4-(2-oxo-1-piperidinyl)-2H-benzo[b]pyran
37. 6-Difluormethylsulfinyl-2,2-dimethyl-4-(2-oxo-1-piperidinyl)-2H-benzo[b]pyran
38. 6-Difluormethylsulfonyl-2,2-dimethyl-4-(2-oxo-1-piperidinyl)-2H-benzo[b]pyran
39. 6-Trifluormethylthio-2,2-dimethyl-4-(2-oxo-1-piperidinyl)-2H-benzo[b]pyran
40. 6-Trifluormethylsulfinyl-2,2-dimethyl-4-(2-oxo-1-piperidinyl)-2H-benzo[b]pyran
41. 6-Trifluormethylsulfonyl-2,2-dimethyl-4-(2-oxo-1-piperidinyl)-2H-benzo[b]pyran
42. 6-(2,2,2-Trifluorethylthio)-2,2-dimethyl-4-(2-oxo-1-piperidinyl)-2H-benzo[b]pyran
43. 6-(2,2,2-Trifluorethylsulfinyl)-2,2-dimethyl-4-(2-oxo-1-piperidinyl)-2H-benzo[b]pyran
44. 6-(2,2,2-Trifluorethylsulfonyl)-2,2-dimethyl-4-(2-oxo-1-piperidinyl)-2H-benzo[b]pyran
45. 6-Difluormethoxy-2,2-dimethyl-4-(2-oxo-1-piperidinyl)-2H-benzo[b]pyran
46. 6-Trifluormethoxy-2,2-dimethyl-4-(2-oxo-1-piperidinyl)-2H-benzo[b]pyran
47. 6-(2,2,2-Trifluorethoxy)-2,2-dimethyl-4-(2-oxo-1-piperidinyl)-2H-benzo[b]pyran
48. 6-(1,1,2,2-Tetrafluorethoxy)-2,2-dimethyl-4-(2-oxo-1-piperidinyl)-2H-benzo[b]pyran
49. 6-Difluormethylthio-3,4-dihydro-2,2-dimethyl-trans-4-(2-cyanimino-3-imidazolidin-1-yl)-2H-benzo[b]pyran-3-ol
50. 6-Difluormethylsulfinyl-3,4-dihydro-2,2-dimethyl-trans-4-(2-cyanimino-3-imidazolidin-1-yl)-2H-benzo[b]pyran-3-ol
51. 6-Difluormethylsulfonyl-3,4-dihydro-2,2-dimethyl-trans-4-(2-cyanimino-3-imidazolidin-1-yl)-2H-benzo[b]pyran-3-ol
52. 6-Trifluormethylthio-3,4-dihydro-2,2-dimethyl-trans-4-(2-cyanimino-3-imidazolidin-1-yl)-2H-benzo[b]pyran-3-ol
53. 6-Trifluormethylsulfinyl-3,4-dihydro-2,2-dimethyl-trans-4-(2-cyanimino-3-imidazolidin-1-yl)-2H-benzo[b]pyran-3-ol
54. 6-Trifluormethylsulfonyl-3,4-dihydro-2,2-dimethyl-trans-4-(2-cyanimino-3-imidazolidin-1-yl)-2H-benzo[b]pyran-3-ol
55. 6-Difluormethoxy-3,4-dihydro-2,2-dimethyl-trans-4-(2-cyanimino-3-imidazolidin-1-yl)-2H-benzo[b]pyran-3-ol
56. 6-Trifluormethoxy-3,4-dihydro-2,2-dimethyl-trans-4-(2-cyanimino-3-imidazolidin-1-yl)-2H-benzo[b]pyran-3-ol
57. 6-Difluormethylthio-2,2-dimethyl-4-(2-cyanimino-3-imidazolidin-1-yl)-2H-benzo[b]pyran
58. 6-Difluormethylsulfinyl-2,2-dimethyl-4-(2-cyanimino-3-imidazolidin-1-yl)-2H-benzo[b]pyran
59. 6-Difluormethylsulfonyl-2,2-dimethyl-4-(2-cyanimino-3-imidazolidin-1-yl)-2H-benzo[b]pyran
60. 6-Trifluormethylthio-2,2-dimethyl-4-(2-cyanimino-3-imidazolidin-1-yl)-2H-benzo[b]pyran
61. 6-Trifluormethylsulfinyl-2,2-dimethyl-4-(2-cyanimino-3-imidazolidin-1-yl)-2H-benzo[b]pyran
62. 6-Trifluormethylsulfonyl-2,2-dimethyl-4-(2-cyanimino-3-imidazolidin-1-yl)-2H-benzo[b]pyran
63. 6-Difluormethoxy-2,2-dimethyl-4-(2-cyanimino-3-imidazolidin-1-yl)-2H-benzo[b]pyran
64. 6-Trifluormethoxy-2,2-dimethyl-4-(2-cyanimino-3-imidazolidin-1-yl)-2H-benzo[b]pyran
65. 6-Trifluormethoxy-2,2-dimethyl-4-(2-oxo-4-N-cyclopropylmethyl-piperazin-1-yl)-2H-benzo[b]pyran
66. 6-Difluormethylthio-3,4-dihydro-2,2-dimethyl-trans-4-(2-oxo-4-N-methylpiperazin-1-yl)-2H-benzo[b]pyran-3-ol
67. 6-Difluormethylsulfinyl-3,4-dihydro-2,2-dimethyl-trans-4-(2-oxo-4-N-methylpiperazin-1-yl)-2H-benzo[b]pyran-3-ol
68. 6-Difluormethylsulfonyl-3,4-dihydro-2,2-dimethyl-trans-4-(2-oxo-4-N-methylpiperazin-1-yl)-2H-benzo[b]pyran-3-ol
69. 6-Trifluormethylthio-3,4-dihydro-2,2-dimethyl-trans-4-(2-oxo-4-N-methylpiperazin-1-yl)-2H-benzo[b]pyran-3-ol
70. 6-Trifluormethylsulfinyl-3,4-dihydro-2,2-dimethyl-trans-4-(2-oxo-4-N-methylpiperazin-1-yl)-2H-benzo[b]pyran-3-ol
71. 6-Trifluormethylsulfonyl-3,4-dihydro-2,2-dimethyl-trans-4-(2-oxo-4-N-methylpiperazin-1-yl)-2H-benzo[b]pyran-3-ol
72. 6-Difluormethoxy-3,4-dihydro-2,2-dimethyl-trans-4-(2-oxo-4-N-methylpiperazin-1-yl)-2H-benzo[b]pyran-3-ol
73. 6-Trifluormethoxy-3,4-dihydro-2,2-dimethyl-trans-4-(2-oxo-4-N-methylpiperazin-1-yl)-2H-benzo[b]pyran-3-ol
74. 6-Trifluormethoxy-2,2-dimethyl-4-(2-oxo-4-N-isopropylpiperazin-1-yl)-2H-benzo[b]pyran
75. 6-Difluormethylthio-2,2-dimethyl-4-(2-oxo-4-N-methylpiperazin-1-yl)-2H-benzo[b]pyran
76. 6-Difluormethylsulfinyl-2,2-dimethyl-4-(2-oxo-4-N-methylpiperazin-1-yl)-2H-benzo[b]pyran
77. 6-Difluormethylsulfonyl-2,2-dimethyl-4-(2-oxo-4-N-methylpiperazin-1-yl)-2H-benzo[b]pyran
78. 6-Trifluormethylthio-2,2-dimethyl-4-(2-oxo-4-N-methylpiperazin-1-yl)-2H-benzo[b]pyran
79. 6-Trifluormethylsulfinyl-2,2-dimethyl-4-(2-oxo-4-N-methylpiperazin-1-yl)-2H-benzo[b]pyran
80. 6-Trifluormethylsulfonyl-2,2-dimethyl-4-(2-oxo-4-N-methylpiperazin-1-yl)-2H-benzo[b]pyran
81. 6-Difluormethoxy-2,2-dimethyl-4-(2-oxo-4-N-methylpiperazin-1-yl)-2H-benzo[b]pyran
82. 6-Trifluormethoxy-2,2-dimethyl-4-(2-oxo-4-N-methylpiperazin-1-yl)-2H-benzo[b]pyran
83. 6-Difluormethylthio-3,4-dihydro-2,2-dimethyl-trans-4-(2-cyanimino-3-thiazolidin-1-yl)-2H-benzo[b]pyran-3-ol
84. 6-Difluormethylsulfinyl-3,4-dihydro-2,2-dimethyl-trans-4-(2-cyanimino-3-thiazolidin-1-yl)-2H-benzo[b]pyran-3-ol
85. 6-Difluormethylsulfonyl-3,4-dihydro-2,2-dimethyl-trans-4-(2-cyanimino-3-thiazolidin-1-yl)-2H-benzo[b]pyran-3-ol
86. 6-Trifluormethylthio-3,4-dihydro-2,2-dimethyl-trans-4-(2-cyanimino-3-thiazolidin-1-yl)-2H-benzo[b]pyran-3-ol
87. 6-Trifluormethylsulfinyl-3,4-dihydro-2,2-dimethyl-trans-4-(2-cyanimino-3-thiazolidin-1-yl)-2H-benzo[b]pyran-3-ol
88. 6-Trifluormethylsulfonyl-3,4-dihydro-2,2-dimethyl-trans-4-(2-cyanimino-3-thiazolidin-1-yl)-2H-benzo[b]pyran-3-ol
89. 6-Difluormethoxy-3,4-dihydro-2,2-dimethyl-trans-4-(2-cyanimino-3-thiazolidin-1-yl)-2H-benzo[b]pyran-3-ol
90. 6-Trifluormethoxy-3,4-dihydro-2,2-dimethyl-trans-4-(2-cyanimino-3-thiazolidin-1-yl)-2H-benzo[b]pyran-3-ol
91. 6-Difluormethylthio-2,2-dimethyl-4-(2-cyanimino-3-thiazolidin-1-yl)-2H-benzo[b]pyran
92. 6-Difluormethylsulfinyl-2,2-dimethyl-4-(2-cyanimino-3-thiazolidin-1-yl)-2H-benzo[b]pyran
93. 6-Difluormethylsulfonyl-2,2-dimethyl-4-(2-cyanimino-3-thiazolidin-1-yl)-2H-benzo[b]pyran
94. 6-Trifluormethylthio-2,2-dimethyl-4-(2-cyanimino-3-thiazolidin-1-yl)-2H-benzo[b]pyran
95. 6-Trifluormethylsulfinyl-2,2-dimethyl-4-(2-cyanimino-3-thiazolidin-1-yl)-2H-benzo[b]pyran
96. 6-Trifluormethylsulfonyl-2,2-dimethyl-4-(2-cyanimino-3-thiazolidin-1-yl)-2H-benzo[b]pyran
97. 6-Difluormethoxy-2,2-dimethyl-4-(2-cyanimino-3-thiazolidin-1-yl)-2H-benzo[b]pyran
98. 6-Trifluormethoxy-2,2-dimethyl-4-(2-cyanimino-3-thiazolidin-1-yl)-2H-benzo[b]pyran
99. 6-Difluormethylthio-3,4-dihydro-2,2-dimethyl-trans-4-(2-cyanimino-3-N-methylimidazolidin-1-yl)-2H-benzo[b]pyran-3-ol
100. 6-Difluormethylsulfinyl-3,4-dihydro-2,2-dimethyl-trans-4-(2-cyanimino-3-N-methylimidazolidin-1-yl)-2H-benzo[b]pyran-3-ol
101. 6-Difluormethylsulfonyl-3,4-dihydro-2,2-dimethyl-trans-4-(2-cyanimino-3-N-methylimidazolidin-1-yl)-2H-benzo[b]pyran-3-ol
102. 6-Trifluormethylthio-3,4-dihydro-2,2-dimethyl-trans-4-(2-cyanimino-3-N-methylimidazolidin-1-yl)-2H-benzo[b]pyran-3-ol
103. 6-Trifluormethylsulfinyl-3,4-dihydro-2,2-dimethyl-trans-4-(2-cyanimino-3-N-methylimidazolidin-1-yl)-2H-benzo[b]pyran-3-ol
104. 6-Trifluormethylsulfonyl-3,4-dihydro-2,2-dimethyl-trans-4-(2-cyanimino-3-N-methylimidazolidin-1-yl)-2H-benzo[b]pyran-3-ol
105. 6-Difluormethoxy-3,4-dihydro-2,2-dimethyl-trans-4-(2-cyanimino-3-N-methylimidazolidin-1-yl)-2H-benzo[b]pyran-3-ol
106. 6-Trifluormethoxy-3,4-dihydro-2,2-dimethyl-trans-4-(2-cyanimino-3-N-methylimidazolidin-1-yl)-2H-benzo[b]pyran-3-ol
107. 6-Difluormethylthio-2,2-dimethyl-4-(2-cyanimino-3-N-methylimidazolidin-1-yl)-2H-benzo[b]pyran
108. 6-Difluormethylsulfinyl-2,2-dimethyl-4-(2-cyanimino-3-N-methylimidazolidin-1-yl)-2H-benzo[b]pyran
109. 6-Difluormethylsulfonyl-2,2-dimethyl-4-(2-cyanimino-3-N-methylimidazolidin-1-yl)-2H-benzo[b]pyran
110. 6-Trifluormethylthio-2,2-dimethyl-4-(2-cyanimino-3-N-methylimidazolidin-1-yl)-2H-benzo[b]pyran
111. 6-Trifluormethylsulfinyl-2,2-dimethyl-4-(2-cyanimino-3-N-methylimidazolidin-1-yl)-2H-benzo[b]pyran
112. 6-Trifluormethylsulfonyl-2,2-dimethyl-4-(2-cyanimino-3-N-methylimidazolidin-1-yl)-2H-benzo[b]pyran
113. 6-Difluormethoxy-2,2-dimethyl-4-(2-cyanimino-3-N-methylimidazolidin-1-yl)-2H-benzo[b]pyran
114. 6-Trifluormethoxy-2,2-dimethyl-4-(2-cyanimino-3-N-methylimidazolidin-1-yl)-2H-benzo[b]pyran
115. 6-Difluormethylthio-3,4-dihydro-2,2-dimethyl-trans-4-(2-cyanimino-3-hexahydropyrimidin-1-yl)-2H-benzo[b]pyran-3-ol
116. 6-Difluormethylsulfinyl-3,4-dihydro-2,2-dimethyl-trans-4-(2-cyanimino-3-hexahydropyrimidin-1-yl)-2H-benzo[b]pyran-3-ol
117. 6-Difluormethylsulfonyl-3,4-dihydro-2,2-dimethyl-trans-4-(2-cyanimino-3-hexahydropyrimidin-1-yl)-2H-benzo[b]pyran-3-ol
118. 6-Trifluormethylthio-3,4-dihydro-2,2-dimethyl-trans-4-(2-cyanimino-3-hexahydropyrimidin-1-yl)-2H-benzo[b]pyran-3-ol
119. 6-Trifluormethylsulfinyl-3,4-dihydro-2,2-dimethyl-trans-4-(2-cyanimino-3-hexahydropyrimidin-1-yl)-2H-benzo[b]pyran-3-ol
120. 6-Trifluormethylsulfonyl-3,4-dihydro-2,2-dimethyl-trans-4-(2-cyanimino-3-hexahydropyrimidin-1-yl)-2H-benzo[b]pyran-3-ol
121. 6-Difluormethoxy-3,4-dihydro-2,2-dimethyl-trans-4-(2-cyanimino-3-hexahydropyrimidin-1-yl)-2H-benzo[blpyran-3-ol
122. 6-Trifluormethoxy-3,4-dihydro-2,2-dimethyl-trans-4-(2-cyanimino-3-hexahydropyrimidin-1-yl)-2H-benzo[b]pyran-3-ol
123. 6-Difluormethylthio-2,2-dimethyl-4-(2-cyanimino-3-hexahydropyrimidin-1-yl)-2H-benzo[b]pyran
124. 6-Difluormethylsulfinyl-2,2-dimethyl-4-(2-cyanimino-3-hexahydropyrimidin-1-yl)-2H-benzo[b]pyran
125. 6-Difluormethylsulfonyl-2,2-dimethyl-4-(2-cyanimino-3-hexahydropyrimidin-1-yl)-2H-benzo[b]pyran
126. 6-Trifluormethylthio-2,2-dimethyl-4-(2-cyanimino-3-hexahydropyrimidin-1-yl)-2H-benzo[b]pyran
127. 6-Trifluormethylsulfinyl-2,2-dimethyl-4-(2-cyanimino-3-hexahydropyrimidin-1-yl)-2H-benzo[b]pyran
128. 6-Trifluormethylsulfonyl-2,2-dimethyl-4-(2-cyanimino-3-hexahydropyrimidin-1-yl)-2H-benzo[b]pyran
129. 6-Difluormethoxy-2,2-dimethyl-4-(2-cyanimino-3-hexahydropyrimidin-1-yl)-2H-benzo[b]pyran
130. 6-Trifluormethoxy-2,2-dimethyl-4-(2-cyanimino-3-hexahydropyrimidin-1-yl)-2H-benzo[b]pyran
131. 6-Difluormethylthio-3,4-dihydro-2,2-dimethyl-trans-4-(2-oxo-4-piperazin-1-yl)-2H-benzo[b]pyran-3-ol
132. 6-Difluormethylsulfinyl-3,4-dihydro-2,2-dimethyl-trans-4-(2-oxo-4-piperazin-1-yl)-2H-benzo[b]pyran-3-ol
133. 6-Difluormethylsulfonyl-3,4-dihydro-2,2-dimethyl-trans-4-(2-oxo-4-piperazin-1-yl)-2H-benzo[b]pyran-3-ol
134. 6-Trifluormethylthio-3,4-dihydro-2,2-dimethyl-trans-4-(2-oxo-4-piperazin-1-yl)-2H-benzo[b]pyran-3-ol
135. 6-Trifluormethylsulfinyl-3,4-dihydro-2,2-dimethyl-trans-4-(2-oxo-4-piperazin-1-yl)-2H-benzo[b]pyran-3-ol
136. 6-Trifluormethylsulfonyl-3,4-dihydro-2,2-dimethyl-trans-4-(2-oxo-4-piperazin-1-yl)-2H-benzo[blpyran-3-ol
137. 6-Difluormethoxy-3,4-dihydro-2,2-dimethyl-trans-4-(2-oxo-4-piperazin-1-yl)-2H-benzo[b]pyran-3-ol
138. 6-Trifluormethoxy-3,4-dihydro-2,2-dimethyl-trans-4-(2-oxo-4-piperazin-1-yl)-2H-benzo[b]pyran-3-ol
139. 6-Difluormethylthio-2,2-dimethyl-4-(2-oxo-4-piperazin-1-yl)-2H-benzo[b]pyran
140. 6-Difluormethylsulfinyl-2,2-dimethyl-4-(2-oxo-4-piperazin-1-yl)-2H-benzo[b]pyran
141. 6-Difluormethylsulfonyl-2,2-dimethyl-4-(2-oxo-4-piperazin-1-yl)-2H-benzo[b]pyran
142. 6-Trifluormethylthio-2,2-dimethyl-4-(2-oxo-4-piperazin-1-yl)-2H-benzo[b]pyran
143. 6-Trifluormethylsulfinyl-2,2-dimethyl-4-(2-oxo-4-piperazin-1-yl)-2H-benzo[b]pyran
144. 6-Trifluormethylsulfonyl-2,2-dimethyl-4-(2-oxo-4-piperazin-1-yl)-2H-benzo[b]pyran
145. 6-Difluormethoxy-2,2-dimethyl-4-(2-oxo-4-piperazin-1-yl)-2H-benzo[b]pyran
146. 6-Trifluormethoxy-2,2-dimethyl-4-(2-oxo-4-piperazin-1-yl)-2H-benzo[b]pyran
147. 6-Difluormethylthio-3,4-dihydro-2,2-dimethyl-trans-4-(2-oxo-4-N-benzylpiperazin-1-yl)-2H-benzo[b]pyran-3-ol
148. 6-Difluormethylsulfinyl-3,4-dihydro-2,2-dimethyl-trans-4-(2-oxo-4-N-benzylpiperazin-1-yl)-2H-benzo[b]pyran-3-ol
149. 6-Difluormethylsulfonyl-3,4-dihydro-2,2-dimethyl-trans-4-(2-oxo-4-N-benzylpiperazin-1-yl)-2H-benzo[b]pyran-3-ol
150. 6-Trifluormethylthio-3,4-dihydro-2,2-dimethyl-trans-4-(2-oxo-4-N-benzylpiperazin-1-yl)-2H-benzo[b]pyran-3-ol
151. 6-Trifluormethylsulfinyl-3,4-dihydro-2,2-dimethyl-trans-4-(2-oxo-4-N-benzylpiperazin-1-yl)-2H-benzo[b]pyran-3-ol
152. 6-Trifluormethylsulfonyl-3,4-dihydro-2,2-dimethyl-trans-4-(2-oxo-4-N-benzylpiperazin-1-yl)-2H-benzo[b]pyran-3-ol
153. 6-Difluormethoxy-3,4-dihydro-2,2-dimethyl-trans-4-(2-oxo-4-N-benzylpiperazin-1-yl)-2H-benzo[b]pyran-3-ol
154. 6-Trifluormethoxy-3,4-dihydro-2,2-dimethyl-trans-4-(2-oxo-4-N-benzylpiperazin-1-yl)-2H-benzo[b]pyran-3-ol
155. 6-Difluormethylthio-2,2-dimethyl-4-(2-oxo-4-N-benzylpiperazin-1-yl)-2H-benzo[b]pyran
156. 6-Difluormethylsulfinyl-2,2-dimethyl-4-(2-oxo-4-N-benzylpiperazin-1-yl)-2H-benzo[b]pyran
157. 6-Difluormethylsulfonyl-2,2-dimethyl-4-(2-oxo-4-N-benzylpiperazin-1-yl)-2H-benzo[b]pyran
158. 6-Trifluormethylthio-2,2-dimethyl-4-(2-oxo-4-N-benzylpiperarin-1-yl)-2H-benzo[b]pyran
159. 6-Trifluormethylsulfinyl-2,2-dimethyl-4-(2-oxo-4-N-benzylpiperazin-1-yl)-2H-benzo[b]pyran
160. 6-Trifluormethylsulfonyl-2,2-dimethyl-4-(2-oxo-4-N-benzylpiperazin-1-yl)-2H-benzo[b]pyran
161. 6-Difluormethoxy-2,2-dimethyl-4-(2-oxo-4-N-benzylpiperazin-1-yl)-2H-benzo[b]pyran
162. 6-Trifluormethoxy-2,2-dimethyl-4-(2-oxo-4-N-benzylpiperazin-1-yl)-2H-benzo[b]pyran
163. 6-Difluormethylthio-3,4-dihydro-2,2-dimethyl-trans-4-(2-oxo-4-N-cyclopropylmethyl-piperazin-1-yl)-2H-benzo[b]pyran-3-ol
164. 6-Difluormethylsulfinyl-3,4-dihydro-2,2-dimethyl-trans-4-(2-oxo-4-N-cyclopropylmethyl-piperazin-1-yl)-2H-benzo[b]pyran-3-ol
165. 6-Difluormethylsulfonyl-3,4-dihydro-2,2-dimethyl-trans-4-(2-oxo-4-N-cyclopropylmethyl-piperazin-1-yl)-2H-benzo[b]pyran-3-ol
166. 6-Trifluormethylthio-3,4-dihydro-2,2-dimethyl-trans-4-(2-oxo-4-N-cyclopropylmethyl-piperazin-1-yl)-2H-benzo[b]pyran-3-ol
167. 6-Trifluormethylsulfinyl-3,4-dihydro-2,2-dimethyl-trans-4-(2-oxo-4-N-cyclopropylmethyl-piperazin-1-yl)-2H-benzo[b]pyran-3-ol
168. 6-Trifluormethylsulfonyl-3,4-dihydro-2,2-dimethyl-trans-4-(2-oxo-4-N-cyclopropylmethyl-piperazin-1-yl)-2H-benzo[b]pyran-3-ol
169. 6-Difluormethoxy-3,4-dihydro-2,2-dimethyl-trans-4-(2-oxo-4-N-cyclopropylmethyl-piperazin-1-yl)-2H-benzo[b]pyran-3-ol
170. 6-Trifluormethoxy-3,4-dihydro-2,2-dimethyl-trans-4-(2-oxo-4-N-cyclopropylmethyl-piperazin-1-yl)-2H-benzo[b]pyran-3-ol
171. 6-Difluormethylthio-3,4-dihydro-2,2-dimethyl-trans-4-(2-oxo-4-N-isopropylpiperazin-1-yl)-2H-benzo[b]pyran-3-ol
172. 6-Difluormethylsulfinyl-3,4-dihydro-2,2-dimethyl-trans-4-(2-oxo-4-N-isopropylpiperazin-1-yl)-2H-benzo[b]pyran-3-ol
173. 6-Difluormethylsulfonyl-3,4-dihydro-2,2-dimethyl-trans-4-(2-oxo-4-N-isopropylpiperazin-1-yl)-2H-benzo[b]pyran-3-ol
174. 6-Trifluormethylthio-3,4-dihydro-2,2-dimethyl-trans-4-(2-oxo-4-N-isopropylpiperazin-1-yl)-2H-benzo[b]pyran-3-ol
175. 6-Trifluormethylsulfinyl-3,4-dihydro-2,2-dimethyl-trans-4-(2-oxo-4-N-isopropylpiperazin-1-yl)-2H-benzo[b]pyran-3-ol
176. 6-Trifluormethylsulfonyl-3,4-dihydro-2,2-dimethyl-trans-4-(2-oxo-4-N-isopropylpiperazin-1-yl)-2H-benzo[b]pyran-3-ol
177. 6-Difluormethoxy-3,4-dihydro-2,2-dimethyl-trans-4-(2-oxo-4-N-isopropylpiperazin-1-yl)-2H-benzo[b]pyran-3-ol
178. 6-Trifluormethoxy-3,4-dihydro-2,2-dimethyl-trans-4-(2-oxo-4-N-isopropylpiperazin-1-yl)-2H-benzo[b]pyran-3-ol
179. 6-Difluormethoxy-2,2-dimethyl-4-(2-oxo-4-N-isopropylpiperazin-1-yl)-2H-benzo[b]pyran
180. 6-Difluormethoxy-2,2-dimethyl-4-(2-oxo-4-N-cyclopropylmethyl-piperazin-1-yl)-2H-benzo[b]pyran
181. 6-Trifluormethylthio-2,2-dimethyl-4-(2-oxo-4-N-cyclopropylmethyl-piperazin-1-yl)-2H-benzo[b]pyran
182. 6-Difluormethoxy-3,4-dihydro-2,2-dimethyl-trans-4-(1-pyrrolidinyl)-2H-benzo[b]pyran-3-ol
183. 6-Difluormethoxy-3,4-dihydro-2,2-dimethyl-trans-4-(2-oxo-3-imidazolidin-1-yl)-2H-benzo[b]pyran-3-ol
184. 6-Trifluormethoxy-3,4-dihydro-2,2-dimethyl-trans-4-(2-oxo-4-N-methoxycarbonyl-piperazin-1-yl)-2H-benzo[b]pyran-3-ol
185. 6-Trifluormethoxy-3,4-dihydro-2,2-dimethyl-trans-4-(2-oxo-4-N-acetyl-piperazin-1-yl)-2H-benzo[b]pyran-3-ol
186. 6-Trifluormethylthio-3,4-dihydro-2,2-dimethyl-trans-4-(2-oxo-4-N-methoxycarbonyl-piperazin-1-yl)-2H-benzo[b]pyran-3-ol
187. 6-Difluormethoxy-3,4-dihydro-2,2-dimethyl-trans-4-(2-oxo-3-N-methylimidazolidin-1-yl)-2H-benzo[b]pyran-3-ol
188. 6-Difluormethoxy-3,4-dihydro-2,2-dimethyl-trans-4-(2-oxo-3-oxazolidin-1-yl)-2H-benzo[b]pyran-3-ol
189. 6-Difluormethoxy-3,4-dihydro-2,2-dimethyl-trans-4-(1-piperidinyl)-2H-benzo[b]pyran-3-ol
190. 6-Difluormethoxy-3,4-dihydro-2,2-dimethyl-trans-4-(1-N-morpholinyl)-2H-benzo[b]pyran-3-ol
191. 6-Difluormethoxy-3,4-dihydro-2,2-dimethyl-trans-4-(2-oxo-3-N-phenylimidazolidin-1-yl)-2H-benzo[b]pyran-3-ol
192. 6-Difluormethoxy-3,4-dihydro-2,2-dimethyl-trans-4-(1-aza-2-oxo-1-cycloheptyl)-2H-benzo[b]pyran-3-ol
193. 6-Difluormethoxy-3,4-dihydro-2,2-dimethyl-trans-4-(2-oxo-4-N-acetyl-piperazin-1-yl)-2H-benzo[b]pyran-3-ol
194. 6-Difluormethoxy-2,2-dimethyl-4-(2-oxo-3-N-methyl-hexahydropyrimidin-1-yl)-2H-benzo[b]pyran
195. 6-Difluormethoxy-2,2-dimethyl-4-(2-oxo-3-thiazolidin-1-yl)-2H-benzo[b]pyran
196. 6-Difluormethoxy-3,4-dihydro-2,2-dimethyl-trans-4-(2-oxo-3-thiazolidin-1-yl)-2H-benzo[b]pyran-3-ol
197. 6-Cyano-3,4-dihydro-2,2-dimethyl-trans-4-(2-nitromethyliden-3-imidazolidin-1-yl)-2H-benzo[b]pyran-3-ol
198. 6-Trifluormethylthio-3,4-dihydro-2,2-dimethyl-trans-4-(2-nitromethyliden-3-imidazolidin-1-yl)-2H-benzo[b]pyran-3-ol
199. 6-Cyano-3,4-dihydro-2,2-dimethyl-trans-4-(2-nitroimino-3-imidazolidin-1-yl)-2H-benzo[b]pyran-3-ol
200. 6-Trifluormethylthio-3,4-dihydro-2,2-dimethyl-trans-4-(2-nitroimino-3-imidazolidin-1-yl)-2H-benzo[b]pyran-3-ol

Die Verbindungen 1 - 48 werden besonders bevorzugt, insbesondere aber 1 bis 6, 12 bis 17, 21, 23 bis 28, 33, 36 bis 41, 46 und ganz besonders bevorzugt werden 4, 6, 15, 17, 21, 26, 28, 39, 41 und 46.

Die erfindungsgemäßen Verbindungen der Formel I, ihre physiologisch verträglichen Salze und Säureadditionssalze sowie deren tautomere und optische Isomere sind therapeutische Wirkstoffe, besitzen hohe pharmakologische Wirkung und sind wertvolle Arzneimittel. Insbesondere zeigen sie vasodilatierende, gefäßspasmolytische, insbesondere broncholytische Wirkung, wobei sich die gefäßspasmolytische Wirkung im gesamten Gefäßsystem oder auch mehr oder weniger isoliert in umschriebenen Gefäßgebieten wie Gehirn-, Coronar- oder Peripheriegefäßen entfalten kann.

Die erfindungsgemäßen Verbindungen haben insbesondere blutdrucksenkende Wirkung und können somit als antihypertensive Mittel verwendet werden.

Die erfindungsgemäßen Substanzen zeichnen sich durch eine beträchtliche Senkung des arteriellen Blutdrucks aus. Dosen von 0.01 - 10 mg/kg s. c. führten zu einer Senkung des Blutdrucks um mindestens 20% an hypertensiven Ratten.

Die erfindungsgemäßen Substanzen zeichnen sich durch eine besondere Beeinflussung der Kaliumionenströmung in den Zellen aus. Insbesondere sind sie Kaliumkanalaktivatoren. Sie eignen sich zur Prophylaxe und zur Behandlung folgender Erkrankungen bei Warmblütern, insbesondere des Menschen:
1. Bluthochdruck, insbesondere arterieller Bluthochdruck,
2. Herzinsuffizienz, Coronar-Insuffizienz, Angina Pectoris,
3. arterielle Verschlußkrankheit und periphere Durchblutungsstörungen,
4. cerebrale Insuffizienz, Migräne, Schwindel, Erkrankungen des Innenohrs beziehungsweise des Gehörapparats,
5. erhöhter Augeninnendruck, Glaukom, Sehschwäche,
6. Niereninsuffizienz, organische Erkrankungen der harnableitenden Wege sowie der Akzessorischen Drüsen der Harnwege, Potenzstörungen,
7. organische Störungen des Magendarmtraktes sowie der Bauchspeicheldrüse und Leber,
8. mangelnde Durchblutung der Kopfhaut, Haarausfall,
9. Erkrankungen der Luftwege einschließlich Asthmabronchiale,
10. metabolische Erkrankungen,
11. spasmogene Erkrankungen des Uterus,
12. Inkontinenz.

Weiterhin fördern die erfindungsgemäßen Verbindungen die Durchblutung der Kopfhaut und den Haarwuchs. Sie sind auch tokolytisch wirksam.

Die erfindungsgemäßen Verbindungen zeigen eine lange Wirkdauer bei nur geringer Toxizität. Daher eignen sie sich insbesondere zur Behandlung akuter und chronischer Herzkrankheiten, zur Therapie des Bluthochdrucks, der Herzinsuffizienz sowie zur Behandlung von Asthma und cerebralen und peripheren Durchblutungsstörungen.

Die Verbindungen der vorliegenden Erfindung können beim Menschen oral oder parenteral in einer Dosierung von 0,001 bis 100 mg, vorzugsweise 0,01 bis 50 mg, besonders bevorzugt 0,05 bis 10 mg pro Tag, angewendet werden, insbesondere auch in unterteilten Dosen, zum Beispiel zweimal bis viermal täglich. Diese Dosierungen sind vorteilhaft für die Behandlung der vorstehend genannten Krankheiten, insbesondere von Herzkrankheiten, Hypertonie, Asthma und Durchblutungsstörungen.

Im allgemeinen hat es sich als vorteilhaft erwiesen, bei intravenöser Applikation Mengen von etwa 0,001 bis 10 mg, vorzugsweise etwa 0,05 bis 5 mg dem Menschen pro Tag zur Erzielung wirksamer Ergebnisse zu verabreichen. Bei oraler Applikation beträgt die Dosierung etwa 0,05 bis 30 mg, vorzugsweise 0,1 bis 10 mg pro Tag beim Menschen.

Die vorstehend genannten Dosierungen werden für die Behandlung der Hypertonie besonders bevorzugt.

Trotzdem kann es gegebenenfalls erforderlich sein, von den genannten Mengen abzuweichen, und zwar in Abhängigkeit vom Körpergewicht beziehungsweise der Art des Applikationsweges, aber auch aufgrund des individuellen Verhaltens gegenüber dem Medikament beziehungsweise der Art von dessen Formulierung und dem Zeitpunkt beziehungsweise Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehrere Einzelgaben über den Tag zu verteilen.

Gegenstand der Erfindung sind auch die erfindungsgemäßen Verbindungen zur Behandlung der vorstehenden Krankheiten sowie Verfahren zur Behandlung dieser Krankheiten, in denen diese Verbindungen verwendet werden sowie ihre Verwendung im Verfahren zur Herstellung von Mitteln, die diese Verbindungen enthalten, zur Behandlung dieser Krankheiten sowie Verfahren zur Herstellung der Verbindungen.

Gemäß der Erfindung werden pharmazeutische Präparate oder Zusammensetzungen geschaffen, die eine erfindungsgemäße Verbindung oder deren pharmazeutisch verträgliches Salz oder Säureadditionssalze zusammen mit einem pharmazeutisch verträglichen Verdünnungsmittel oder Träger enthalten.

Die Verbindungen gemäß der Erfindung können mit üblichen pharmazeutisch verträglichen Verdünnungsmitteln oder Trägern und gegebenenfalls mit anderen Hilfsmitteln vermischt und beispielsweise oral oder parenteral verabreicht werden. Sie können vorzugsweise oral in Form von Granulaten, Kapseln, Pillen, Tabletten, Filmtabletten, Dragees, Sirupen, Emulsionen, Suspensionen, Dispersionen, Aerosolen und Lösungen sowie Flüssigkeiten oder parenteral in Form von Lösungen, Emulsionen oder Suspensionen verabreicht werden. Oral zu verabreichende Präparate können einen oder mehrere Zusätze wie Süßungsmittel, Aromatisierungsmittel, Farbstoffe und Konservierungsmittel enthalten. Tabletten können den Wirkstoff mit üblichen pharmazeutisch verträglichen Hilfsmitteln vermischt enthalten, zum Beispiel inerten Verdünnungsmitteln wie Calciumcarbonat, Natriumcarbonat, Lactose und Talk, Granulierungsmitteln und Mitteln, die den Zerfall der Tabletten bei oraler Verabreichung fördern wie Stärke oder Alginsäure, Bindemitteln wie Stärke oder Gelatine, Gleitmitteln wie Magnesiumstearat, Stearinsäure und Talk.

Geeignete Trägerstoffe sind beispielsweise Milchzucker (Lactose), Gelatine, Maisstärke, Stearinsäure, Ethanol, Propylenglycol, Ether des Tetrahydrofurfurylalkohols und Wasser.

Als Hilfsstoffe seien beispielsweise aufgeführt:
Wasser, nicht-toxische organische Lösungsmittel, wie Paraffine (z.B. Erdölfraktionen), pflanzliche Öle (z.B. Erdnuß-/Sesam-Öl), Alkohole (z.B. Ethylalkohol, Glycerin), Glykole (z.B. Propylenglykol, Polyethylenglykol), feste Trägerstoffe, wie z.B. natürliche Gesteinsmehle (z.B. Kaoline, Tonerden, Talkum, Kreide), synthetische Gesteinsmehle (z.B. hochdisperse Kieselsäure, Silikate), Zucker (z.B. Rohr-, Milch- und Traubenzucker), Emulgiermittel (z.B. Polyoxyethylen-Fettsäure-Ester, Polyoxyethylen-Fettalkohol-Ether, Alkylsulfonate und Arylsulfonate), Dispergiermittel (z.B. Lignin; Sulfitablaugen, Methylcellulose, Stärke und Polyvinylpyrrolidon) und Gleitmittel (z.B. Magnesiumstearat, Talkum, Stearinsäure und Natriumlaurylsulfat).

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Die Applikation erfolgt in üblicher Weise, vorzugsweise oral oder parenteral, insbesondere perlingual oder intravenös. Im Falle der oralen Anwendung können Tabletten selbstverständlich außer den genannten Trägerstoffen auch Zusätze, wie Natriumcitrat, Calziumcarbonat und Dicalziumphosphat zusammen mit verschiedenen Zuschlagstoffen, wie Stärke, vorzugsweise Kartoffelstärke, Gelatine und dergleichen enthalten. Weiterhin können Gleitmittel wie Magnesiumstearat, Natriumlaurylsulfat und Talkum zum Tablettieren mitverwendet werden. Im Falle wäßriger Suspensionen und/oder Elixieren, die für orale Anwendungen gedacht sind, können die Wirkstoffe außer mit den obengenannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Die Tabletten können nach bekannten Arbeitsweisen überzogen werden, um den Zerfall und die Resorption im Magen-Darmtrakt zu verzögern, wodurch die Aktivität des Wirkstoffs sich über eine Längere Zeitspanne erstrecken kann. Ebenso kann in den Suspensionen der Wirkstoff mit Hilfsmitteln vermischt sein, die für die Herstellung solcher Zusammensetzungen üblich sind, zum Beispiel Suspendiermitteln wie Methylcellulose, Tragacanth oder Natriumalginat, Netzmitteln wie Lecithin, Polyoxyethylenstearat und Polyoxyethylensorbitanmonooleat und Konservierungsmitteln wie Ethylparahydroxybenzoat. Kapseln können den Wirkstoff als einzigen Bestandteil oder vermischt mit einem festen Verdünnungsmittel wie Calciumcarbonat, Calciumphosphat oder Kaolin enthalten. Die injizierbaren Präparate werden ebenfalls in an sich bekannter Weise formuliert.

Die pharmazeutischen Präparate können den Wirkstoff in einer Menge von 0,1 bis 90 Gewichtsprozent, insbesondere 1 bis 90 Gewichtsprozent enthalten, d.h. in Mengen, die ausreichend sind, um den angegebenen Dosierungsspielraum zu erreichen, wobei der Rest ein Trägerstoff oder Zusatzstoff ist. Im Hinblick auf die Herstellung und Verabreichung werden feste Präparate wie Tabletten und Kapseln bevorzugt. Vorzugsweise enthalten die Präparate den Wirkstoff in einer Menge von 0,05 bis 10 mg.

Die Verbindungen der Formel I sind durch nucleophile Ringöffnung von 2,2,6-substituierten 3,4-Dihydro-3,4-epoxy-2H-benzo(b)pyranen der Formel II zugänglich:

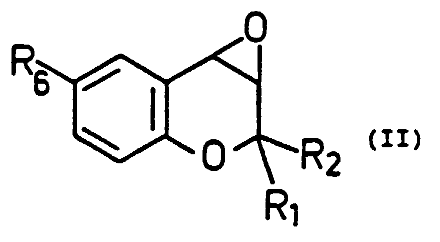

Als Nucleophile werden vorzugsweise Ammoniak, primäre und sekundäre organische Amine, Salze und Ester von alpha-omega-Aminocarbonsäuren sowie Anionen cyclischer Amide und cyclischer Harnstoff-, Carbamidsäure-, Thiocarbamidsäure- oder Cyanguanidin-Derivate eingesetzt. Die daraus resultierenden Verbindungen der Formel I fallen als trans-Isomere an und sind praktisch frei von cis-Isomeren.

Als sekundäre Amine werden insbesondere die Heterocyclen H-A und H-B eingesetzt, als cyclische Amide der Heterocyclus C als Anion, das durch Einwirkung einer starken Base erhältlich ist.

Als cyclisches Harnstoff-, Carbamidsäure- oder Thiocarbamidsäure-Derivat wird der Heterocyclus D eingesetzt, nachdem man durch Einwirkung einer starken Base dessen Anion erzeugt hat. Der Heterocyclus E, in dem p, Y, und Z die oben angegebene Bedeutung haben, wird in Form seines Anions eingesetzt, das mit einer starken Base erhältich ist.

Die folgenden Herstellungsverfahren werden bevorzugt:
Eine Untergruppe (1) von Verbindungen der Formel I, bei der R₆ für die Substituentenklasse R₆', R₅ für die Heterocyclenreste A und B, R₃ für Hydroxy und R₄ für Wasserstoff steht und R₁ und R₂ die oben angegebene Bedeutung haben, kann durch Umsetzung von Verbindungen der allgemeinen Formel IIa 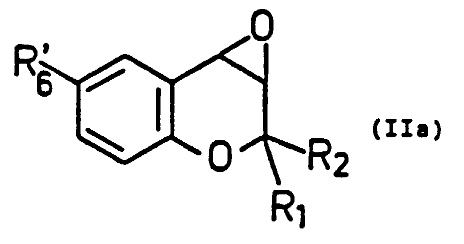 mit Heterocyclen der allgemeinen Formel H-A oder H-B, in denen A und B die oben angegebene Bedeutung haben, hergestellt werden.

Die Verbindungen der Formel H-A oder H-B sind bekannt oder in Analogie zu bekannten Verfahren herstellbar.

Die Reaktion des Oxirans IIa kann bei Temperaturen zwischen -10^{o}C bis +200^{o}C, bevorzugt jedoch bei Raumtemperatur oder leicht erhöhter Temperatur, z.B. 20 - 100^{o}C durchgeführt werden.

Die Reaktion erfolgt vorzugsweise in einem Alkohol wie Methanol, Ethanol oder Propanol oder in einem niederen Keton wie Aceton oder Butanon oder in einem geeigneten Ether wie Dioxan oder ohne Lösungsmittel, zweckmäßigerweise bei der Rückflußtemperatur des Reaktionsgemisches, falls ein Lösungsmittel vorliegt. Es entstehen Verbindungen der Formel I, in denen R₃ mit der Bedeutung Hydroxy und R₅ mit der Bedeutung A oder B stereospezifisch **trans** zueinander angeordnet sind.

Eine weitere Untergruppe **(2)** von Verbindungen der Formel I, bei der R₁ und R₂ die oben angegebene Bedeutung haben, R₃ für Hydroxy, R₄ für Wasserstoff, R₆ für R₆' mit der oben angegebenen Bedeutung und R₅ für die Heterocyclen-Reste C und D, worin k, m, n und Y die angegebene Bedeutung haben und X für Sauerstoff steht, sind aus Oxiranen der Formel IIa, in denen R₁, R₂ und R₆' die oben angegebene Bedeutung haben, und Heterocyclen der Struktur H-C und H-D, wobei k, m, n und Y die oben angegebene Bedeutung haben und X für Sauerstoff steht, erhalten werden.

Die Reaktion des Oxirans IIa der oben beschriebenen Gruppe von Heterocyclen H-C und H-D erfolgt in einem Lösungsmittel wie Dimethylsulfoxid in Anwesenheit einer Base wie Natriumhydrid bei Temperaturen zwischen 0 und 80^{o}C, vorzugsweise bei Raumtemperatur.

Die Heterocyclen der Formel H-C sind bekannt, die der Formel H-D sind bekannt oder können in Analogie zu bekannten Verfahren hergestellt werden u.a. wie in J. Med. Chem. **24**, 1089-92 (1981) beschrieben.

Heterocyclen der Formel H-D, in denen m 1 oder 2 und k 1 oder 2 ist, unter der Maßgabe, daß m + k 1, 2 oder 3 ist und Y die Bedeutung unsubstituiertes Amino hat, werden zweckmäßig in Derivate mit geschützter Aminogruppe übergeführt, bevor sie unter obengenannten Bedingungen mit Oxiranen IIa umgesetzt werden.

Eine solche Schutzgruppe für die geschützten Aminogruppen können die obengenannten Reste R₇ sein, insbesondere aber Benzyl, Benzylcarbonyl, Phenylcarbonyl, t-Butyloxycarbonyl, Benzyloxycarbonyl, C₁₋₂-Alkylcarbonyl oder Triphenylmethyl, und sie wird nach erfolgter Umsetzung mit Oxiranen der Formel IIa unter den jeweils für die betreffende Schutzgruppe günstigsten Bedingungen hydrogenolytisch oder hydrolytisch entfernt, falls dies erwünscht ist. Von den obengenannten Schutzgruppen wird Acetyl und Benzyl bevorzugt.

Verbindungen der Untergruppe **(2)**, bei denen R₅ für C, X für Sauerstoff und n für 2 oder 3 steht, sind außerdem in Analogie zu den in EP 0 076 075 beschriebenen Verfahren durch Cyclisierung von offenkettigen Vorstufen zugänglich.

Ausgehend von Verbindungen der Untergruppe **(2)** ist eine weitere Untergruppe **(3)** von Verbindungen der Formel I, bei der R₁ und R₂ die oben angegebene Bedeutung haben, R₃ für Hydroxy, R₄ für Wasserstoff, R₆ für R₆' mit der oben angegebenen Bedeutung und R₅ für die Heterocyclen-Reste C und D, worin k, m, n und Y die angegebene Bedeutung haben, jedoch X für Schwefel steht, durch Umsetzung von Verbindungen der Untergruppe **(2)** mit Schwefelungsreagentien wie Schwefelwasserstoff, Phosphorpentasulfid oder Lawesson's Reagens (p-Methoxyphenylthiophosphinsulfid, dimer) zugänglich.

Die Schwefelungsreaktion wird unter den für das betreffende Reagens üblichen Bedingungen durchgeführt, z.B. wird Schwefelwasserstoff bevorzugt säurekatalysiert (z.B. mit Chlorwasserstoff) in einem polaren Solvens wie Essigsäure oder Ethanol umgesetzt.

Die Umsetzung mit Lawesson's Reagens erfolgt bevorzugt unter Rückflußtemperatur eines trockenen Lösungsmittels wie Methylenchlorid oder Toluol.

Eine weitere Untergruppe **(4)** von Verbindungen der Formel I, bei der R₁ und R₂ die angegebene Bedeutung hat, R₃ für Hydroxy, R₄ für Wasserstoff und R₆ für beide Substituentenklassen R₆' und R₆" mit der für R₆' und R₆" angegebenen Bedeutung steht und R₅ die Bedeutung E hat, ist aus Verbindungen der allgemeinen Formel IIa und IIb durch Umsetzung mit Heterocyclen der Formel H-E, in denen E die angegebene Bedeutung hat, zugänglich:

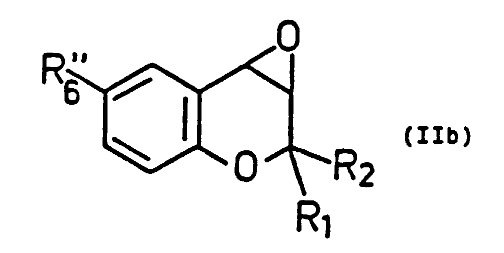

Die Heterocyclen der Formel H-E sind bekannt oder können nach bekannten Verfahren, die z.B. in J. Heterocycl. Chem. 19, 1205 (1982), ibid 24, 275 (1987), DE 22 05 745, Helv. Chim. Acta 67, 1669 (1984), Can. J. Chem. 39, 1787 (1961), EP 0 277 317 oder Chem. Ber. 100, 591 (1967) beschrieben sind, hergestellt werden.

Die Umsetzung von IIa und IIb mit H-E erfolgt in Lösungsmitteln wie Dimethylsulfoxid in Anwesenheit einer Base wie Natriumhydrid unter den für Untergruppe **(2)** beschriebenen Bedingungen.

Alternativ können Verbindungen der obengenannten Untergruppe **(4)**, in denen Z die Bedeutung =N-CN hat, aus Verbindungen der Untergruppe **(3)**, in denen R₁, R₂ und R₆' die angegebene Bedeutung haben, R₃ für Hydroxy, R₄ für Wasserstoff und R₅ für den Rest D steht, worin k, m, n und Y die angegebene Bedeutung haben und X für Schwefel steht, oder aus analogen Verbindungen, in denen R₆ die Bedeutung R₆" hat und die im EP 0 107 423 beschrieben sind, durch Umsetzung mit Bleicyanamid PbNCN in einem Lösungsmittel wie Ethanol oder Dimethylsulfoxid bei Temperaturen zwischen 20^{o}C und der Rückflußtemperatur des verwendeten Lösungsmittels, bevorzugt bei der Rückflußtemperatur des verwendeten Lösungsmittels hergestellt werden.

Die Verbindungen der vorstehend beschriebenen Untergruppen **(1)** - **(4)** enthalten eine freie Hydroxygruppe R₃ und können daher, sofern sie keine reaktive, unsubstituierte Aminogruppe enthalten, d.h. sofern Y in Formel B nicht für unsubstituiertes Amino und Y in Formel D nur dann für unsubstituiertes Amino steht, wenn gleichzeitig m = 0 ist, durch O-Alkylierung oder O-Acylierung in eine weitere Untergruppe **(5)** von Verbindungen der Formel I übergeführt werden, in der R₃ die Bedeutung C₁₋₆-Alkoxy oder C₁₋₈-Alkylcarbonyloxy, Formyloxy, C₁₋₈-Monoalkylaminocarbonyloxy oder C₁₋₈-Dialkylaminocarbonyloxy hat, R₁ und R₂ die angegebene Bedeutung haben, R₄ für Wasserstoff und R₆ für R₆' steht, falls R₅ die Bedeutung (A), (B), (C) oder (D) hat und für R₆' R₆" steht, falls R₅ die Bedeutung (E) hat.

Verbindungen der Untergruppe **(5)**, in denen R₅ die Bedeutung B oder D hat und Y für unsubstituiertes Amino steht, sind durch O-Alkylierung oder O-Acylierung von Verbindungen der Untergruppe **(1)**, **(2)** oder **(3)**, in denen Y die Bedeutung substituiertes Amino - NR₇ - hat, durch anschließende Abspaltung der Schutzgruppe R₇, wie für Untergruppe **(2)** beschrieben, zugänglich.

Eine Alkylierung kann z.B. durch Verwendung eines C₁₋₆-Alkyliodids in einem inerten Solvens wie Toluol oder Dimethylformamid in Anwesenheit einer Base wie Kaliumhydroxid oder Bariumoxid ausgeführt werden.

Eine Veresterung kann durch Verwendung eines C₁₋₈-Acylchlorids oder Acylanhydrids oder eines anderen aktivierten Derivats der betreffenden Alkansäure, gegebenenfalls unter Anwesenheit einer organischen Base wie Pyridin oder Triethylamin oder einer anorganischen Base wie Kaliumcarbonat, gegebenenfalls unter Mitwirkung von Katalysatoren wie 4-(N,N-Dimethylamino)-pyridin oder Kondensationsreagentien wie Dicyclohexylcarbodiimid in einem inerten Solvens, gegebenenfalls bei erhöhter Temperatur durchgeführt werden.

Eine Umsetzung zu Carbonaten erfolgt analog durch Reaktion mit Chlorameisensäure-C₁₋₈-alkylestern unter oben angegebenen Bedingungen.

Die Umsetzung zu Carbamaten erfolgt entweder in Analogie zu oben beschriebenen Verfahren durch Reaktion mit Mono- oder Dialkylaminocarbamoylchloriden oder durch Umsetzung mit C₁₋₈-Alkylisocyanaten in einem inerten Solvens, z.B. Toluol, bei Temperaturen zwischen 0^{o}C und der Siedetemperatur des Reaktionsgemisches.

Durch Umsetzung mit Ameisensäure in Anwesenheit von Pyridin läßt sich Formyloxy einführen.

Eine weitere Untergruppe **(6)** von Verbindungen der Formel I, bei der R₃ und R₄ zusammen eine Bindung bilden, R₁ und R₂ die angegebene Bedeutung haben, R₅ für die Reste C, D und E steht und R₆ für R₆' steht, falls R₅ für C, D und E steht oder R₆ für R₆' und R₆" steht, falls R₅ für E steht, ist durch Dehydratisierung von Verbindungen der Untergruppen **(2)**, **(3)** und **(4)**, bei denen R₁-R₆ die jeweils dort angegebene Bedeutung haben, zugänglich.

Die Dehydratisierung wird durch Reagentien wie Natriumhydrid in einem inerten Solvens wie Tetrahydrofuran, vorzugsweise bei Rückflußtemperatur des Reaktionsgemisches ausgeführt. Verbindungen der Untergruppe **(6)** können gegebenenfalls als Nebenprodukte bei der Umsetzung von Oxiranen der Formel IIa mit Heterocyclen der Formel H-C, H-D oder H-E beziehungsweise bei der Umsetzung von Oxiranen IIb mit H-E, infolge einer Weiterreaktion der intermediär gebildeten Hydroxyverbindungen der Untergruppen **(2)** und **(4)** unter den für die betreffenden Untergruppen **(2)**, **(3)** und **(4)** angegebenen Reaktionsbedingungen erhalten werden.

Durch Umsetzung von Oxiranen der Formel IIa mit Heterocyclen der Formel H-C, H-D oder H-E beziehungsweise von Oxiranen IIb mit H-E in einem Lösungsmittel wie Dimethylsulfoxid unter Anwesenheit einer Base wie Natriumhydrid bei erhöhter Temperatur, vorzugsweise bei 40^{o}C, können die Verbindungen der Untrgruppe **(6)** auch direkt, d.h. ohne Isolierung von Intermediaten der Untergruppen **(2)** und **(4)**, ausschließlich oder als Hauptprodukt erhalten werden.

Eventuell anfallende Gemische von Verbindungen der Untergruppe **(2)** und **(6)** beziehungsweise **(4)** und **(6)** können durch übliche Methoden wie Chromatographie oder Kristallisation in die reinen Komponenten aufgetrennt werden.

Verbindungen der allgemeinen Formel I, in denen R₁ und R₂ die angegebene Bedeutung haben, R₃ für Hydroxy oder - wie oben definiert - substituiertes Hydroxy und R₄ für Wasserstoff steht und R₅ für die oben definierten Heterocyclenreste A, B, C, D und E steht, wobei im Falle der Reste B, D und E, Y nicht für Schwefel steht und R₆' die Bedeutung Difluormethylthio, Trifluormethylthio oder 2,2,2-Trifluorethylthio hat, können durch geeignete Oxidationsmittel wie z.B. Wasserstoffperoxid in Eisessig oder Oxone^{R} in Methanol-Wasser-Gemischen bei Temperaturen zwischen 0^{o}C und der Rückflußtemperatur des Reaktionsgemisches, vorzugsweise bei Temperaturen zwischen 20 und 60^{o}C, in Verbindungen der Formel I übergeführt werden, in denen R₆ die Bedeutung Difluormethylsulfinyl, Difluormethylsulfonyl, Trifluormethylsulfinyl, Trifluormethylsulfonyl, 2,2,2-Trifluorethylsulfinyl oder 2,2,2-Trifluorethylsulfonyl hat. Eventuell anfallende Gemische von Sulfinyl- und Sulfonylverbindung können durch übliche Methoden wie Kristallisation oder Chromotographie rein erhalten werden.

Durch das für Verbindungen der Untergruppe **(5)** angegebene Verfahren läßt sich aus den Verbindungen der allgemeinen Formel I, in denen R₁ und R₂ die angegebene Bedeutung haben, R₃ für Hydroxy oder Wasserstoff und R₅ für die oben definierten Heterocyclenreste A, B, C, D und E steht, mit der Maßgabe, daß in den Resten B, C und E Y nicht für Schwefel steht, und R₆' die Bedeutung Difluormethylsulfinyl, Difluormethylsulfonyl, Trifluormethylsulfinyl, Trifluormethylsulfonyl, 2,2,2-Trifluorethylsulfinyl und Trifluormethylsulfonyl hat, z.B. durch Behandlung mit Natriumhydrid in siedendem Tetrahydrofuran, Verbindungen der allgemeinen Formel I erhalten, bei denen R₁ und R₂ die oben angegebene Bedeutung haben, R₃ und R₄ zusammen eine Bindung bilden und R₅ und R₆ die vorstehende Bedeutung haben.

Durch das für Verbindungen der Untergruppe **(5)** angegebene Verfahren, lassen sich aus den durch vorstehend beschriebenes Verfahren erhaltenen Verbindungen mittels Natriumhydrid in Tetrahydrofuran bei Rückflußtemperatur des Reaktionsgemisches Verbindungen der allgemeinen Formel I erhalten, bei denen R₃ und R₄ zusammen eine Bindung bilden, wobei R₁, R₂, R₅ die oben angegebene Bedeutung haben, mit der Einschränkung, daß Y nicht für Schwefel steht und R₆' die Bedeutung Difluormethylsulfinyl, Difluormethylsulfonyl, Trifluormethylsulfinyl, Trifluormethylsulfonyl, 2,2,2-Trifluorethylsulfinyl oder 2,2,2-Trifluorethylsulfonyl hat.

Oxirane der Formel IIa und IIb können - bevorzugt **in situ** - aus Verbindungen der Formel IIIa und IIIb 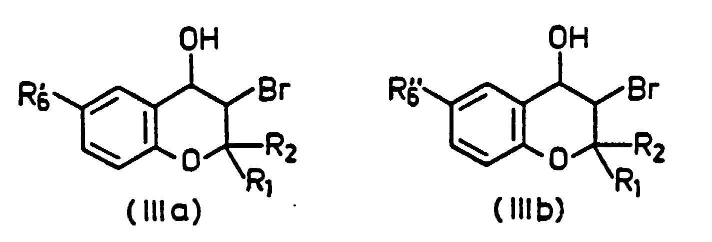 durch Umsetzung mit einer Base wie Natriumhydrid in einem Lösungsmittel wie DMSO bei Temperaturen zwischen 0 und 80^{o}C vorzugsweise bei 20-25^{o}C hergestellt werden. In diesen Fällen ist es vorteilhaft, die Heterocyclen H-C, H-D und H-E erst dann der Reaktionsmischung zuzusetzen, wenn die Oxiranbildung abgeschlossen ist.

Falls eine Isolierung des Oxirans IIa und IIb erwünscht ist, kann die Umsetzung der Bromhydrine IIIa oder IIIb auch in einem geeigneten Ether wie Diethylether, Dioxan oder Tetrahydrofuran mit einer Base wie Kaliumhydroxid oder Natriumhydrid oder in wäßrigen Mischungen wassermischbarer Ether und einer Base wie Kaliumhydroxid erfolgen. In diesen Fällen ist die Verwendung von Natriumhydrid in Tetrahydrofuran bevorzugt.

Verbindungen der allgemeinen Formel IIIa und IIIb, bei denen Bromatom und Hydroxygruppe **trans** zueinander angeordnet sind, können nach an sich üblichen Verfahren herstellt werden. Ein solches Verfahren kann wie folgt dargestellt werden und ist mehrfach beschrieben worden, z.B. EP 0 076 075, J. Org. Chem. **38**, (22), 3832 (1973), ibid. **39** (7), 881 (1974), ibid. **37** (6), 841 (1972):

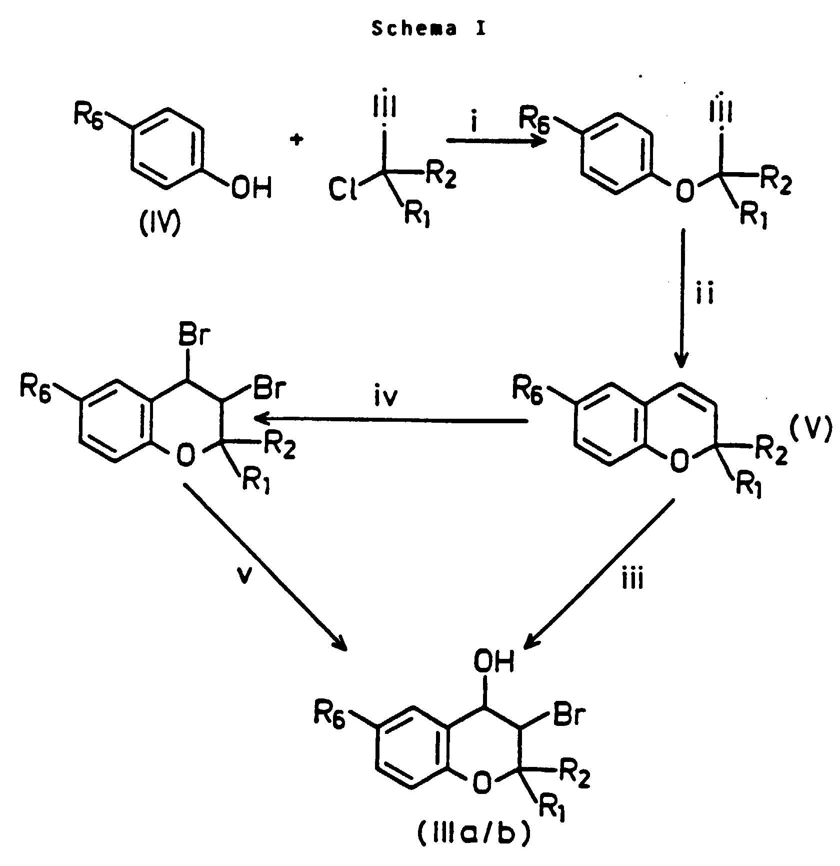

Die folgenden Bedingungen werden bevorzugt:
i) z.B. K₂CO₃/Aceton; Rückfluß
   oder K₂CO₃/Butanon; Rückfluß
   oder K₂CO₃/Dimethylformamid, 90^{o}C
   oder NaOH/40% Triethylbenzylammoniumhydroxid in Methanol; Raumtemperatur
ii) z.B. 1,2-Dichlorbenzol, 180^{o}C
   oder N,N' N,N'Diethylanilin, 200°C
   oder ohne Lösungsmittel, 200^{o}C
iii) N-Bromsuccinimid / Dimethylsulfoxid / Wasser
iv) Brom, Tetrachlormethan
v) Aceton / Wasser

Die 4-substituierten Phenole IV, in denen R₆ die oben angegebene Bedeutung hat, sind bekannt oder können nach bekannten Verfahren hergestellt werden, z.B. durch Reduktion der entsprechenden 4-substituierten Nitroaromaten, beispielsweise mit Wasserstoff und Raneynickel als Katalysator oder durch nascierenden Wasserstoff zu den entsprechenden 4-substituierten Anilinen und Diazotierung und Verkochung derselben zu den besagten 4-substituierten Phenolen.

In Fällen, in denen R₆ die Bedeutung Difluormethylthio, Difluormethylsulfinyl, Difluormethylsulfonyl, Trifluormethylthio, Trifluormethylsulfinyl, Trifluormethylsulfonyl, 2,2,2-Trifluormethylthio, 2,2,2-Trifluorethylsulfinyl und 2,2,2-Trifluorethylsulfonyl hat, können die genannten Restehäufig vorteilhafter durch chemische Transformationen von Zwischenprodukten, in denen R₆ eine andere als die obengenannte Bedeutung hat, eingeführt werden, z.B. werden 2H-Benzo(b)pyrane der allgemeinen Formel V, in denen R₆ die Bedeutung Difluormethylsulfonyl, Trifluormethylsulfonyl und 2,2,2-Trifluorethylsulfonyl hat, durch Umsetzung der entsprechenden Fluoralkylsulfonylfluoride mit 2H-Benzo(b)pyranen der allgemeinen Formel V, in denen R₆ die Bedeutung MgHal hat, wobei Hal die Bedeutung Chlor, Jod und insbesondere Brom hat, erhalten.

Ebenso ist es möglich, die oben beschriebenen Grignard-Verbindungen von 2H-Benzo(b)pyranen mit Disulfiden der allgemeinen Formel R-S-S-R, in denen R die Bedeutung Trifluormethyl, Difluormethyl und 2,2,2-Trifluorethyl hat, zu 2H-Benzo(b)pyranen, in denen R₆ die Bedeutung Difluormethylthio, Trifluormethylthio und 2,2,2-Trifluorethylthio hat, umzusetzen.

Weiterhin ist es möglich, ausgehend von Zwischen- oder Endprodukten, in denen R₆ ein Schwefelatom enthält, nach an sich üblichen Methoden durch Reduktion und insbesondere Oxidation zu Zwischen- oder Endprodukten, in denen das bezeichnete Schwefelatom eine andere Oxidationsstufe aufweist, zu gelangen.

Als mögliche Oxidationsmittel seinen beispielhaft genannt: Kaliumpermanganat, Natriumperiodat, Chromtrioxid oder bevorzugt Oxone^{R} (Kaliummonopersulfat) bzw. Wasserstoffperoxid/Eisessig.

So kann z.B. 4-Difluormethylsulfonyl-phenol, 4-Trifluormethylsulfonyl-phenol oder 4-(2,2,2-Trifluorethylsulfonyl)-phenol günstiger als nach bekannten Verfahren durch Oxidation der entsprechenden Fluoralkylthiophenole mit Oxone^{R} in Methanol-Wasser-Gemischen bei Temperaturen zwischen -10^{o}C und der Rückflußtemperatur des Reaktionsgemisches, vorzugsweise bei Temperaturen zwischen 0^{o}C und 25^{o}C erhalten werden.

Inbesondere in Fällen, in denen R₆ die Bedeutung Difluormethylsulfinyl, Trifluormethylsulfinyl oder 2,2,2-Trifluorethylsulfinyl hat, ist es günstiger, ausgehend von den entsprechenden 4-Fluoralkylthiophenolen gemäß Schema I zunächst die entsprechenden 6-Fluoralkyl-2H-benzo(b)-pyrane V, bei denen R₁ und R₂ die oben angegebene Bedeutung haben, herzustellen und anschließend die gewünschte Oxidation zu den jeweiligen 6-Fluoralkylsulfinyl-2H-benzo(b)pyranen der allgemeinen Formel V, bei denen R₆ die obengenannte Bedeutung hat, vorzunehmen. Überraschenderweise ist die Selektivität dieser Reaktion bei den genannten Verbindungsklassen sehr hoch.

Die verwendeten Ausgangsmaterialien sind bekannt oder können nach an sich bekannten Verfahren bzw. analog zu den hier beschriebenen oder analog zu an sich bekannten Verfahren hergestellt werden.

Die Verbindungen der Formel I können sowohl Basen als auch Säuren beziehungweise amphoter sein und daher in der Form ihrer Salze oder Säureadditionssalze aus den Reaktionsgemischen isoliert werden. Sie lassen sich als Basen mit geeigneten anorganischen oder organischen Säuren nach bekannten Verfahren in Salze überführen oder bilden als Säuren mit Basen Salze.

Bevorzugt werden physiologisch verträgliche Salze oder Säureadditionssalze. Hierfür sind als anorganische Säuren beispielsweise Schwefelsäure oder Halogenwasserstoffsäuren, zum Beispiel Salzsäure, und als organische Säuren, zum Beispiel Fumarsäure, Maleinsäure, Zitronensäure und Weinsäure, geeignet. Zur Herstellung wird die heiße alkoholische Lösung der Base mit der alkoholischen Lösung einer geeigneten Säure versetzt, und man erhält nach Etherzusatz das Salz. Bevorzugte Salze sind die Alkali-, Erdalkali- und Ammoniumsalze der Verbindungen der Formel I, die mit den entsprechenden Basen, insbesondere Natrium-, oder Kaliumhydroxid erhalten werden.

Die erfindungsgemäßen Verbindungen der Formel I können Chiralitätszentren aufweisen und können, abhängig von den Substituenten, weitere asymmetrische Schwefel- oder Kohlenstoffatome besitzen und daher als Racemate und Diastereoisomere vorliegen. Diastereoisomere können in bekannter Weise aufgrund der physikalisch-chemischen Unterschiede ihrer Bestandteile in ihre racemischen Modifikationen getrennt werden. Racemate können nach bekannten Methoden getrennt werden, beispielsweise durch Umkristallisieren in optisch aktiven Lösungsmitteln, durch Mikroorganismen oder Reaktion mit einer optisch aktiven Säure oder Base, die mit der racemischen Verbindung ein Salz bildet, Trennung der Diastereoisomeren durch fraktionierte Kristallisation und Freisetzung der Enantiomeren durch geeignete Mittel. Besonders geeignete optisch aktive Säuren sind beispielsweise die d- und l-Formen der Weinsäure, Ditoluylweinsäure, Äpfelsäure, Mandelsäure, Kamphersulfonsäure oder Pyrrolidon-carbonsäure. Geeignete optisch aktive Basen sind alpha-Phenylethylamin, Menthylamin, Ephedrin, Brucin und Chinin. Vorteilhafterweise wird der aktivere der Antipoden isoliert. Gemäß der Erfindung ist es jedoch auch möglich, die reinen Enantiomeren durch asymmetrische Synthese zu erhalten.

Die folgenden Herstellungsverfahren werden besonders bevorzugt:
Verfahren zur Herstellung der Verbindungen gemäß Anspruch 1, dadurch gekennzeichnet, daß man
a) Oxirane der allgemeinen Formel IIa, in denen R₁, R₂ und R₆' die oben angegebene Bedeutung haben, mit Anionen C⁻ und D⁻ von Heterocyclen der allgemeinen Formel H-C und H-D, in denen X die Bedeutung Sauerstoff und Y, k, m, n die oben angegebene Bedeutung haben, mit der Einschränkung, daß für jene Heterocyclen H-D, für die m ungleich 0 gilt, Y nicht für unsubstituiertes Amino steht, zu Verbindungen der allgemeinen Formel I, in denen R₁, R₂ und R₆' die oben angegebene Bedeutung haben, R₃ für Hydroxy, R₄ für Wasserstoff und R₅ für die Heterocyclenreste C und D, in denen X für Sauerstoff steht und Y, k, m, n die oben angegebene Bedeutung haben, mit der Ausnahme, daß Y nicht für unsubstituiertes Amino steht, falls m ungleich 0 ist, umsetzt, wobei die Substituenten R₃ und R₅ der Verbindungen der Formel I **trans** zueinander angeordnet sind und daß man
b) jene erfindungsgemäße Verbindungen gemäß a) für die Y für substituiertes Amino und m für 1 oder 2 steht, durch Hydrolyse, Hydrogenolyse oder Dealkylierung in Verbindungen der Formel I gemäß a) überführt, in denen Y auch dann für unsubstituiertes Amino steht, falls m ungleich 0 gilt, wobei wiederum R₃ und R₅ **trans** zueinander angeordnet sind und daß man
c) Oxirane der allgemeinen Formel IIa gemäß a) mit Aminen der allgemeinen Formel H-A und H-B zu Verbindungen der Formel I, in denen R₁, R₂ und R₆' die oben angegebene Bedeutung haben und R₅ für A oder B, mit der für n und Y oben angegebenen Bedeutung steht, R₄ für Wasserstoff und R₃ für Hydroxy steht, umsetzt, wobei R₃ und R₅ **trans** zueinander angeordnet sind und daß man
d) Oxirane der allgemeinen Formel IIa und IIb, in denen R₁, R₂, R₆' und R₆" die oben angegebene Bedeutung haben, mit Anionen E⁻ von Heterocyclen der allgemeinen Formel H-E, in denen Y, Z und p die oben angegebene Bedeutung haben, zu Verbindungen der allgemeinen Formel I, in denen R₁, R₂, R₆' und R₆" die oben angegebene Bedeutung haben, R₃ für Hydroxy, R₄ für Wasserstoff und R₅ für E mit der für Y, Z und p angegebenen Bedeutung steht, umsetzt, wobei R₃ und R₅ **trans** zueinander angeordnet sind und daß man
e) erfindungsgemäße Verbindungen der Formel I gemäß a) und b) mit Schwefelungsreagentien, z.B. bevorzugt Lawessons Reagens in einem inerten Solvens wie z.B. Toluol zu Verbindungen der Formel I, in denen R₁, R₂, R₃, R₄, und R₆' wie unter a) definiert sind, R₅ für die Reste C, D mit der für Y, k, m, n angegebenen Bedeutung steht, wobei jedoch X für Schwefel steht, umsetzt und daß man
f) erfindungsgemäße Verbindungen der Formel I gemäß a), d) und e), sowie jene erfindungsgemäßen Verbindungen gemäß c), bei denen Y nicht für unsubstituiertes Amino steht, durch Umsetzung mit Alkylhalogeniden, -mesylaten, -brosylaten, -tosylaten, Acylhalogeniden, -anhydriden, -imidazoliden, Alkylcarbonylhalogeniden oder -anhydriden, Mono- oder Dialkylaminocarbonylhalogeniden, Phosgen oder Alkylisocyanaten in Verbindungen der allgemeinen Formel I, in denen R₁, R₂, R₄, R₆' R₆" und R₅ gemäß a), c), d) und e) definiert sind, mit der Einschränkung, daß Y im Rest B nicht für unsubstituiertes Amino steht, und R₃ für C₁₋₆-Alkoxy, Formyloxy, C₁₋₈-Alkylcarbonyloxy, C₁₋₈-Monoalkylamino, C₁₋₈-Dialkylamino steht, umwandelt und daß man
g) jene erfindungsgemäßen Verbindungen der Formel I gemäß f), bei denen Y für substituiertes, insbesondere benzylsubstituiertes Amino steht, durch geeignete Maßnahmen, insbesondere Hydrogenolyse in Verbindungen der Formel I gemäß f) überführt, bei denen Y für unsubstituiertes Amino steht und daß man
h) erfindungsgemäße Verbindungen der Formel I gemäß a), b), d) und e) in Anwesenheit eines dehydratisierenden Agens wie z.B. Natriumhydrid in einem inerten Solvens wie z.B. Tetrahydrofuran zu Verbindungen der allgemeinen Formel I umsetzt, in der R₁, R₂, R₅, R₆' und R₆" die oben angegebene Bedeutung haben und R₃ zusammen mit R₄ eine Bindung bildet und daß man
i) Oxirane der allgemeinen Formeln IIa bzw. IIb, in denen R₁, R₂ und R₆' bzw. R₆" die oben angegebene Bedeutung haben unter Anwesenheit von mindestens zwei Äquivalenten einer Base zunächst zu erfindungsgemäßen Verbindungen gemäß a) und d) umsetzt, diese jedoch nicht isoliert, sondern durch Verlängerung der Reaktionszeit und insbesondere durch Erhöhen der Reaktionstemperatur, auf vorzugsweise 40°C, in situ in erfindungsgemäße Verbindungen gemäß h) überführt und diese nach an sich üblichen Verfahren isoliert und daß man
j) jene erfindungsgemäßen Verbindungen gemäß a), b), c), d), f), g) und h), in denen R₆' die Bedeutung Difluormethylthio, Trifluormethylthio und 2,2,2-Trifluorethylthio und gleichzeitig X und Y eine andere Bedeutung als Schwefel hat mit geeigneten Oxidationsmitteln, bevorzugt Oxone^{R} bzw. Wasserstoffperoxid/Eisessig in jene erfindungsgemäße Verbindungen gemäß a), b), c), d), g) und h) überführt, in denen R₆' die Bedeutung Difluormethylsulfinyl, Trifluormethylsulfinyl, 2,2,2-Trifluorethylsulfinyl und insbesondere Trifluormethylsulfonyl, 2,2,2-Trifluorethylsulfonyl, Difluormethylsulfonyl hat bzw. eventuell anfallende Gemische von Sulfinyl- und Sulfonyl-Verbindung durch an sich übliche Verfahren in die reinen Komponenten auftrennt.

Die folgenden Beispiele sollen die Erfindung verdeutlichen:

### BEISPIEL A

### 6-Cyano-3,4-dihydro-2,2-dimethyl-trans-4-(2-cyanimino-3-imidazolidin-1-yl)-2H-benzo(b)pyran-3-ol

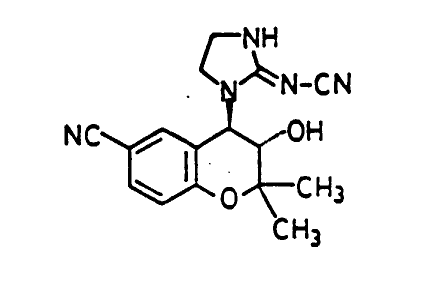

### Methode A:

2,41 g (0,012 Mol) 6-Cyano-3,4-dihydro-3,4-oxiranyl-2,2-dimethyl-2H-benzo(b)pyran und 1,76 g (0,016 Mol) 2-Cyanimino-imidazolidin werden in 25 ml trockenem Dimethylsulfoxid gelöst und portionsweise mit 0,38 g (0,016 Mol) entöltem Natriumhydrid versetzt, so daß die Temperatur 25°C nicht überschreitet. Man läßt 72 Stunden bei Raumtemperatur unter Schutzgasatmosphäre rühren und hydrolysiert vorsichtig mit 80 ml Wasser. Die ausgefallenen Kristalle werden mit Wasser nachgewaschen, getrocknet und aus Diisopropylether umkristallisiert.
Ausbeute: 1,12 g (30% d. Th.) 6-Cyano-3,4-dihydro-2,2-dimethyl-trans-4-(2-cyanimino-3-imidazolidin-1-yl)-2H-benzo(b)pyran-3-ol)
Smp. 250°C (dec.)

### Methode B:

Die gemäß Methode A nach Hydrolyse erhaltene Wasserphase wird mit Ethylacetat extrahiert (3 x 100 ml) und die vereinigten Phasen werden eingeengt. Der Rückstand wird in Toluol aufgenommen, die organische Phase mit Wasser gewaschen (3 x 100 ml), über Natriumsulfat getrocknet und eingeengt. Der Rückstand wird umkristallisiert (Ethylacetat/Diisopropylether) oder durch Säulenchromatographie (Kieselgel Si 60, Chloroform/Methanol 95:5) gereinigt und man erhält die Verbindung des Beispiels 1.

Analog wurden nach Methode A oder B (s. Tab. 1) aus dem entsprechenden Epoxid und dem entsprechenden Amid-, Carbamid-, Thiocarbamid-, Harnstoff-, Cyanguanidin- bzw. Nitromethyliden-Heterocyclus die Verbindungen der Beispiele 1, 2, 6, 8 bis 17, 21, 23 bis 25, 27, 30, 32 bis 35, 37 und 41 bis 43 erhalten.

### Beispiel B

### 6-Difluormethoxy-3,4-dihydro-2,2-dimethyl-trans-4-(1-pyrrolidinyl)-2H-benzo(b)pyran-3-ol

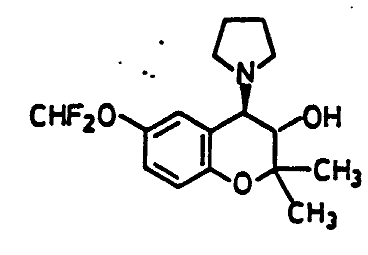

### Methode C:

2,42 g (10 mMol) 6-Difluormethoxy-3,4-dihydro-3,4-oxiranyl-2,2-dimethyl-2H-benzo(b)pyran und 0,85 g (12 mMol) Pyrrolidin werden in 30 ml trockenem Ethanol 20 Stunden bei Rückflußtemperatur gehalten. Anschließend wird das Lösungsmittel im Vakuum entfernt, der Rückstand mit Hexan versetzt und mit etherischer HCl-Lösung als Hydrochlorid gefällt. Man erhält 350 mg farblose Kristalle (10 % d. Th.) 6-Difluormethoxy-3,4-dihydro-2,2-dimethyl-trans-4-(1-pyrrolidinyi)-2H-benzo(b)pyran-3-ol.
Smp. 176-178°C

Analog wurden Beispiel 4 und Beispiel 5 erhalten. Die Verbindung des Beispiels 5 wurde als freie Base aus Hexan/Ether umkristallisiert.

### Beispiel C

### 6-Cyano-2,2-dimethyl-(2-cyanimino-3-thiazolidin-1-yl)-2H-benzo(b)pyran

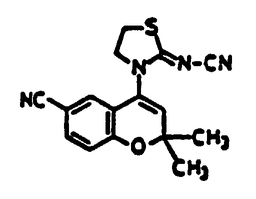

2,41 g (0,012 Mol) 6-Cyano-3,4-dihydro-3,4-oxiranyl-2,2-dimethyl-2H-benzo (b)pyran und 2,03 g (0,016 Mol) 2-Cyanimino-thiazolidin wurden, wie oben beschrieben, mit 0,38 g (0,016 Mol) Natriumhydrid in DMSO bei 45°C umgesetzt. Nach 100 Stunden wurde hydrolysiert und nach Methode B aufgearbeitet. Man erhält 186 mg farblose Kristalle 6-Cyano-2,2-dimethyl-(2-cyanimino-3-thiazolidin-1-yl)- 2H-benzo(b)pyran, Smp. 230-232°C (Ausbeute: 5% d. Th.).

Analog wurde Beispiel 7, 20, 22, 26, 28, 29, 31, 36 und 38 erhalten (s. Tab. 1).

### Beispiel D

### 6-Difluormethoxy-3,4-dihydro-2,2-dimethyl-trans-4-(2-oxo-1-piperidinyl)-2H-benzo(b)pyran-3-ol

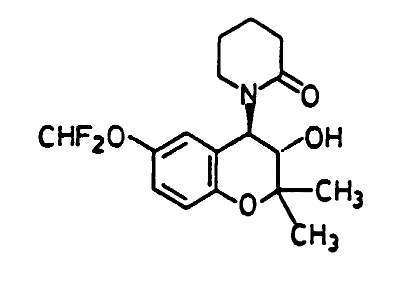

2,78 g (0,012 Mol) 6-Difluor-3,4-dihydro-3,4-oxiranyl-2,2-dimethyl-2H-benzo(b)pyran wurden nach Methode A mit 1,58 g (0,016 Mol) 2-Oxopiperidin umgesetzt. Man erhält 2,05 g 6-Difluormethoxy-3,4-dihydro-2,2-dimethyl-trans-4-(2-oxo-1-piperidinyl)-2H-benzo(b)pyran-3-ol als farblose Kristalle, Smp. 165°C (Ausbeute 50% d. Th.).

### Beispiel E

### 6-Trifluormethylsulfinyl-3,4-dihydro-2,2-dimethyl-trans-4-(2-oxo-1-piperidinyl)-2H-benzo(b)pyran-3-ol

Das beschriebene Epoxid (Beispiel 13') wird mit 2-Piperidinon entsprechend Methode A umgesetzt und nach Methode B aufgearbeitet.
Ausbeute: 1,1 g (42% d. Th.) der in der überschrift genannten Verbindung.

### Beispiel F

### 6-Trifluormethylsulfonyl-3,4-dihydro-2,2-dimethyl-trans-4-(2-oxo-1-piperidinyl)-2H-benzo(b)pyran-3-ol und 6-Trifluormethylsulfinyl-3,4-dihydro-2,2-dimethyl-trans-4-(2-oxo-1-piperidinyi)-2H-benzo(b)pyran-3-ol

0,2 g (5,3 mMol) 6-Trifluormethylthio-3,4-dihydro-2,2-dimethyl-trans-4-(2-oxo-1-piperidinyl)-2H-benzo(b)pyran-3-ol werden 7 ml Methanol gelöst und bei 0°C unter Rühren wird eine Suspension von 1 g Oxone^{R} in 5 ml Wasser zugegeben. Nach 5 Tagen Rühren bei Raumtemperatur wird mit 20 ml Wasser verdünnt und mit Chloroform (50 ml) extrahiert. Nach Trocknen und Eindampfen verbleiben 220 mg weiße Kristalle. Mittels HPLC (Laufmittel 98 : 2 Chloroform:Methanol) erhält man 140 mg (64% d. Th.) 6-Trifluormethylsulfonyl-3,4-dihydro-2,2-dimethyl-trans-4-(2-oxo-1-piperidinyl)-2H-benzo(b)pyran-3-ol und 15 mg (7% d. Th.) 6-Trifluormethylsulfinyl-3,4-dihydro-2,2-dimethyl-trans-4-(2-oxo-1-piperidinyl)-2H-benzo(b)pyran-3-ol.

### Beispiel G

### 6-Difluormethoxy-3,4-dihydro-2,2-dimethyl-trans-4-(2-oxo-4-piperazin-1-yl)-2H-benzo(b)pyran-3-ol

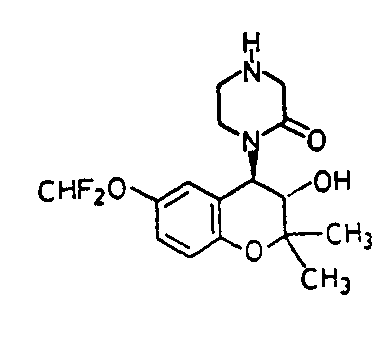

1,77 g (4,1 mMol) der Verbindung Nr. 165 des Beispiels 28 wurden in 100 ml Methanol/Wasser/Eisessig (83/15/2) gelöst und unter Zusatz von 200 mg Pearlman's Katalysator 3,5 Stunden bei Raumtemperatur und 10 bar Wasserstoffdruck im Autoklaven hydriert. Der Katalysator wurde abfiltriert, das Filtrat eingeengt und mit ethanolischer Chlorwasserstofflösung behandelt. Nach Eindampfen wurde aus Aceton umkristallisiert. Man erhält 1,2 g farblose Kristalle 6-Difluormethoxy-3,4-dihydro-2,2-dimethyl-trans-4-(2-oxo-4-piperazin-1-yl)-2H-benzo(b)pyran-3-ol, Smp. 240°C (dec.), (Ausbeute 77,5% d. Th.).

### Beispiel H

### 6-Trifluormethylsulfonyl-3,4-dihydro-2,2-dimethyl-trans-4-(2-oxo-1-pyrrolidinyt)-2H-benzo(b)-pyran-3-ol und 6-Trifluormethylsulfinyl-3,4-dihydro-2,2-dimethyl-trans-4-(2-oxo-1-pyrrolidinyl)-2H-benzo(b)pyran-3-ol

0,2 g (5,5 mMol) 6-Trifluormethylthio-3,4-dihydro-2,2-dimethyl-trans-4-(2-oxo-1-pyrrolidinyl)-2H-benzo(b)pyran-3-ol werden in 7 ml Methanol gelöst und bei 0°C unter Rühren wird eine Suspension von 1 g Oxone^{R} in 5 ml Wasser zugegeben. Nach 5 Tagen Rühren bei Raumtemperatur wird mit 20 ml Wasser verdünnt und mit Chloroform extrahiert (3 x 50 ml). Nach Trocknen und Eindampfen verbleiben 0,2 g weiße Kristalle. Mittels HPLC (Laufmittel Chloroform: Methanol 98:2) erhält man 120 mg (55% d.Th.) 6-Trifluormethylsulfonyl-3,4-dihydro-2,2-dimethyl-trans-4-(2-oxo-1-pyrrolidinyl)-2H-benzo(b)pyran-3-ol und 15 mg 6-Trifluormethylsulfinyl-3,4-dihydro-2,2-dimethyl-trans-4-(2-oxo-1-pyrrolidinyl)-2H-benzo(b)pyran-3-ol.

### Beispiel I

### 6-Trifluormethylsulfinyl-3,4-dihydro-2,2-dimethyl-trans-4-(2-oxo)-1-pyrrolidinyl)-2H-benzo(b)pyran-3-ol

Das oben beschriebene Epoxid (Beispiel 13') wurde mit 2-Pyrrolidinon entsprechend Methode A umgesetzt und nach Methode B aufgearbeitet.
Ausbeute: 1,2 g (48% d. Th.) der in der überschrift genannten Verbindung.

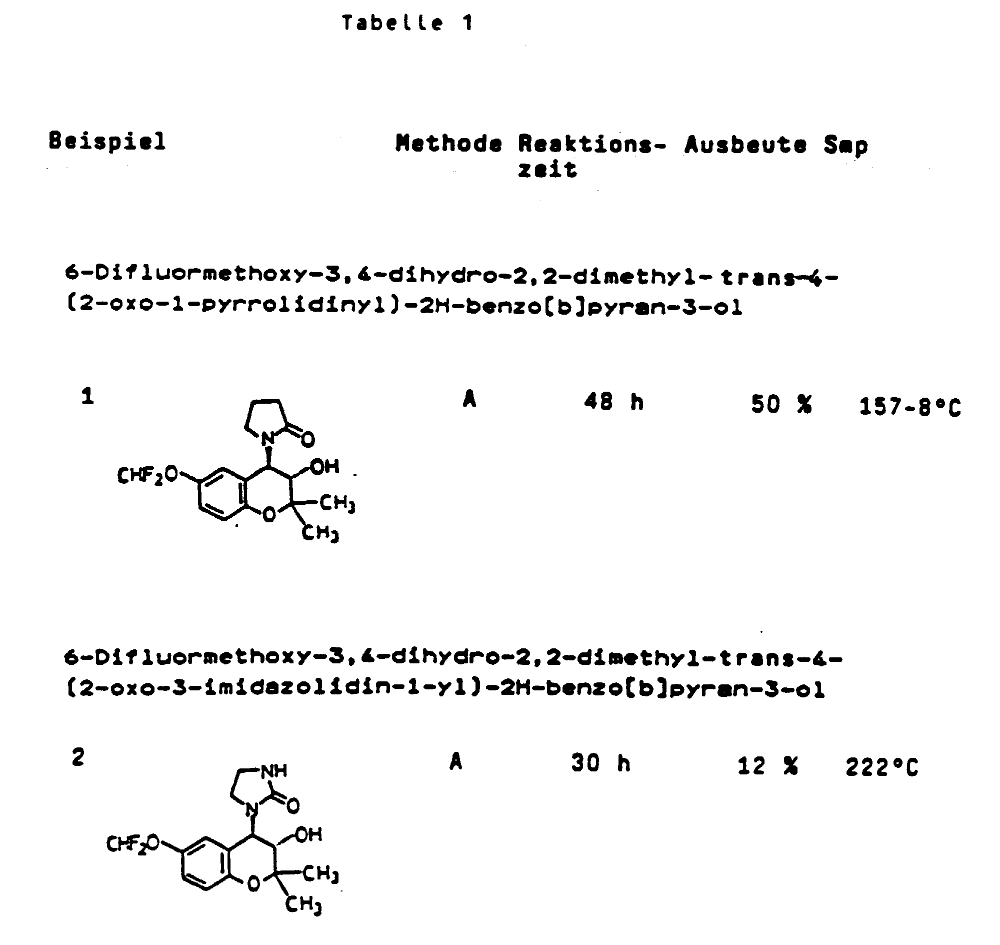

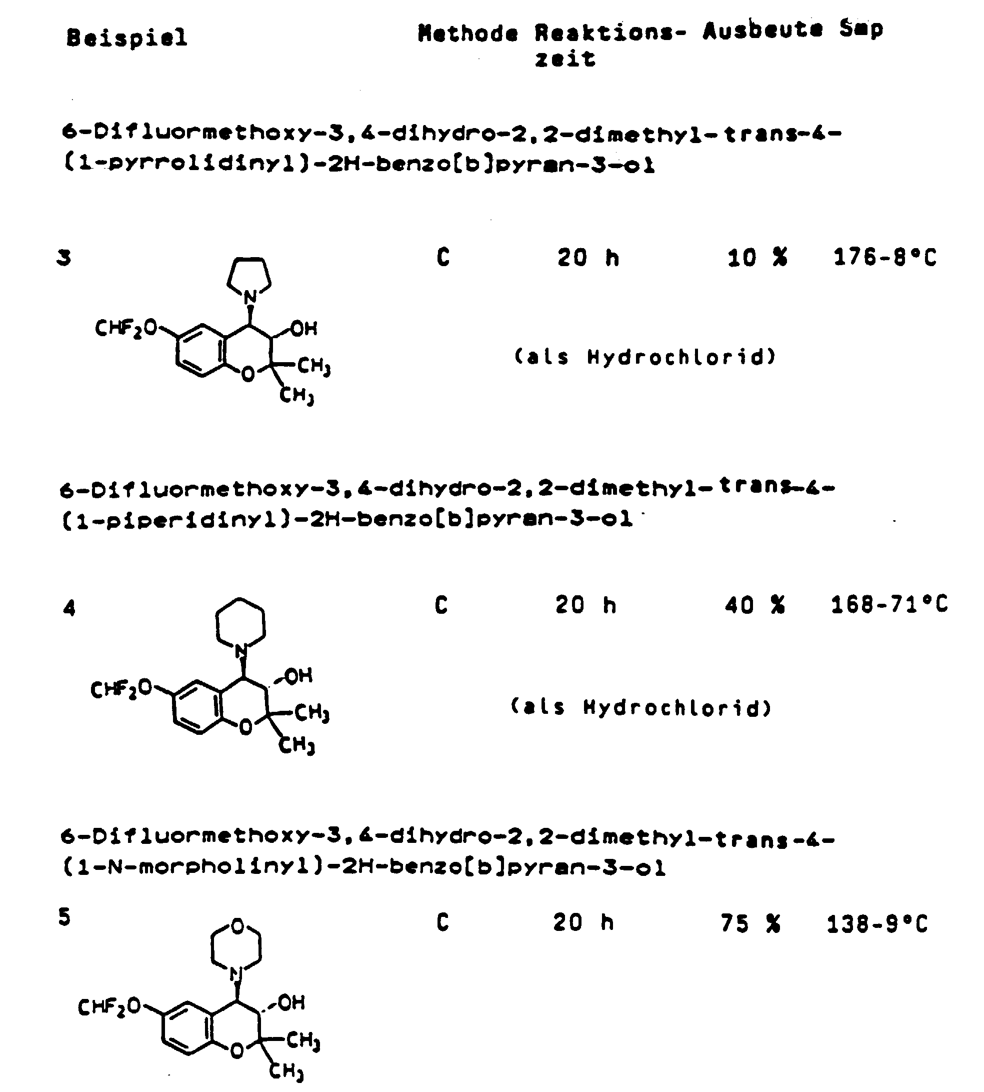

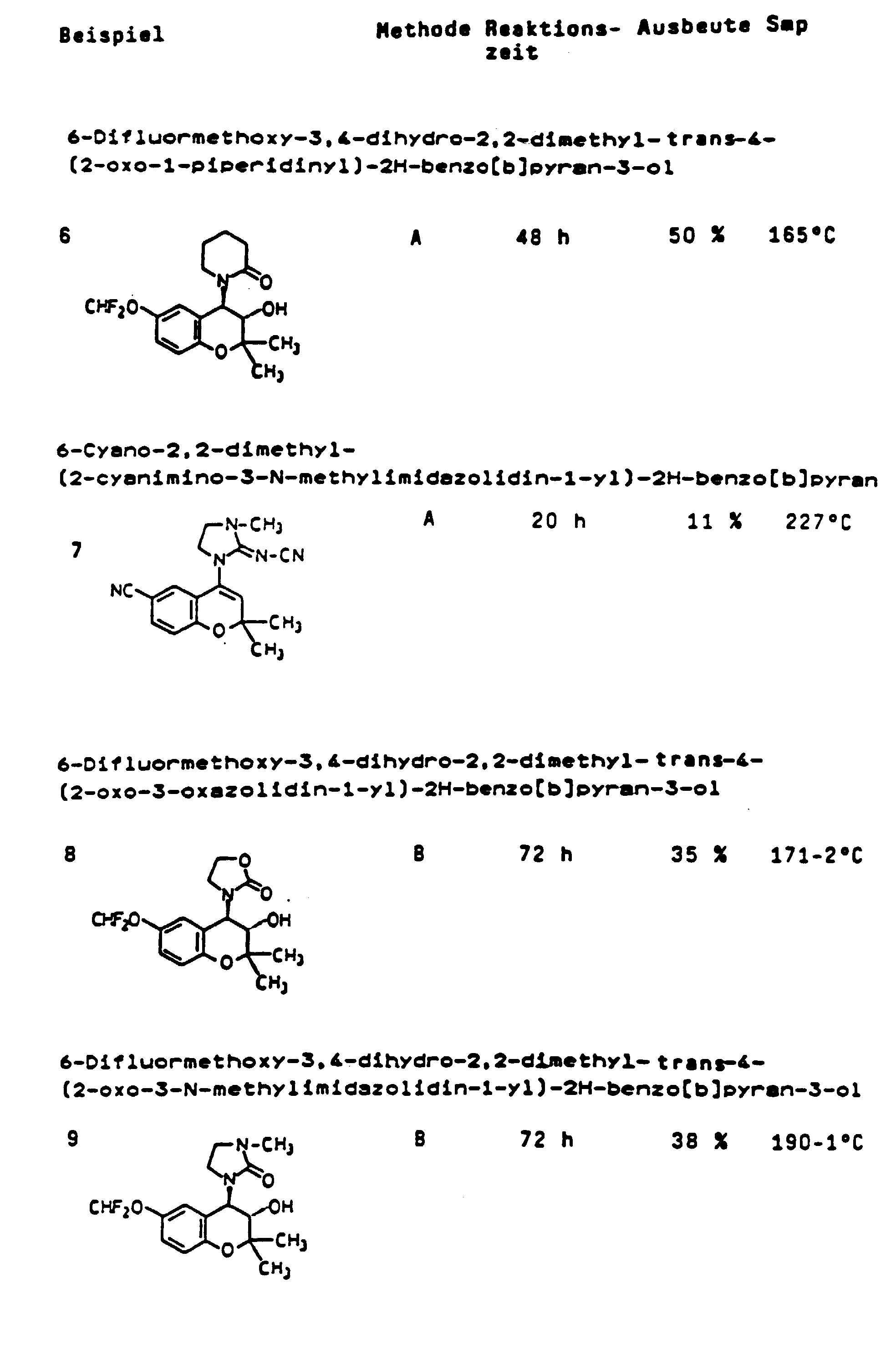

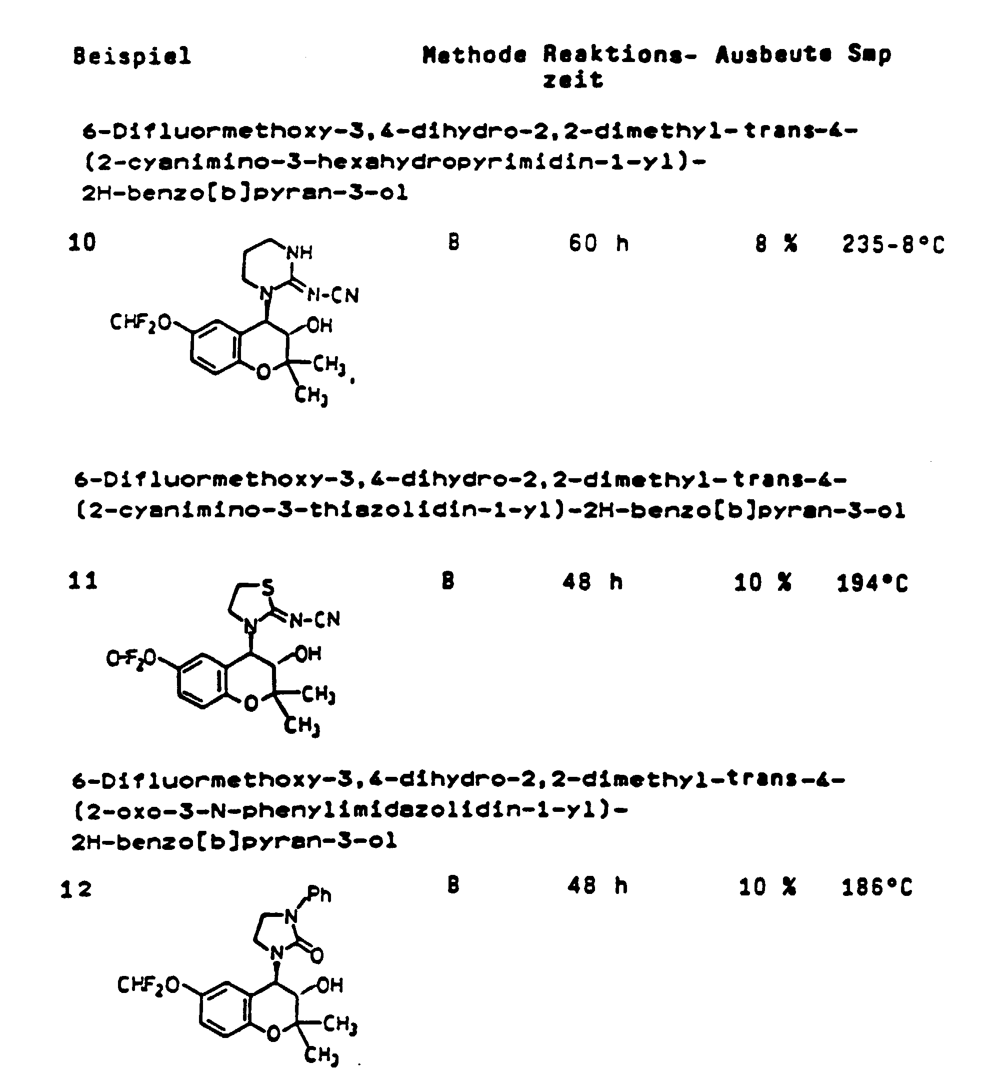

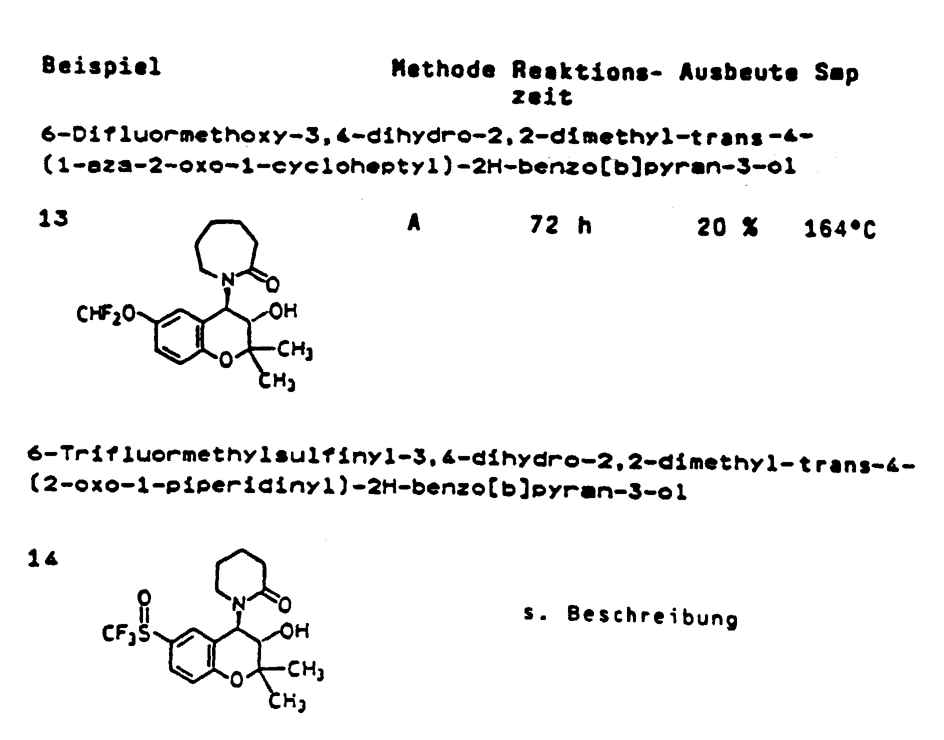

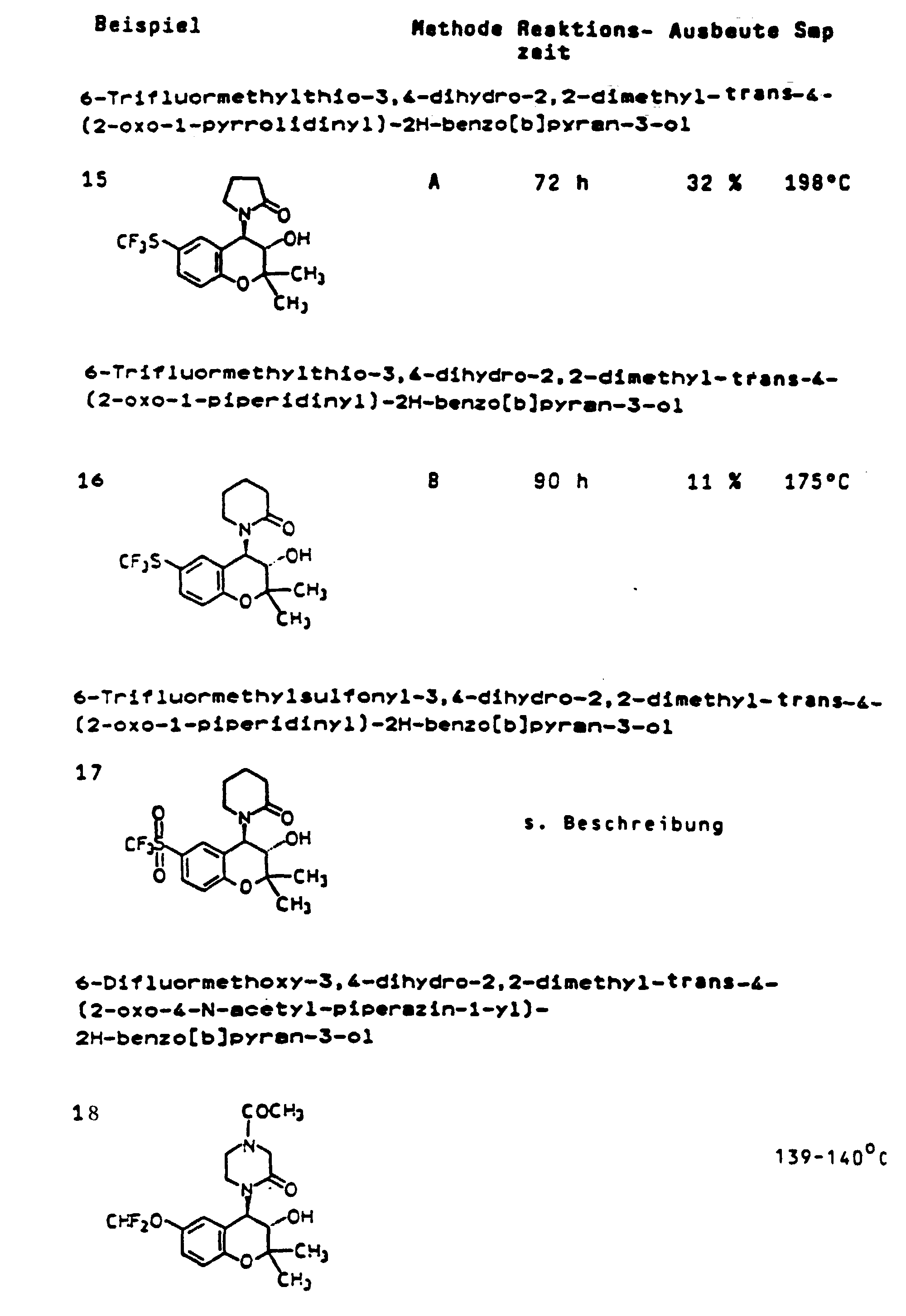

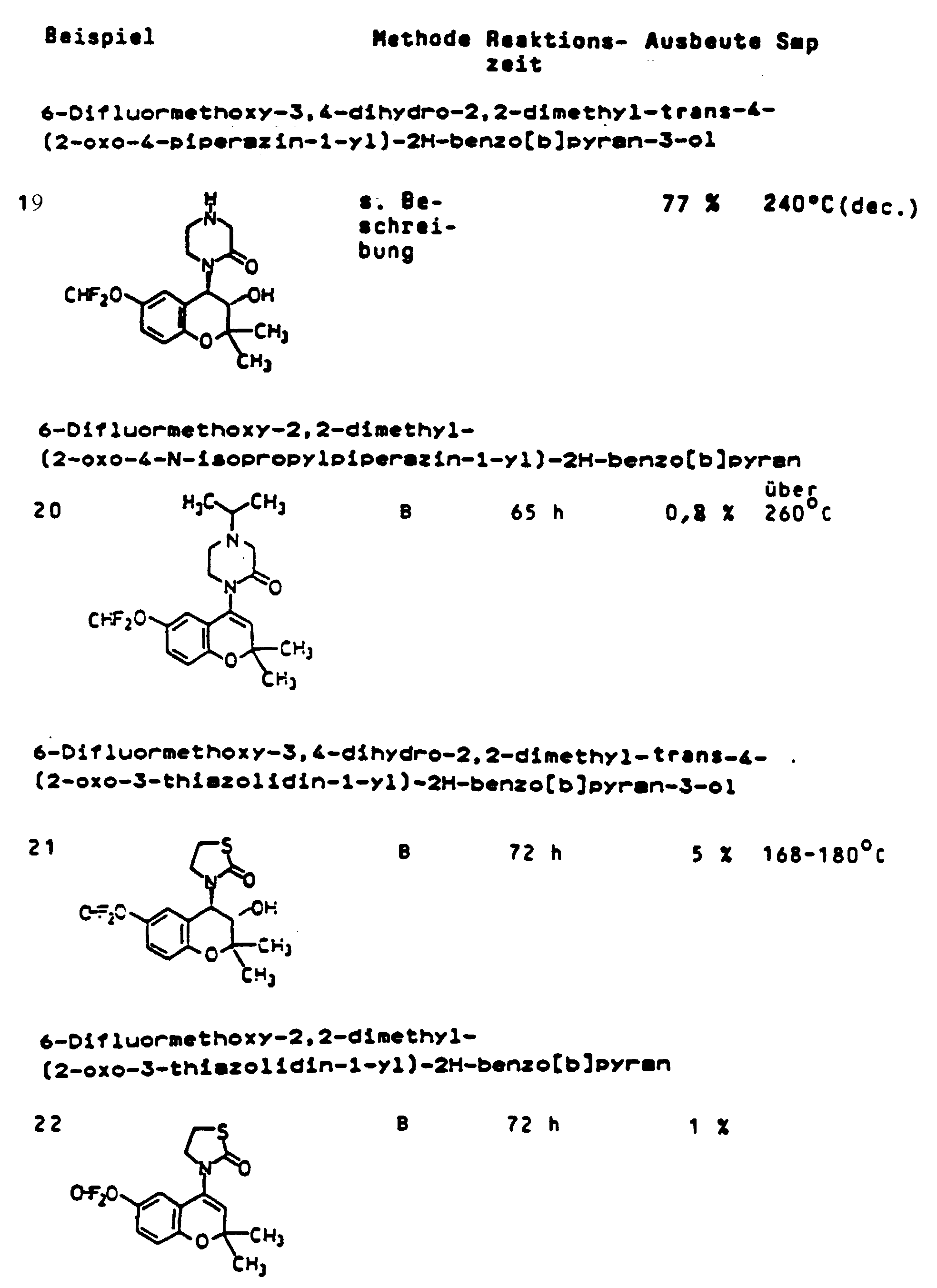

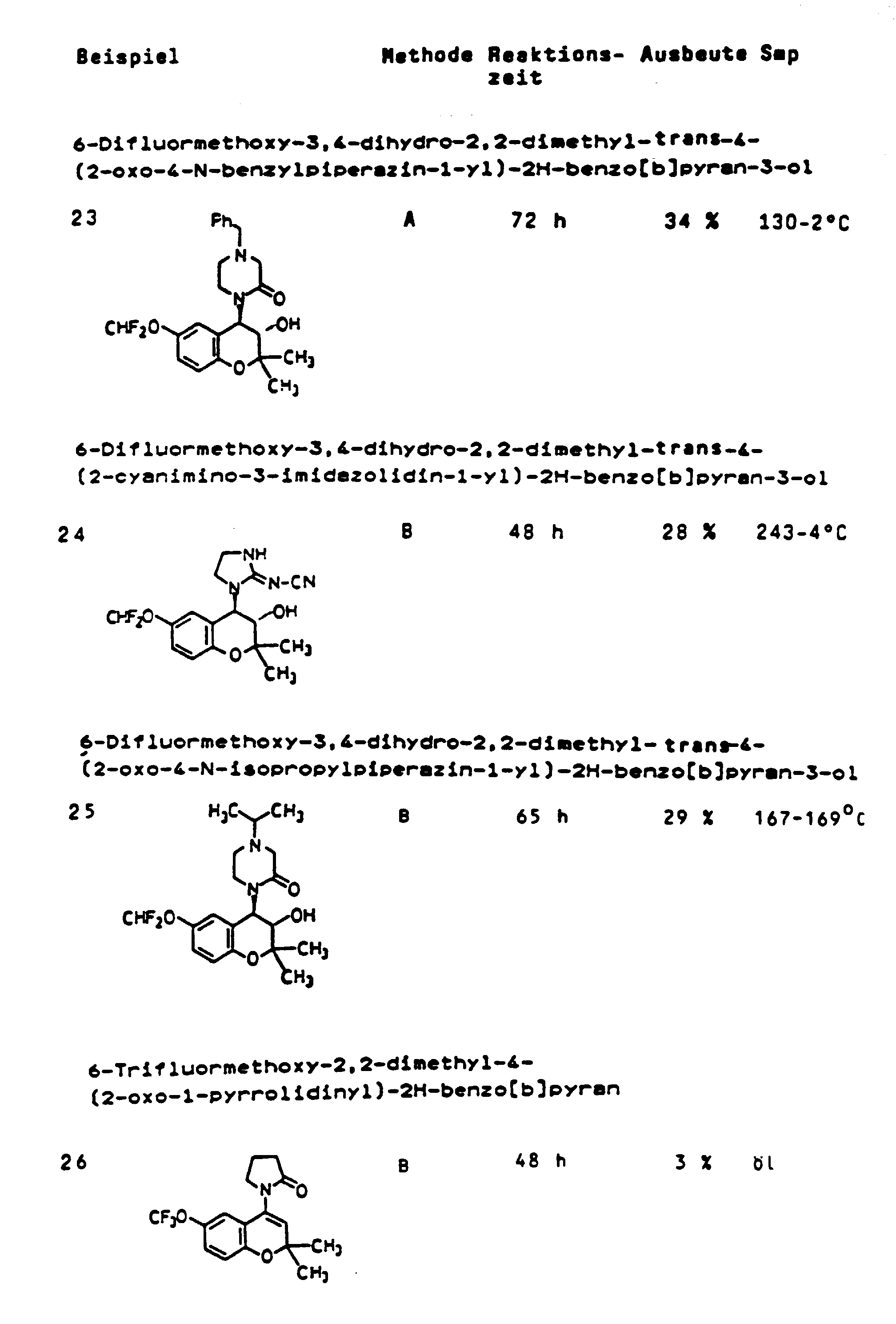

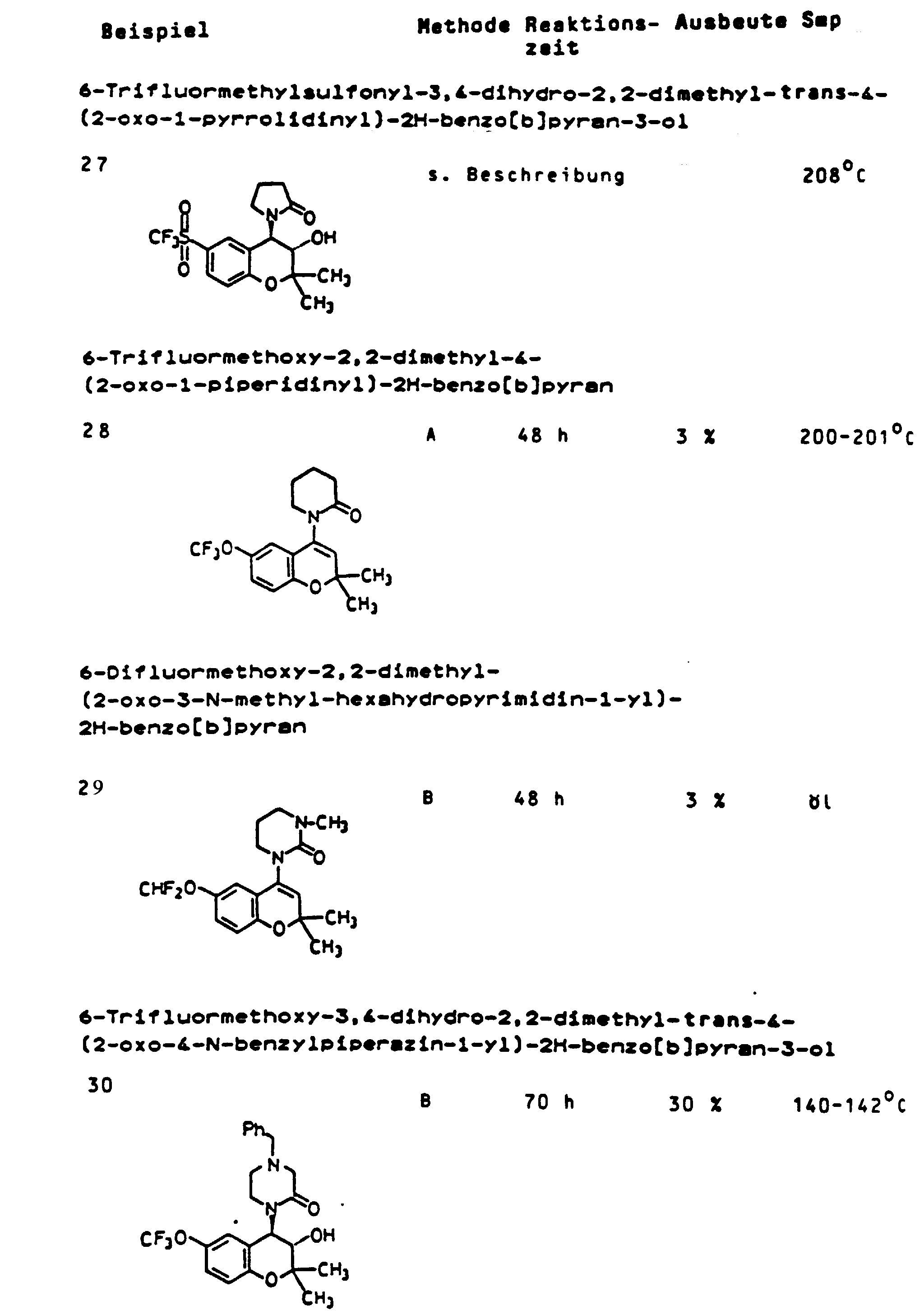

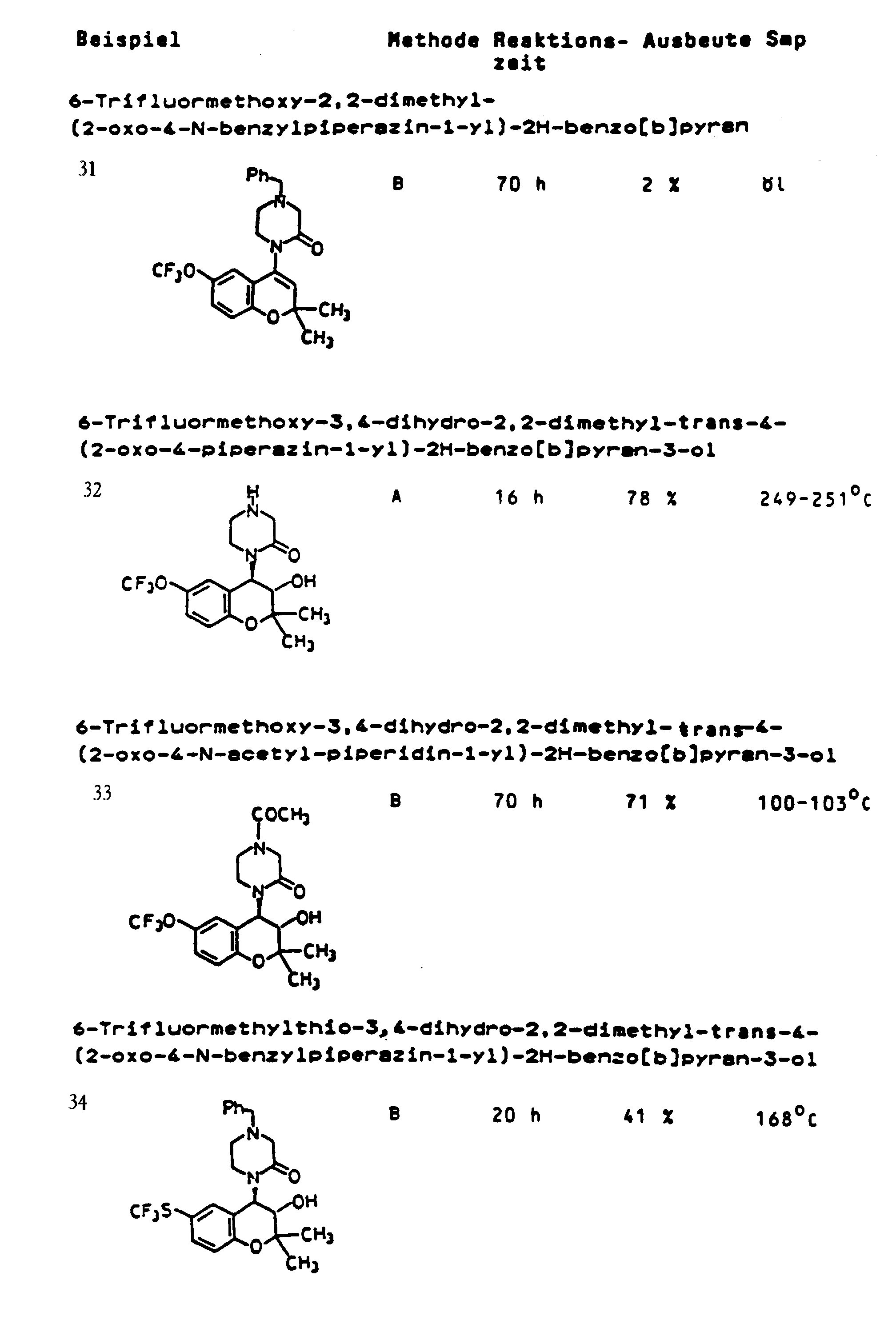

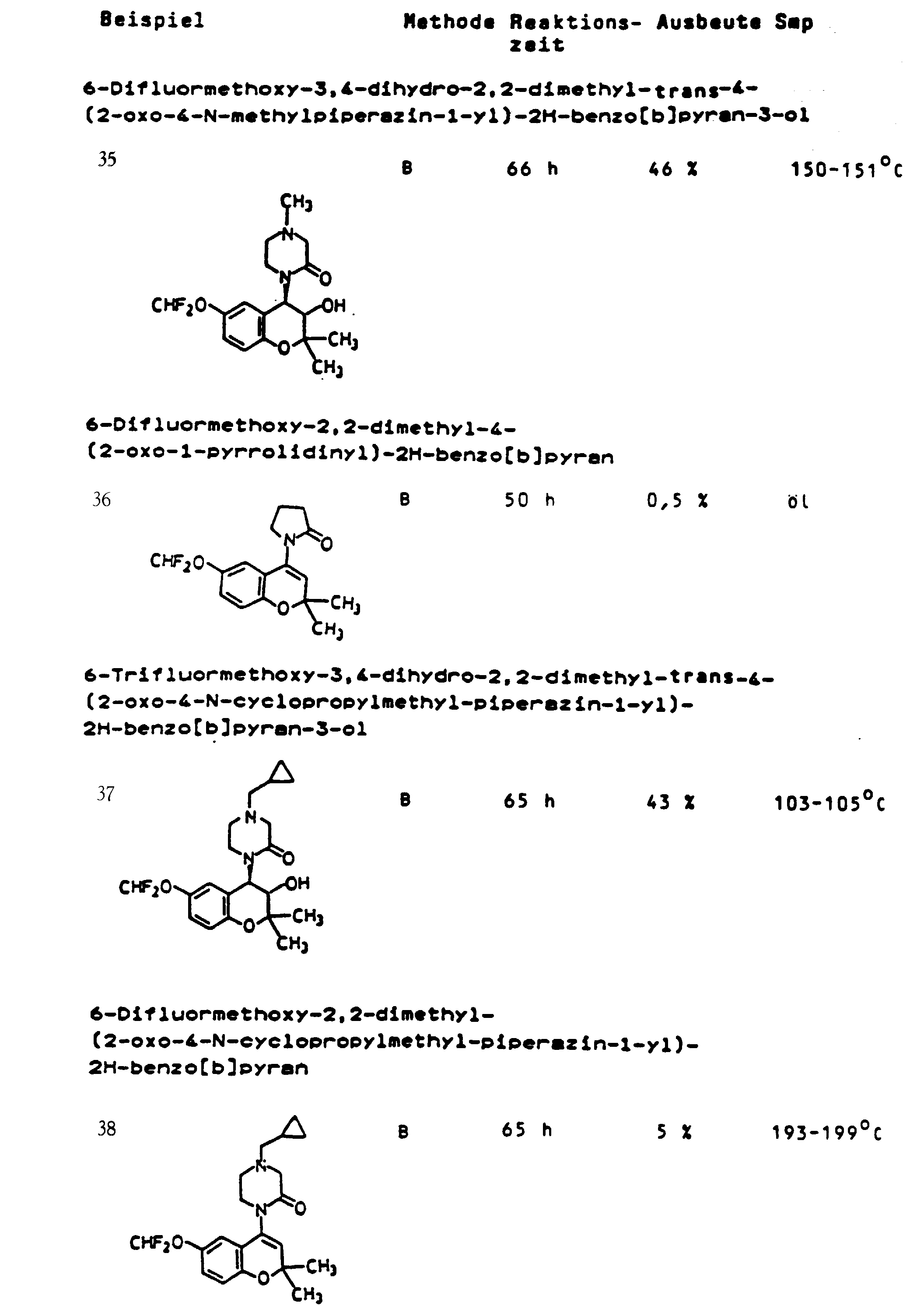

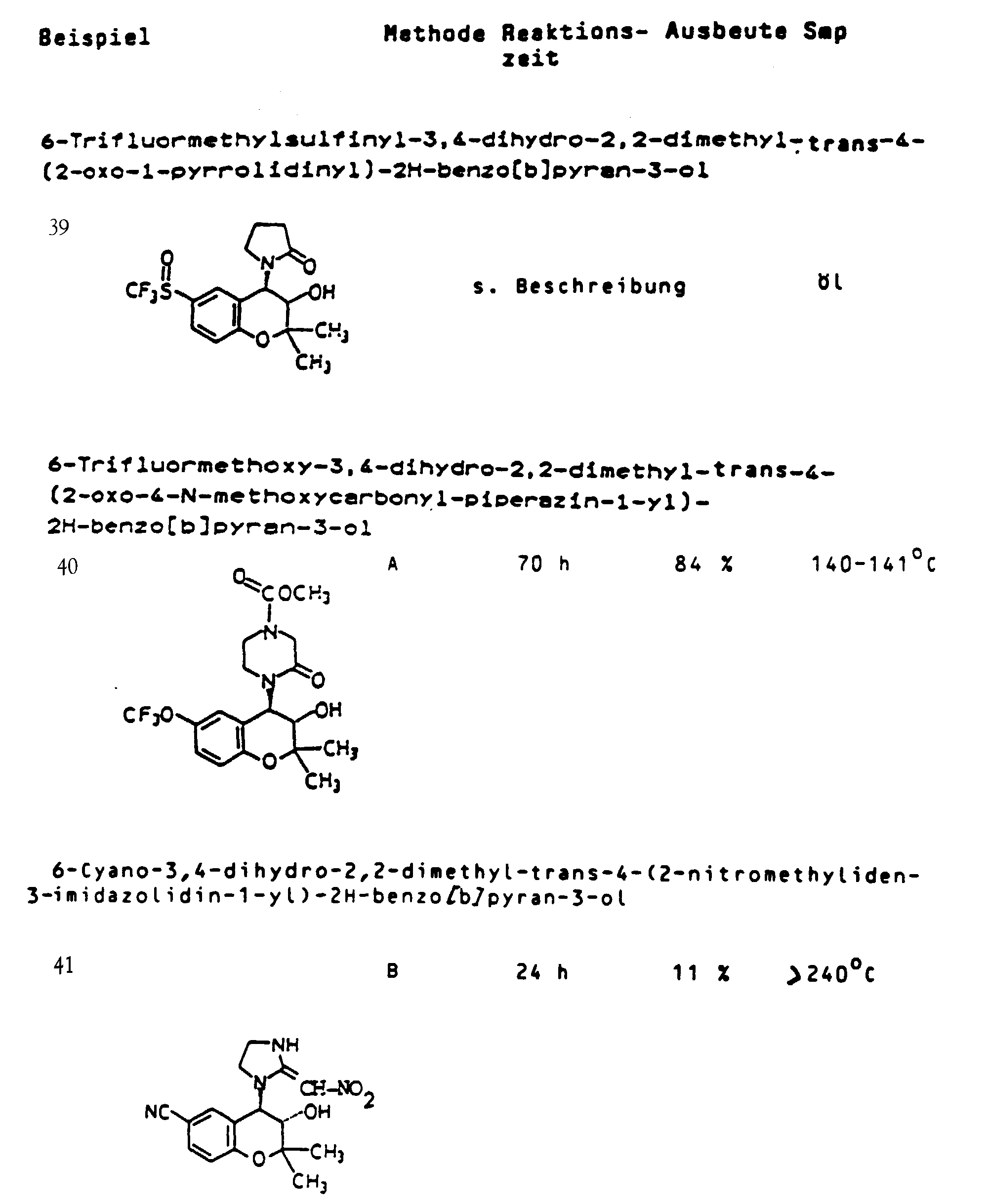

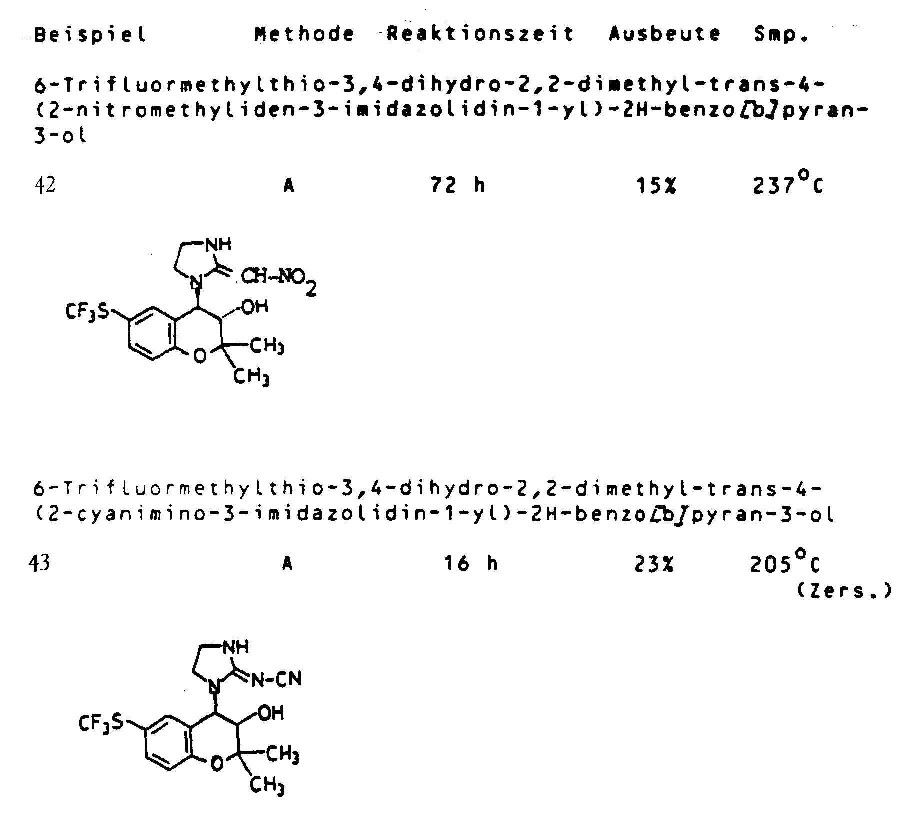

### Beispiel 44

### 4-Trifluormethylsulfonyl-phenol

### Methode A':

1 g (5,2 mMol) 4-Trifluormethylthiophenol werden in 20 ml Methanol gelöst und bei 0°C wird unter Rühren eine Suspension von 9,6 g Oxone^{R} in 20 ml Wasser zugegeben. Nach 5 Tagen Rühren bei Raumtemperatur wird mit 50ml Wasser verdünnt und mit Chloroform extrahiert (3 x 50 ml). Nach Trocknen und Eindampfen verbleiben 1.1 g farblose Kristalle 4-Trifluormethylsulfonyl-phenol (94% d. Th.) M⁺=226 (8)

### Methode B':

1 g (5,2 mMol) 4-Trifluormethylthio-phenol wird zusammen mit 4 ml Eisessig und 4 ml 30%igem Wasserstoffperoxid 20 Stunden bei 50°C gerührt, anschließend werden nochmals 2 ml 30%iges Wasserstoffperoxid zugesetzt und nach weiteren 2 Stunden bei 50°C wird wie bei A' aufgearbeitet. Nach Chromatographie an Kieselgel erhält man 230 mg farblose Kristalle 4-Trifluormethylsulfonyl-phenol (20% d. Th.) Smp. 123°C (Lit.: 119-120°C)

### Beispiel 45

### 4-Difluormethylsulfonyl-phenol

Die Verbindung ist analog dem unter 44, A' oder 44, B' beschriebenen Verfahren zugänglich.

### Beispiel 46

### 4-(2,2,2-Trifluorethylsulfonyl)-phenol

Die Verbindung ist analog Verfahren 44, A' oder 44, B' erhältlich.

### Beispiel 47

### 6-Trifluormethylthio-2,2-dimethyl-2H-benzo(b)pyran

### a) 3-(4-(Trifluormethylthio)-phenoxy)-3-methyl-1-butin

In einer Lösung von 19,4 g (0,1 Mol) 4-(Trifluormethylthio)-phenol in 250 ml trockenem Butanon werden 13,8 g (0,1 Mol) getrocknetes Kaliumcarbonat und 1,6 g (0,01 Mol) Kaliumiodid suspendiert und 15,4 g (0,15 Mol) 3-Chlor-3-methyl-1-butin zugetropft. Dann wird unter Rühren 20 Stunden unter Rückfluß erhitzt, nochmals werden 15,4 g 3-Chlor-3-methyl-1-butin und 13,8 g Kaliumcarbonat zugesetzt und weiterhin wird 40 Stunden unter Rückfluß erhitzt. Anorganische Bestandteile werden abfiltriert, die Lösung eingeengt, der Rückstand in 200 ml Methylenchlorid aufgenommen und mit 1N NaOH-Lösung extrahiert. Die organische Phase wird mit Wasser gewaschen, getrocknet und eingeengt, der Rückstand wird über Kieselgel filtriert.
Ausbeute: 22 g (85% d. Th.)

### b) 6-Trifluormethylthio-2,2-dimethyl-2H-benzo(b)pyran

22 g (0,085 Mol) der vorstehend (4'a) beschriebenen Verbindung werden in 50 ml 1,2-Dichlorbenzol unter Argon 2 Stunden auf 180°C erhitzt und anschließend im Vakuum fraktioniert.
Ausbeute: 13 g farbloses öl (59,1% d. Th.) Kp 55°C/2 Pa

### Beispiel 48

### 6-Trifluormethylsulfonyl-2,2-dimethyl-2H-benzo(b)pyran

1 g (4,4 mMol) 4-Trifluormethylsulfonyl-phenol werden zusammen mit 0,66 g Kaliumcarbonat, 80 mg Kaliumiodid und 2 g 3-Chlor-3-methyl-1-butin in 13 ml trockenem Butanon 20 Stunden unter Argon bei 80 - 90°C gerührt. Dann werden nochmals 0,33 g Kaliumcarbonat, 40 mg Kaliumiodid und 1 g 3-Chlor-3-methyl-1-butin zugesetzt und es wird weitere 20 Stunden bei 80 - 90°C gerührt. Man läßt abkühlen, filtriert und dampft das Filtrat ein. Der Rückstand wird in 20 ml Methylenchlorid aufgenommen, mit Wassergewaschen (2 x 20 ml), getrocknet und eingedampft. Das verbleibende Öl (1,3 g) wird in o-Dichlorbenzol (3 ml) 3 Stunden unter Argon auf 180°C erhitzt. Nach Abdestillieren des Lösungsmittels wird an Kieselgel chromatographiert. Man erhält 0,5 g farbloses Öl (50% d. Th.). M⁺=292 (5).

### Beispiel 49

### 6-Trifluormethoxy-2,2-dimethyl-2H-benzo(b)-pyran

In einer Lösung von 90 g (0,5 Mol) 4-Trifluormethoxyphenol in 900 ml trockenem Aceton werden 69,2 g (0,5 Mol) getrocknetes Kaliumcarbonat und 8,3 g (0,05 Mol) Kaliumiodid suspendiert und es werden 70 g (0,68 Mol) 3-Chlor-3-methyl-1-butin zugetropft.

Nach 36 Stunden Rühren bei Rückflußtemperatur werden nochmals 35 g (0,34 Mol) 3-Chlor-3-methyl-1-butin zugesetzt und es wird weitere 36 Stunden bei Rückflußtemperatur gerührt. Die abgekühlte Suspension wird filtriert, mit Aceton nachgewaschen, das Filtrat wird eingeengt, der Rückstand in Methylenchlorid aufgenommen und mit 1N-NaOH-Lösung extrahiert. Die Methylenchloridphase wird neutral gewaschen, getrocknet und eingedampft.
Ausbeute: 67 g (54,9% d. Th.)
67 g der vorstehenden Verbindung werden in 380 ml 1,2-Dichlorbenzol 4 Stunden auf 180°C erhitzt.
Fraktionierung im Vakuum ergibt das gewünschte Produkt.
Ausbeute: 45 g (67,2% d.Th.) Kp: 75 - 80°C/1,3 Pa

### Beispiel 50

### 6-Trifluormethylsulfinyl-2,2-dimethyl-2H-benzo(b)pyran

### a) 2 g (7,7 mMol)

6-Trifluormethylthio-2,2-dimethyl-2H-benzo(b)pyran (beispiel 4') werden in 40 ml Methanol gelöst und bei 0°C wird eine Suspension von 14,2 g (23,1 mMol) Oxone^{R} in 40 ml Wasser zugesetzt. Nach 2 Tagen Rühren bei Raumtemperatur wird mit 50 ml Wasser verdünnt, mit Chloroform extrahiert (3 x 100ml) und der nach Trocknen und Eindampfen verbleibende Rückstand an Kieselgel chromatographiert. Man erhält 1 g farbloses Öl (47% d. Th.) M⁺=276(9)

### b) 6-Trifluormethylsulfonyl-2,2-dimethyl-2H-benzo(b)pyran

2 g (7,7 mMol) 6-Trifluormethylthio-2,2-dimethyl-2H-benzo(b)pyran werden zusammen mit 6 ml Eisessig und 6 ml 30% Wasserstoffperoxid 6 Tage bei Raumtemperatur gerührt, anschließend wird auf 100 ml mit Wasser verdünnt und mit Chloroform extrahiert (3 x 70 ml). Nach Trocknen und Eindampfen verbleiben 2 g farbloses Öl. Mittels HPLC (Laufmittel 98:2 Chloroform:Methanol) erhält man 0,3 g farbloses öl 6-Trifluormethylsulfinyl-2,2-dimethyl-2H-benzo(b)pyran (14% d. Th.) und 0,2 g farbloses öl 8-Trifluormethylsulfonyl-2,2-dimethyl-2H-benzo(b)pyran (9% d. Th.).

Analog wurden:

### Beispiel 51

### 6-Difluormethylsulfinyl-2,2-dimethyl-2H-benzo(b)pyran

und

### Beispiel 52

### 6-(2,2,2-Trifluorethylsulfinyl)-2,2-dimethyl-2H-benzo(b)pyran

erhalten.

Weitere 6-substituierte 2H-Benzo(b)pyrane der allgemeinen Formel V werden analog hergestellt.

### Beispiel 53

### 6-Trifluormethylthio-3,4-dihydro-3-bromo-2,2-dimethyl-2H-benzo(b)pyran4-ol

Einer Lösung von 7 g (0,027 Mol) des vorstehend beschriebenen Benzopyrans (Beispiel 4'b) in 60 ml DMSO und 1 ml Wasser werden bei 20 - 25°C 7,7 g (0,043 Mol) N-Bromsuccinimid zugesetzt. Nach einer Stunde Rühren wurde auf Eis gegeben und mit Ethylacetat extrahiert (3 x 100 ml). Die vereinigten organischen Phasen wurden mit Wasser gewaschen (3 x 50 ml), getrocknet und eingeengt, wobei das Produkt auskristaltisiert.
Ausbeute: 8 g (83% d. Th.) leicht bräunliche Kristalle.

### Beispiel 54

### 6-Trifluormethylthio-3,4-dihydro-3,4-oxiranyl-2,2-dimethyl-2H-benzo(b)pyran

Zu einer Lösung von 32 g (0,09 Mol) des vorstehend beschriebenen Bromhydrins in 500 ml trockenem Tetrahydrofuran wurden unter Stickstoff entöltes Natriumhydrid (3,5 g, 80%, in Paraffinöl) portionsweise zugesetzt. Nach einer Stunde Rühren bei Raumtemperatur wird nochmals 1 g Natriumhydrid zugegeben. Nach einer weiteren Stunde wird über Kieselgel abfiltriert und die Lösung eingedampft.
Ausbeute: 26 g (100% d. Th.)

### Beispiel 55

### 6-Trifluormethylsulfinyl-3,4-dihydro-2,2-dimethyl-3-bromo-2H-benzo(b)pyran-4-ol

Zu einer Lösung von 5 g (18 mMol) 6-Trifluormethylsulfinyl-2,2-dimethyl-2H-benzo(b)pyran in 45 ml DMSO und 0,7 ml Wasser werden bei 20 - 25°C 51 g (28,6 mMol) N-Bromsuccinimid zugesetzt. Nach einer Stunde Rühren wurde auf Eis gegeben und mit Ethylacetat extrahiert (3 x 100ml). Die vereinigten organischen Phasen werden mit Wasser gewaschen (3 x 50 ml), getrocknet und eingeengt, wobei das Produkt auskristallisiert.
Ausbeute 6,1 g (90% d. Th.) hellbraune Kristalle.

### Beispiel 56

### 6-Trifluormethylsulfinyl-3,4-dihydro-2,2-dimethyl-3,4-oxiranyl-2H-benzo(b)pyran

Zu einer Lösung von 5,6 g (15 mMol) des vorstehend beschriebenen Bromhydrins in 80 ml trockenem Tetrahydrofuran wird unter Stickstoff entöltes Natriumhydrid (0,58 g, 80%, in Paraffinöl) portionsweise zugesetzt. Nach einer Stunde Rühren bei Raumtemperatur wird nochmals 0,2 g Natriumhydrid zugegeben. Nach einer weiteren Stunde wird über Kieselgel abfiltriert und die Lösung eingedampft.
Ausbeute: 4,3 g (100% d. Th.)

### Beispiel 57

### Herstellungsverfahren für Verbindungen der Formel H-E

### Methode A''

Zu 0,2 Mol Aminoverbindung werden 29,2g (0,2 Mol) Cyanimidodithiokohlensäuredimethylester in 1000 ml Toluol zugetropft und 6 Stunden unter Rückfluß gekocht. Nach Abkühlen wird der Niederschlag abgesaugt, am Aceton umkristallisiert und getrocknet.

### Methode B''

Eine Lösung von 0,2 Mol Aminoverbindung in 100 ml Methanol wird unter Rühren zu einer Lösung von 47,6 g (0,2 Mol) Cyanimidokohlensäurediphenylester in 400 ml Methanol getropft und 10 Minuten zum Sieden erhitzt. Der nach Einengen verbleibende Rückstand wird mit Ether verrieben, abgesaugt und getrocknet.

Analog wurden die in der Tabelle angegebenen Verbindungen erhalten:

| Ausgangsverbindung: N₂N-(CH₂)ₚ-Y H-E | | | |
|---|---|---|---|
| p | Y | Ausbeute % (H-E) | Methode |
| 2 | N-CH₃ | 44 | A |
| 2 | N-C₆H₅ | 28 * | A |
| 3 | N-H | 67 | A |
| 2 | NH | 52 | A |
| 2 | O | 60 | B |
| 2 | S | 67 | B |

| | | | |
|---|---|---|---|
| * Cyclisierung durch Reaktion mit Natriumhydrid | | | |

### Beispiel 58

### Herstellung von Tabletten und Kapseln

Tabletten und Kapseln, welche die nachstehend angegebenen Bestandteile enthalten, werden nach bekannten Arbeitsweisen hergestellt. Diese sind für die Behandlung der vorstehend genannten Krankheiten, insbesondere der Hypertonie in Dosierungsmengen von jeweils einer Tablette oder Kapsel einmal täglich geeignet.

| Bestandteile | Gewicht (mg) | |
|---|---|---|
| | Tablette | Kapsel |
| 6-Trifluormethylthio-3,4-dihydro-2,2-dimethyl-trans-4-(2-oxo-1-piperidinyl)-2H-benzo(b)pyran-3-ol | 0,2 | 0,1 |
| Tragacanth | 10 | |
| Lactose | 247,5 | 300 |
| Maisstärke | 25 | |
| Talk | 15 | |
| Magnesiumstearat | 2,5 | |

### Beispiel 59

### Herstellung von Ampullen

Ampullen, die die im folgenden genannten Bestandteile enthalten, lassen sich in bekannter Weise herstellen. Der Wirkstoff wird in Wasser und 1,2-Propandiol gelöst und unter Stickstoff in Glasampullen abgefüllt.

| | |
|---|---|
| 6-Trifluormethylsulfonyl-3,4-dihydro-2,2-dimethyl-trans-4-(2-oxo-1-pyrrolidinyl)-2H-benzo(b)pyran-3-ol | 0,02 mg |
| 1,2-Propandiol | 0,8 ml |
| dest. Wasser ad | 2,0 ml |

## Patentansprüche (Patentansprüche für folgende(n) Vertragsstaat(en): AT, BE, CH DE, FR, GB, IT, LI, NL, SE)

1. Benzopyran-Derivate der allgemeinen Formel I, 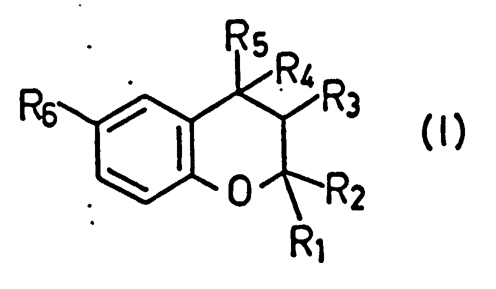 in der
R₁ und R₂, die gleich oder verschieden sein können, Wasserstoff, C₁₋₆-Alkyl, C₃₋₆-verzweigt-Alkyl, C₃₋₇-Cycloalkyl oder
zusammen mit dem von ihnen eingeschlossenen Kohlenstoffatom C₃₋₇-Spiroalkyl bedeuten,
R₃ Hydroxy, C₁₋₈-Alkoxy, Formyloxy, C₁₋₈-Alkylcarbonyloxy, C₁₋₈-Alkoxycarbonyloxy, C₁₋₈-Mono-alkylaminocarbonyloxy oder C₁₋₈-Dialkylamino-carbonyloxy bedeutet, wobei die C₁₋₈-Alkyl- oder -Alkoxygruppen sowohl linear als auch verzweigt sein können, und
R₄ für Wasserstoff steht oder
R₃ und R₄ zusammen eine Bindung bilden,
R₅ einen Heterocyclus der Formel A 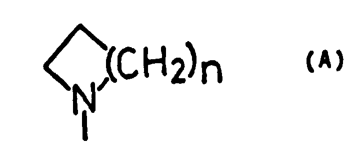 bedeutet, wobei n für 1, 2, 3 oder 4 steht,
oder einen Heterocyclus der Formel B 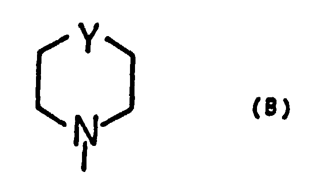 bedeutet, wobei Y für Sauerstoff, Schwefel,
unsubstituiertes Amino -NH-, substituiertes Amino -NR₇- steht und R₇ geradkettiges C₁₋₉-Alkyl, verzweigtes C₃₋₇-Alkyl, C₃₋₇-Cycloalkyl, durch C₃₋₇-Cycloalkyl substituiertes geradkettiges oder verzweigtes C₁₋₉-Alkyl, C₁₋₈-Alkylcarbonyl, C₁₋₈-Alkoxycarbonyl, Benzyl, Triphenylmethyl, Phenyl, Benzyloxycarbonyl, Phenylcarbonyl oder Benzylcarbonyl bedeutet,
oder R₅ einen Heterocyclus der Formel C 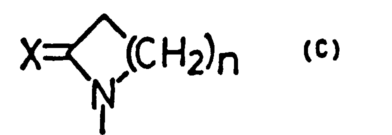 bedeutet, wobei X für Sauerstoff oder Schwefel steht und n für 1, 2, 3 oder 4 steht,
oder einen Heterocyclus der Formel D 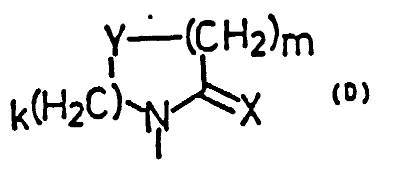 bedeutet, wobei m 0, 1 oder 2 und k 1, 2 oder 3 bedeutet, jedoch so, daß (m + k) 1, 2 oder 3 ist und weiterhin X und Y die für die Formeln B und C angegebene Bedeutung haben,
oder einen Heterocyclus der Formel E 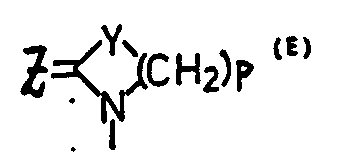 bedeutet, wobei Z für Cyanimino N-CN, cis oder trans Nitromethyliden (c/t) CH-NO₂ oder Nitroimino N-NO₂ steht, Y die oben angegebene Bedeutung hat und p 2 oder 3 bedeutet, und
R₆ in Abhängigkeit von R₅ für die zwei Klassen von Substituenten R₆' und R₆'' steht, wobei, falls R₅ einen Heterocyclus A, B, C oder D bedeutet, R₆ für die Substituentenklasse R₆' steht und
R₆' Difluormethoxy, Trifluormethoxy, Trifluorethoxy, Tetrafluorethoxy, Difluormethylthio, Difluormethylsulfinyl, Difluormethylsulfonyl, Trifluormethylthio, Trifluormethylsulfinyl, Trifluormethylsulfonyl, Trifluorethylthio, Trifluorethylsulfinyl oder Trifluorethylsulfonyl bedeutet,
oder, falls R₅ den Heterocyclus E bedeutet, R₆ für die vorstehende Substituentenklasse R₆' und für die Substituentenklasse R₆'' steht und
R₆'' Cyano, Nitro, C₁₋₆-Alkyl, C₃₋₈-Cycloalkyl, Formyl und C₁₋₆-Alkylcarbonyl bedeutet, wobei sich der Heterocyclus R₅ in trans-Stellung zu dem Rest R₃ befindet, wenn R₃ und R₄ nicht zusammen eine Bindung bedeuten, sondern R₄ für Wasserstoff steht, sowie deren Salze und Säureadditionssalze, Tautomere und optische Isomere,
ausgenommen Verbindungen der Formel (I), bei denen zugleich R₁ und R₂ beide Methyl, R₃ Hydroxyl, R₅ einen heterocyclichen Rest der Formel (C), X Sauerstoff, n 2, 3 oder 4 und R₆ Trifluormethoxy oder Trifluorethoxy bedeuten,
und ausgenommen Verbindungen der Formel (I), bei denen zugleich R₁ und R₂ C₁₋₆₋Alkyl oder C₃₋₆₋verzweigtes Alkyl, R₃ Hydroxy, R₄ Wasserstoff oder R₃ und R₄ zusammen eine Bindung, R₅ einen Heterocyclus der Formel (E), worin Y für Schwefel, Amino oder substituiertes Amino mit R₇ in der Bedeutung C₁₋₆-Alkyl oder verzweigtes C₃₋₆-Alkyl und Z für Cyanimino stehen, und R₆ Cyano bedeuten.

2. Verbindungen der Formel 1a: 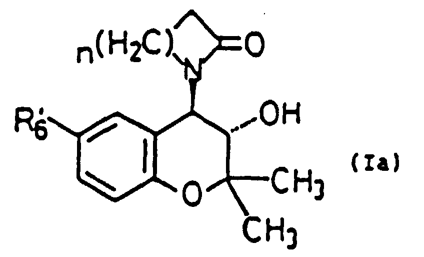 worin n die Zahl 2 oder 3 und R₆' Difluormethoxy, Trifluormethylthio, Difluormethylthio, Trifluormethylsulfonyl, Difluormethylsulfonyl und Tetrafluorethoxy bedeutet.

3. Verbindungen der Formel 1a': 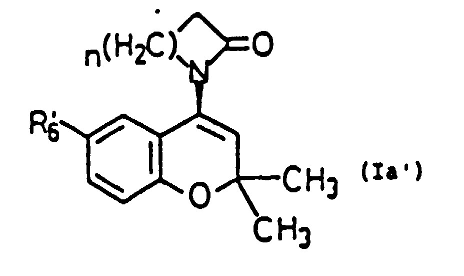 worin n die Zahl 2 oder 3 und R₆' Difluormethoxy, Trifluormethoxy, Trifluormethylthio, Difluormethylthio, Trifluormethylsulfonyl, Difluormethylsulfonyl, Trifluorethoxy und Tetrafluorethoxy bedeutet.

4. Verbindung gemäß Anspruch 1, ausgewählt aus der Gruppe 6-Trifluormethylthio-3,4-dihydro-2,2-dimethyl-trans-4-(2-oxo-1-pyrrolidinyl)-2H-benzo(b)pyran-3-ol,
6-Trifluormethylsulfonyl-3,4-dihydro-2,2-dimethyl-trans-4-(2-oxo-1-pyrrolidinyl)-2H-benzo(b)pyran-3-ol,
6-Trifluormethylsulfonyl-3,4-dihydro-2,2-dimethyl-trans-4-(2-oxo-1-piperidinyl)-2H-benzo(b)pyran-3-ol,
6-Difluormethoxy-3,4-dihydro-2,2-dimethyl-trans-4-(2-oxo-1-piperidinyl)-2H-benzo(b)pyran-3-ol,
6-Trifluormethylthio-2,2-dimethyl-4-(2-oxo-1-pyrrolidinyl)-2H-benzo(b)pyran,
6-Trifluormethylsulfonyl-2,2-dimethyl-4-(2-oxo-1-pyrrolidinyl)-2H-benzo(b)-pyran,
6-Trifluormethylthio-2,2-dimethyl-4-(2-oxo-1-piperidinyl)-2H-benzo(b)pyran,
6-Trifluormethylsulfonyl-2,2-dimethyl-4-(2-oxo-1-piperidinyl)-2H-benzo(b)-pyran,
6-Trifluormethoxy-2,2-dimethyl-4-(2-oxo-1-piperidinyl)-2H-benzo(b)pyran,
6-Trifluormethoxy-2,2-dimethyl-4-(2-oxo-1-pyrrolidinyl)-2H-benzo[b]pyran.

5. 6-Trifluormethylthio-3,4-dihydro-2,2-dimethyl-trans-4-(2-oxo-1-piperidinyl)-2H-benzo(b)pyran-3-ol,

6. Verfahren zur Herstellung der Verbindungen gemäß Anspruch 1, dadurch gekennzeichnet, daß man
a) Oxirane der allgemeinen Formel IIa, in denen R₁, R₂ und R₆' die oben angegebene Bedeutung haben, mit Anionen C⁻ und D⁻ von Heterocyclen der allgemeinen Formel H-C und H-D, in denen X die Bedeutung Sauerstoff und Y, k, m, n die oben angegebene Bedeutung haben, mit der Einschränkung, daß für jene Heterocyclen H-D, für die m ungleich 0 gilt, Y nicht für unsubstituiertes Amino steht, zu Verbindungen der allgemeinen Formel I, in denen R₁, R₂ und R₆' die oben angegebene Bedeutung haben, R₃ für Hydroxy, R₄ für Wasserstoff und R₅ für die Heterocyclenreste C und D, in denen X für Sauerstoff steht und Y, k, m, n die oben angegebene Bedeutung haben, mit der Ausnahme, daß Y nicht für unsubstituiertes Amino steht, falls m ungleich 0 ist, umsetzt, wobei die Substituenten R₃ und R₅ der Verbindungen der Formel I **trans** zueinander angeordnet sind und daß man
b) jene erfindungsgemäße Verbindungen gemäß a) für die Y für substituiertes Amino und m für 1 oder 2 steht, durch Hydrolyse, Hydrogenolyse oder Dealkylierung in Verbindungen der Formel I gemäß a) überführt, in denen Y auch dann für unsubstituiertes Amino steht, falls m ungleich 0 gilt, wobei wiederum R₃ und R₅ **trans** zueinander angeordnet sind und daß man
c) Oxirane der allgemeinen Formel IIa gemäß a) mit Aminen der allgemeinen Formel H-A und H-B zu Verbindungen der Formel I, in denen R₁, R₂ und R₆' die oben angegebene Bedeutung haben und R₅ für A oder B, mit der für n und Y oben angegebenen Bedeutung steht, R₄ für Wasserstoff und R₃ für Hydroxy steht, umsetzt, wobei R₃ und R₅ trans zueinander angeordnet sind und daß man
d) Oxirane der allgemeinen Formel IIa und IIb, in denen R₁, R₂, R₆' und R₆" die oben angegebene Bedeutung haben, mit Anionen E⁻ von Heterocyclen der allgemeinen Formel H-E, in denen Y, Z und p die oben angegebene Bedeutung haben, zu Verbindungen der allgemeinen Formel I, in denen R₁, R₂, R₆' und R₆'' die oben angegebene Bedeutung haben, R₃ für Hydroxy, R₄ für Wasserstoff und R₅ für E mit der für Y, Z und p angegebenen Bedeutung steht, umsetzt, wobei R₃ und R₅ trans zueinander angeordnet sind und daß man
e) erfindungsgemäße Verbindungen der Formel I gemäß a) und b) mit Schwefelungsreagentien, z.B. bevorzugt Lawessons Reagens in einem inerten Solvens wie z.B. Toluol zu Verbindungen der Formel I, in denen R₁, R₂, R₃, R₄ und R₆' wie unter a) definiert sind, R₅ für die Reste C, D mit der für Y, k, m, n angegebenen Bedeutung steht, wobei jedoch X für Schwefel steht, umsetzt und daß man
f) erfindungsgemäße Verbindungen der Formel I gemäß a), d) und e), sowie jene erfindungsgemäßen Verbindungen gemäß c), bei denen Y nicht für unsubstituiertes Amino steht, durch Umsetzung mit Alkylhalogeniden, -mesylaten, -brosylaten, -tosylaten, Acylhalogeniden, -anhydriden, -imidazoliden, Alkylcarbonylhalogeniden oder -anhydriden, Mono- oder Dialkylaminocarbonylhalogeniden, Phosgen oder Alkylisocyanaten in Verbindungen der allgemeinen Formel I, in denen R₁, R₂, R₄, R₆', R₆'' und R₅ gemäß a), c), d) und e) definiert sind, mit der Einschränkung, daß Y im Rest B nicht für unsubstituiertes Amino steht, und R₃ für C₁₋₆-Alkoxy, Formyloxy, C₁₋₈-Alkylcarbonyloxy, C₁₋₈-Monoalkylamino, C₁₋₈-Dialkylamino steht, umwandelt und daß man
g) jene erfindungsgemäßen Verbindungen der Formel I gemäß f), bei denen Y für substituiertes, insbesondere benzylsubstituiertes Amino steht, durch geeignete Maßnahmen, insbesondere Hydrogenolyse in Verbindungen der Formel I gemäß f) überführt, bei denen Y für unsubstituiertes Amino steht und daß man
h) erfindungsgemäße Verbindungen der Formel I gemäß a), b), d) und e) in Anwesenheit eines dehydratisierenden Agens wie z.B. Natriumhydrid in einem inerten Solvens wie z.B. Tetrahydrofuran zu Verbindungen der allgemeinen Formel I umsetzt, in der R₁, R₂, R₅, R₆' und R₆'' die oben angegebene Bedeutung haben und R₃ zusammen mit R₄ eine Bindung bildet und daß man
i) Oxirane der allgemeinen Formeln IIa bzw. IIb, in denen R₁, R₂ und R₆' bzw. R₆'' die oben angegebene Bedeutung haben unter Anwesenheit von mindestens zwei Äquivalenten einer Base zunächst zu erfindungsgemäßen Verbindungen gemäß a) und d) umsetzt, diese jedoch nicht isoliert, sondern durch Verlängerung der Reaktionszeit und insbesondere durch Erhöhen der Reaktionstemperatur, auf vorzugsweise 40°C, in situ in erfindungsgemäße Verbindungen gemäß h) überführt und diese nach an sich üblichen Verfahren isoliert und daß man
j) jene erfindungsgemäßen Verbindungen gemäß a), b), c), d), f), g) und h), in denen R₆' die Bedeutung Difluormethylthio, Trifluormethylthio und 2,2,2-Trifluorethylthio und gleichzeitig X und Y eine andere Bedeutung als Schwefel hat mit geeigneten Oxidationsmitteln, bevorzugt Kaliumperoxomonosulfat bzw. Wasserstoffperoxid/Eisessig in jene erfindungsgemäße Verbindungen gemäß a), b), c), d), g) und h) überführt, in denen R₆' die Bedeutung Difluormethylsulfinyl, Trifluormethylsulfinyl, 2,2,2-Trifluorethylsulfinyl und insbesondere Trifluormethylsulfonyl, 2,2,2-Trifluorethylsulfonyl, Difluormethylsulfonyl hat bzw. eventuell anfallende Gemische von Sulfinyl- und Sulfonyl-Verbindung durch an sich übliche Verfahren in die reinen Komponenten auftrennt.

7. Pharmazeutisches Präparat, dadurch gekennzeichnet, daß es eine oder mehrere der Verbindungen gemäß Anspruch 1 oder deren physiologisch verträgliche Salze und gegebenenfalls übliche Trägerstoffe und/oder Verdünnungsmittel enthält.

8. Verbindungen der Formeln IIa, IIIa und Va 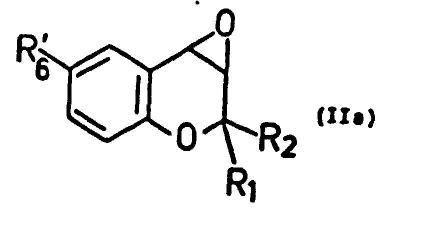 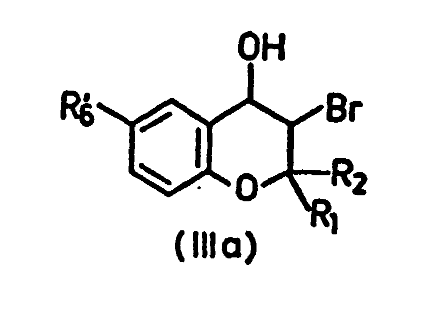 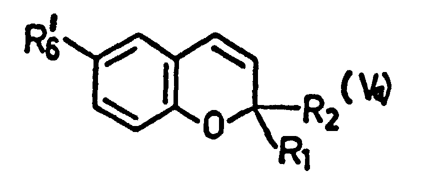 mit der vorstehend angegebenen Bedeutung von R₁, R₂, R₆',
ausgenommen Verbindungen der Formel (IIa),
bei denen zugleich R₁ und R₂ beide Methyl und R₆' Trifluomethoxy oder Trifluorethoxy bedeuten.

9. Verbindungen gemäß Anspruch 1, dadurch gekennzeichnet, daß R₃ und R₄ zusammen eine Bindung bilden, so daß zwischen C₃ und C₄-Position des Benzopyrangerüstes eine Doppelbindung besteht.

## Patentansprüche (Patentansprüche für folgende(n) Vertragsstaat(en): ES)

1. Verfahren zur Herstellung von Benzopyran-Derivaten der allgemeinen Formel I, 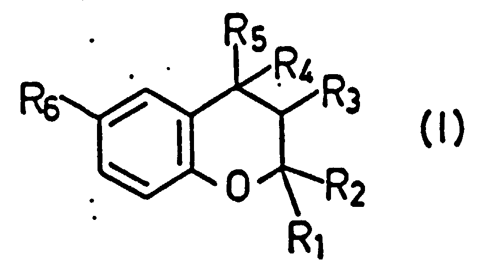 in der
R₁ und R₂, die gleich oder verschieden sein können, Wasserstoff, C₁₋₆-Alkyl, C₃₋₆-verzweigt-Alkyl, C₃₋₇-Cycloalkyl oder
zusammen mit dem von ihnen eingeschlossenen Kohlenstoffatom C₃₋₇-Spiroalkyl bedeuten,
R₃ Hydroxy, C₁₋₈-Alkoxy, Formyloxy, C₁₋₈-Alkylcarbonyloxy, C₁₋₈-Alkoxycarbonyloxy, C₁₋₈-Mono-alkylaminocarbonyloxy oder C₁₋₈-Dialkylamino-carbonyloxy bedeutet, wobei die C₁₋₈-Alkyl- oder -Alkoxygruppen sowohl linear als auch verzweigt sein können, und
R₄ für Wasserstoff steht oder
R₃ und R₄ zusammen eine Bindung bilden,
R₅ einen Heterocyclus der Formel A 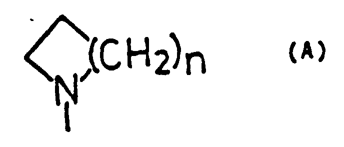 bedeutet, wobei n für 1, 2, 3 oder 4 steht,
oder einen Heterocyclus der Formel B 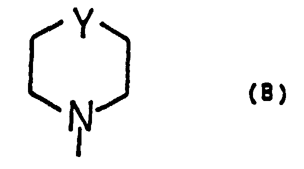 bedeutet, wobei Y für Sauerstoff, Schwefel, unsubstituiertes Amino -NH-, substituiertes Amino -NR₇- steht und R₇ geradkettiges C₁₋₉-Alkyl, verzweigtes C₃₋₇-Alkyl, C₃₋₇-Cycloalkyl, durch C₃₋₇-Cycloalkyl substituiertes geradkettiges oder verzweigtes C₁₋₉-Alkyl, C₁₋₈-Alkylcarbonyl, C₁₋₈-Alkoxycarbonyl, Benzyl, Triphenylmethyl, Phenyl, Benzyloxycarbonyl, Phenylcarbonyl oder Benzylcarbonyl bedeutet, oder R₅ einen Heterocyclus der Formel C 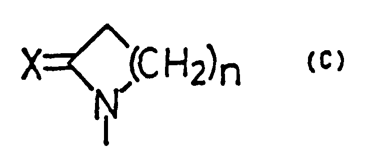 bedeutet, wobei X für Sauerstoff oder Schwefel steht und n für 1, 2, 3 oder 4 steht,
oder einen Heterocyclus der Formel D 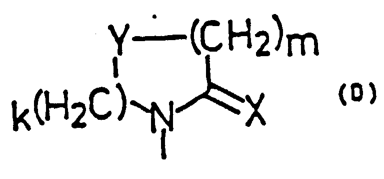 bedeutet, wobei m 0, 1 oder 2 und k 1, 2 oder 3 bedeutet, jedoch so, daß (m + k) 1, 2 oder 3 ist und weiterhin X und Y die für die Formeln B und C angegebene Bedeutung haben,
oder einen Heterocyclus der Formel E 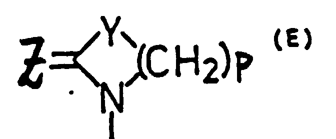 bedeutet, wobei Z für Cyanimino N-CN, cis oder trans Nitromethyliden (c/t) CH-NO₂ oder Nitroimino N-NO₂ steht, Y die oben angegebene Bedeutung hat und p 2 oder 3 bedeutet, und
R₆ in Abhängigkeit von R₅ für die zwei Klassen von Substituenten R₆' und R₆'' steht, wobei, falls R₅ einen Heterocyclus A, B, C oder D bedeutet, R₆ für die Substituentenklasse R₆' steht und
R₆' Difluormethoxy, Trifluormethoxy, Trifluorethoxy, Tetrafluorethoxy, Difluormethylthio, Difluormethylsulfinyl, Difluormethylsulfonyl, Trifluormethylthio, Trifluormethylsulfinyl, Trifluormethylsulfonyl, Trifluorethylthio, Trifluorethylsulfinyl oder Trifluorethylsulfonyl bedeutet,
oder, falls R₅ den Heterocyclus E bedeutet, R₆ für die vorstehende Substituentenklasse R₆' und für die Substituentenklasse R₆'' steht und
R₆'' Cyano, Nitro, C₁₋₆-Alkyl, C₃₋₈-Cycloalkyl, Formyl und C₁₋₆-Alkylcarbonyl bedeutet, wobei sich der Heterocyclus R₅ in trans-Stellung zu dem Rest R₃ befindet, wenn R₃ und R₄ nicht zusammen eine Bindung bedeuten, sondern R₄ für Wasserstoff steht, sowie deren Salzen und Säureadditionssalzen, Tautomeren und optischen Isomeren,
ausgenommen Verbindungen der Formel (I), bei denen zugleich R₁ und R₂ beide Methyl, R₃ hydroxyl, R₅ einen heterocyclichen Rest der Formel (C), X Sauerstoff, n 2, 3 oder 4 und R₆ Trifluormethoxy oder Trifluorethoxy bedeuten,
und ausgenommen Verbindungen der Formel (I), bei denen zugleich R₁ und R₂ C₁₋₆-Alkyl oder C₃₋₆-verzweigtes Alkyl, R₃ Hydroxy, R₄ Wasserstoff oder R₃ und R₄ zusammen eine Bindung, R₅ einen Heterocyclus der Formel (E), worin Y für Schwefel, Amino oder substituiertes Amino mit R₇ in der Bedeutung C₁₋₆-Alkyl oder verzweigtes C₃₋₆-Alkyl und Z für Cyanimino stehen, und R₆ Cyano bedeuten
**dadurch gekennzeichnet, daß man**
a) Oxirane der allgemeinen Formel IIa, in denen R₁, R₂ und R₆' die oben angegebene Bedeutung haben, mit Anionen C⁻ und D⁻ von Heterocyclen der allgemeinen Formel H-C und H-D, in denen X die Bedeutung Sauerstoff und Y, k, m, n die oben angegebene Bedeutung haben, mit der Einschränkung, daß für jene Heterocyclen H-D, für die m ungleich 0 gilt, Y nicht für unsubstituiertes Amino steht, zu Verbindungen der allgemeinen Formel I, in denen R₁, R₂ und R₆' die oben angegebene Bedeutung haben, R₃ für Hydroxy, R₄ für Wasserstoff und R₅ für die Heterocyclenreste C und D, in denen X für Sauerstoff steht und Y, k, m, n die oben angegebene Bedeutung haben, mit der Ausnahme, daß Y nicht für unsubstituiertes Amino steht, falls m ungleich 0 ist, umsetzt, wobei die Substituenten R₃ und R₅ der Verbindungen der Formel I trans zueinander angeordnet sind und daß man
b) jene erfindungsgemäße Verbindungen gemäß a) für die Y für substituiertes Amino und m für 1 oder 2 steht, durch Hydrolyse, Hydrogenolyse oder Dealkylierung in Verbindungen der Formel I gemäß a) überführt, in denen Y auch dann für unsubstituiertes Amino steht, falls m ungleich 0 gilt, wobei wiederum R₃ und R₅ trans zueinander angeordnet sind und daß man
c) Oxirane der allgemeinen Formel IIa gemäß a) mit Aminen der allgemeinen Formel H-A und H-B zu Verbindungen der Formel I, in denen R₁, R₂ und R₆' die oben angegebene Bedeutung haben und R₅ für A oder 8, mit der für n und Y oben angegebenen Bedeutung steht, R₄ für Wasserstoff und R₃ für Hydroxy steht, umsetzt, wobei R₃ und R₅ trans zueinander angeordnet sind und daß man
d) Oxirane der allgemeinen Formel IIa und IIb, in denen R₁, R₂, R₆' und R₆'' die oben angegebene Bedeutung haben, mit Anionen E⁻ von Heterocyclen der allgemeinen Formel H-E, in denen Y, Z und p die oben angegebene Bedeutung haben, zu Verbindungen der allgemeinen Formel I, in denen R₁, R₂, R₆' und R₆'' die oben angegebene Bedeutung haben, R₃ für Hydroxy, R₄ für Wasserstoff und R₅ für E mit der für Y, Z und p angegebenen Bedeutung steht, umsetzt, wobei R₃ und R₅ trans zueinander angeordnet sind und daß man
e) erfindungsgemäße Verbindungen der Formel I gemäß a) und b) mit Schwefelungsreagentien, z.B. bevorzugt Lawessons Reagens in einem inerten Solvens wie z.8. Toluol zu Verbindungen der Formel I, in denen R₁, R₂, R₃, R₄ und R₆' wie unter a) definiert sind, R₅ für die Reste C, D mit der für Y, k, m, n angegebenen Bedeutung steht, wobei jedoch X für Schwefel steht, umsetzt und daß man
f) erfindungsgemäße Verbindungen der Formel I gemäß a), d) und e), sowie jene erfindungsgemäßen Verbindungen gemäß c), bei denen Y nicht für unsubstituiertes Amino steht, durch Umsetzung mit Alkylhalogeniden, -mesylaten, -brosylaten, -tosylaten, Acylhalogeniden, -anhydriden, -imidazoliden, Alkylcarbonylhalogeniden oder -anhydriden, Mono- oder Dialkylaminocarbonylhalogeniden, Phosgen oder Alkylisocyanaten in Verbindungen der allgemeinen Formel I, in denen R₁, R₂, R₄, R₆', R₆'' und R₅ gemäß a), c), d) und e) definiert sind, mit der Einschränkung, daß Y im Rest 8 nicht für unsubstituiertes Amino steht, und R₃ für C₁₋₆-Alkoxy, Formyloxy, C₁₋₈-Alkylcarbonyloxy, C₁₋₈-Monoalkylamino, C₁₋₈-Dialkylamino steht, umwandelt und daß man
g) jene erfindungsgemäßen Verbindungen der Formel I gemäß f), bei denen Y für substituiertes, insbesondere benzylsubstituiertes Amino steht, durch geeignete Maßnahmen, insbesondere Hydrogenolyse in Verbindungen der Formel I gemäß f) überführt, bei denen Y für unsubstituiertes Amino steht und daß man
h) erfindungsgemäße Verbindungen der Formel I gemäß a), b), d) und e) in Anwesenheit eines dehydratisierenden Agens wie z.B. Natriumhydrid in einem inerten Solvens wie z.B. Tetrahydrofuran zu Verbindungen der allgemeinen Formel I umsetzt, in der R₁, R₂, R₅, R₆' und R₆'' die oben angegebene Bedeutung haben und R₃ zusammen mit R₄ eine Bindung bildet und daß man
i) Oxirane der allgemeinen Formeln IIa bzw. IIb, in denen R₁, R₂ und R₆' bzw. R₆'' die oben angegebene Bedeutung haben unter Anwesenheit von mindestens zwei Äquivalenten einer Base zunächst zu erfindungsgemäßen Verbindungen gemäß a) und d) umsetzt, diese jedoch nicht isoliert, sondern durch Verlängerung der Reaktionszeit und insbesondere durch Erhöhen der Reaktionstemperatur, auf vorzugsweise 40°C, in situ in erfindungsgemäße Verbindungen gemäß h) überführt und diese nach an sich üblichen Verfahren isoliert und daß man
j) jene erfindungsgemäßen Verbindungen gemäß a), b), c), d), f), g) und h), in denen R₆' die Bedeutung Difluormethylthio, Trifluormethylthio und 2,2,2-Trifluorethylthio und gleichzeitig X und Y eine andere Bedeutung als Schwefel hat mit geeigneten Oxidationsmitteln, bevorzugt Oxone^{R} bzw. Wasserstoffperoxid/Eisessig in jene erfindungsgemäße Verbindungen gemäß a), b), c), d), g) und h) überführt, in denen R₆' die Bedeutung Difluormethylsulfinyl, Trifluormethylsulfinyl, 2,2,2-Trifluorethylsulfinyl und insbesondere Trifluormethylsulfonyl, 2,2,2-Trifluorethylsulfonyl, Difluormethylsulfonyl hat bzw. eventuell anfallende Gemische von Sulfinyl- und Sulfonyl-Verbindung durch an sich übliche Verfahren in die reinen Komponenten auftrennt, und/oder
k) erwünschtenfalls erhaltene Verbindungen in Salze oder Säureadditionssalze überführt, und/oder
l) erwünschtenfalls erhaltene Verbindungen in ihre optischen Antipoden auftrennt.

2. Verfahren gemäß Anspruch 1, zur Herstellung von Verbindungen der Formel Ia 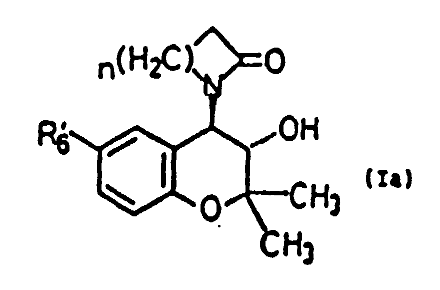 worin n die Zahl 2 oder 3 und R₆' Difluormethoxy, Trifluormethylthio, Difluormethylthio, Trifluormethylsulfonyl, Difluormethylsulfonyl und Tetrafluorethoxy bedeutet.

3. Verfahren gemäß Anspruch 1, zur Herstellung von Verbindungen der Formel Ia' 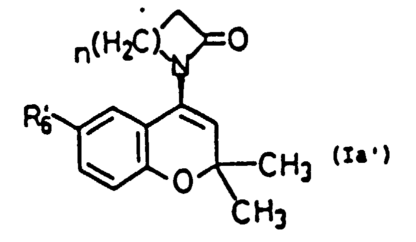 worin n die Zahl 2 oder 3 und R₆' Difluormethoxy, Trifluormethoxy, Trifluormethylthio, Difluormethylthio, Trifluormethylsulfonyl, Difluormethylsulfonyl, Trifluorethoxy und Tetrafluorethoxy bedeutet.

4. Verfahren gemäß Anspruch 1, zur Herstellung von 6-Triftuormethytthio-3,4-dihydro-2,2-dimethyl-trans-4-(2-oxo-1-piperidinyl)-2H-benzo(b)pyran-3-ol,

5. Verfahren gemäß Anspruch 1, worin R₃ und R₄ zusammen eine Bindung bilden, so daß zwischen C₃- und C₄-Position des Benzopyrangerüstes eine Doppelbindung besteht.

6. Verwendung einer Verbindung I nach Anspruch 1 zur Herstellung eines pharmazeutischen Präparates zur Prophylaxe und Behandlung von Bluthochdruck, Herzinsuffizienz, Angina pectoris, arterieller Verschlußkrankheit, cerebraler und peripherer Durchblutungsstörunqen, Asthma, cerebraler Insuffizienz, Migräne, Schwindel, Erkrankungen des Innenohrs beziehungsweise des Gehörapparates, erhöhtem Augeninnendruck, Glaukom, Sehschwäche, akuten und chronischen Herzkrankheiten.

7. Verfahren zur Herstellung eines pharmazeutischen Präparates, dadurch gekennzeichnet, daß man eine oder mehrere der Verbindungen der Formel I gemäß Anspruch 1 oder deren physiologisch verträgliche Salze und gegebenenfalls übliche Trägerstoffe und/oder Verdünnungsmittel mischt.

## Patentansprüche (Patentansprüche für folgende(n) Vertragsstaat(en): GR)

1. Verfahren zur Herstellung von Benzopyran-Derivaten der allgemeinen Formel I, 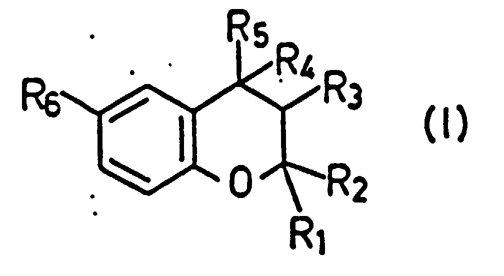 in der
R₁ und R₂, die gleich oder verschieden sein können, Wasserstoff, C₁₋₆-Alkyl, C₃₋₆-verzweigt-Alkyl, C₃₋₇-Cycloalkyl oder
zusammen mit dem von ihnen eingeschlossenen Kohlenstoffatom C₃₋₇-Spiroalkyl bedeuten,
R₃ Hydroxy, C₁₋₈-Alkoxy, Formyloxy, C₁₋₈-Alkylcarbonyloxy, C₁₋₈-Alkoxycarbonyloxy, C₁₋₈-Mono-alkylaminocarbonyloxy oder C₁₋₈-Dialkylamino-carbonyloxy bedeutet, wobei die C₁₋₈-Alkyl- oder -Alkoxygruppen sowohl linear als auch verzweigt sein können, und
R₄ für Wasserstoff steht oder
R₃ und R₄ zusammen eine Bindung bilden,
R₅ einen Heterocyclus der Formel A 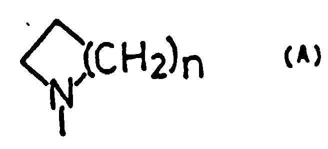 bedeutet, wobei n für 1, 2, 3 oder 4 steht,
oder einen Heterocyclus der Formel B 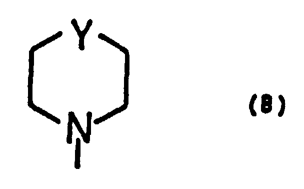 bedeutet, wobei Y für Sauerstoff, Schwefel, unsubstituiertes Amino -NH-, substituiertes Amino -NR₇- steht und R₇ geradkettiges C₁₋₉-Alkyl, verzweigtes C₃₋₇-Alkyl, C₃₋₇-Cycloalkyl, durch C₃₋₇-Cycloalkyl substituiertes geradkettiges oder verzweigtes C₁₋₉-Alkyl, C₁₋₈-Alkylcarbonyl, C₁₋₈-Alkoxycarbonyl, Benzyl, Triphenylmethyl, Phenyl, Benzyloxycarbonyl, Phenylcarbonyl oder Benzylcarbonyl bedeutet, oder R₅ einen Heterocyclus der Formel C 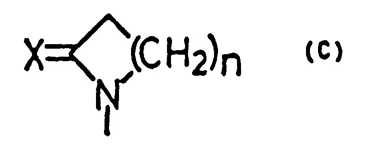 bedeutet, wobei X für Sauerstoff oder Schwefel steht und n für 1, 2, 3 oder 4 steht,
oder einen Heterocyclus der Formel D 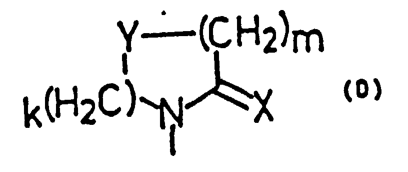 bedeutet, wobei m 0, 1 oder 2 und k 1, 2 oder 3 bedeutet, jedoch so, daß (m + k) 1, 2 oder 3 ist und weiterhin X und Y die für die Formeln B und C angegebene Bedeutung haben,
oder einen Heterocyclus der Formel E 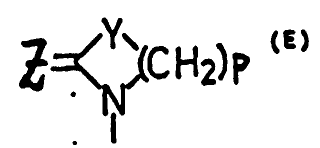 bedeutet, wobei Z für Cyanimino N-CN, cis oder trans Nitromethyliden (c/t) CH-NO₂ oder Nitroimino N-NO₂ steht, Y die oben angegebene Bedeutung hat und p 2 oder 3 bedeutet, und
R₆ in Abhängigkeit von R₅ für die zwei Klassen von Substituenten R₆' und R₆'' steht, wobei, falls R₅ einen Heterocyclus A, B, C oder D bedeutet, R₆ für die Substituentenklasse R₆' steht und
R₆' Difluormethoxy, Trifluormethoxy, Trifluorethoxy, Tetrafluorethoxy, Difluormethylthio, Difluormethylsulfinyl, Difluormethylsulfonyl, Trifluormethylthio, Trifluormethylsulfinyl, Trifluormethylsulfonyl, Trifluorethylthio, Trifluorethylsulfinyl oder Trifluorethylsulfonyl bedeutet,
oder, falls R₅ den Heterocyclus E bedeutet, R₆ für die vorstehende Substituentenklasse R₆' und für die Substituentenklasse R₆'' steht und
R₆'' Cyano, Nitro, C₁₋₆-Alkyl, C₃₋₈-Cycloalkyl, Formyl und C₁₋₆-Alkylcarbonyl bedeutet, wobei sich der Heterocyclus R₅ in trans-Stellung zu dem Rest R₃ befindet, wenn R₃ und R₄ nicht zusammen eine Bindung bedeuten, sondern R₄ für Wasserstoff steht, sowie deren Salzen und Säureadditionssalzen, Tautomeren und optischen Isomeren,
ausgenommen Verbindungen der Formel (I), bei denen zugleich R₁ und R₂ beide Methyl, R₃ Hydroxyl, R₅ einen heterocyclichen Rest der Formel (C), X Sauerstoff, n 2, 3 oder 4 und R₆ Trifluormethoxy oder Trifluorethoxy bedeuten,
und ausgenommen Verbindungen der Formel (I), bei denen zugleich R₁ und R₂ C₁₋₆-Alkyl oder C₃₋₆-verzweigtes Alkyl, R₃ Hydroxy, R₄ Wasserstoff oder R₃ und R₄ zusammen eine Bindung, R₅ einen Heterocyclus der Formel (E), worin Y für Schwefel, Amino oder substituiertes Amino mit R₇ in der Bedeutung C₁₋₆-Alkyl oder verzweigtes C₃₋₆-Alkyl und Z für Cyanimino stehen, und R₆ Cyano bedeuten.
**dadurch gekennzeichnet, daß man**
a) Oxirane der allgemeinen Formel IIa, in denen R₁, R₂ und R₆' die oben angegebene Bedeutung haben, mit Anionen C⁻ und D⁻ von Heterocyclen der allgemeinen Formel H-C und H-D, in denen X die Bedeutung Sauerstoff und Y, k, m, n die oben angegebene Bedeutung haben, mit der Einschränkung, daß für jene Heterocyclen H-D, für die m ungleich 0 gilt, Y nicht für unsubstituiertes Amino steht, zu Verbindungen der allgemeinen Formel I, in denen R₁, R₂ und R₆' die oben angegebene Bedeutung haben, R₃ für Hydroxy, R₄ für Wasserstoff und R₅ für die Heterocyclenreste C und D, in denen X für Sauerstoff steht und Y, k, m, n die oben angegebene Bedeutung haben, mit der Ausnahme, daß Y nicht für unsubstituiertes Amino steht, falls m ungleich 0 ist, umsetzt, wobei die Substituenten R₃ und R₅ der Verbindungen der Formel I **trans** zueinander angeordnet sind und daß man
b) jene erfindungsgemäße Verbindungen gemäß a) für die Y für substituiertes Amino und m für 1 oder 2 steht, durch Hydrolyse, Hydrogenolyse oder Dealkylierung in Verbindungen der Formel I gemäß a) überführt, in denen Y auch dann für unsubstituiertes Amino steht, falls m ungleich 0 gilt, wobei wiederum R₃ und R₅ **trans** zueinander angeordnet sind und daß man
c) Oxirane der allgemeinen Formel IIa gemäß a) mit Aminen der allgemeinen Formel H-A und H-B zu Verbindungen der Formel I, in denen R₁, R₂ und R₆' die oben angegebene Bedeutung haben und R₅ für A oder 8, mit der für n und Y oben angegebenen Bedeutung steht, R₄ für Wasserstoff und R₃ für Hydroxy steht, umsetzt, wobei R₃ und R₅ trans zueinander angeordnet sind und daß man
d) Oxirane der allgemeinen Formel IIa und IIb, in denen R₁, R₂, R₆' und R₆'' die oben angegebene Bedeutung haben, mit Anionen E⁻ von Heterocyclen der allgemeinen Formel H-E, in denen Y, Z und p die oben angegebene Bedeutung haben, zu Verbindungen der allgemeinen Formel I, in denen R₁, R₂, R₆' und R₆'' die oben angegebene Bedeutung haben, R₃ für Hydroxy, R₄ für Wasserstoff und R₅ für E mit der für Y, Z und p angegebenen Bedeutung steht, umsetzt, wobei R₃ und R₅ trans zueinander angeordnet sind und daß man
e) erfindungsgemäße Verbindungen der Formel I gemäß a) und b) mit Schwefelungsreagentien, z.B. bevorzugt Lawessons Reagens in einem inerten Solvens wie z.B. Toluol zu Verbindungen der Formel I, in denen R₁, R₂, R₃, R₄ und R₆' wie unter a) definiert sind, R₅ für die Reste C, D mit der für Y, k, m, n angegebenen Bedeutung steht, wobei jedoch X für Schwefel steht, umsetzt und daß man
f) erfindungsgemäße Verbindungen der Formel I gemäß a), d) und e), sowie jene erfindungsgemäßen Verbindungen qemäß c), bei denen Y nicht für unsubstituiertes Amino steht, durch Umsetzung mit Alkylhalogeniden, -mesylaten, -brosylaten, -tosylaten, Acylhalogeniden, -anhydriden, -imidazoliden, Alkylcarbonylhalogeniden oder -anhydriden, Mono- oder Dialkylaminocarbonylhalogeniden, Phosgen oder Alkylisocyanaten in Verbindungen der allgemeinen Formel I, in denen R₁, R₂, R₄, R₆', R₆'' und R₅ gemäß a), c), d) und e) definiert sind, mit der Einschränkung, daß Y im Rest B nicht für unsubstituiertes Amino steht, und R₃ für C₁₋₆-Alkoxy, Formyloxy, C₁₋₈-Alkylcarbonyloxy, C₁₋₈-Monoalkylamino, C₁₋₈-Dialkylamlno steht, umwandelt und daß man
g) jene erfindungsgemäßen Verbindungen der Formel I gemäß f), bei denen Y für substituiertes, insbesondere benzylsubstituiertes Amino steht, durch geeignete Maßnahmen, insbesondere Hydrogenolyse in Verbindungen der Formel I gemäß f) überführt, bei denen Y für unsubstituiertes Amino steht und daß man
h) erfindungsgemäße Verbindungen der Formel I gemäß a), b), d) und e) in Anwesenheit eines dehydratisierenden Agens wie z.B. Natriumhydrid In einem inerten Solvens wie z.B. Tetrahydrofuran zu Verbindungen der allgemeinen Formel I umsetzt, in der R₁, R₂, R₅, R₆' und R₆'' die oben angegebene Bedeutung haben und R₃ zusammen mit R₄ eine Bindung bildet und daß man
i) Oxirane der allgemeinen Formeln IIa bzv. IIb, in denen R₁, R₂ und R₆' bzw. R₆'' die oben angegebene Bedeutung haben unter Anwesenheit von mindestens zwei Äquivalenten einer Base zunächst zu erfindungsgemäßen Verbindungen gemäß a) und d) umsetzt, diese jedoch nicht isoliert, sondern durch Verlängerung der Reaktionszeit und insbesondere durch Erhöhen der Reaktionstemperatur, auf vorzugsweise 40°C, in situ in erfindungsgemäße Verbindungen gemäß h) überführt und diese nach an sich üblichen Verfahren isoliert und daß man
j) jene erfindungsgemäßen Verbindungen gemäß a), b), c), d), f), g) und h), in denen R₆' die Bedeutung Difluormethylthio, Trifluormethylthio und 2,2,2-Trifluorethylthio und gleichzeitig X und Y eine andere Bedeutung als Schwefel hat mit geeigneten 0xidationsmitteln, bevorzugt Oxone^{R} bzw. Wasserstoffperoxid/Eisessig in jene erfindungsgemäße Verbindungen gemäß a), b), c), d), g) und h) überführt, in denen R₆' die Bedeutung Difluormethylsulfinyl, Trifluormethylsulfinyl, 2,2,2-Trifluorethylsulfinyl und insbesondere Trifluormethylsulfonyl, 2,2,2-Trifluorethylsulfonyl, Difluormethylsulfonyl hat bzw. eventuell anfallende Gemische von Sulfinyl- und Sulfonyl-Verbindung durch an sich übliche Verfahren in die reinen Komponenten auftrennt, und/oder
k) erwünschtenfalls erhaltene Verbindungen in Salze oder Säureadditionssalze überführt, und/oder
l) erwünschtenfalls erhaltene Verbindungen in ihre optischen Antipoden auftrennt.

2. Verfahren gemäß Anspruch 1, zur Herstellung von Verbindungen der Formel Ia 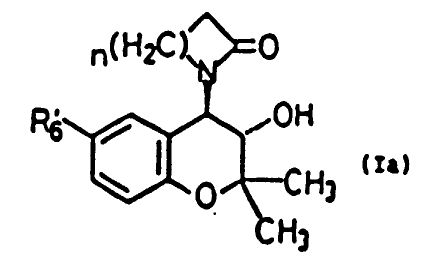 worin n die Zahl 2 oder 3 und R₆' Difluormethoxy, Trifluormethylthio, Difluormethylthio, Trifluormethylsulfonyl, Difluormethylsulfonyl und Tetrafluorethoxy bedeutet.

3. Verfahren gemäß Anspruch 1, zur Herstellung von Verbindungen der Formel Ia' 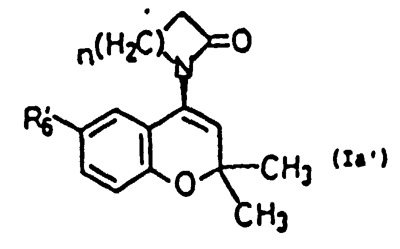 worin n die Zahl 2 oder 3 und R₆' Difluormethoxy, Trifluormethoxy, Trifluormethylthio, Difluormethylthio, Trifluormethylsulfonyl, Difluormethylsulfonyl, Trifluorethoxy und Tetrafluorethoxy bedeutet.

4. Verfahren gemäß Anspruch 1, zur Herstellung von 6-Trifluormethylthio-3,4-dihydro-2,2-dimethyl-trans-4-(2-oxo-1-piperidinyl)-2H-benzo(b)pyran-3-ol,

5. Verfahren gemäß Anspruch 1, worin R₃ und R₄ zusammen eine Bindung bilden, so daß zwischen C₃- und C₄-Position des Benzopyrangerüstes eine Doppelbindung besteht.

6. Verwendung einer Verbindung I nach Anspruch 1 zur Herstellung eines pharmazeutischen Präparates zur Prophylaxe und Behandlung von Bluthochdruck, Herzinsuffizienz, Angina pectoris, arterieller Verschlußkrankheit, cerebraler und peripherer Durchblutungsstörungen, Asthma, cerebraler Insuffizienz, Migräne, Schwindel, Erkrankungen des Innenohrs beziehungsweise des Gehörapparates, erhöhtem Augeninnendruck, Glaukom, Sehschwäche, akuten und chronischen Herzkrankheiten.

7. Verfahren zur Herstellung eines pharmazeutischen Präparates, dadurch gekennzeichnet, daß man eine oder mehrere der Verbindungen der Formel I gemäß Anspruch 1 oder deren physiologisch verträgliche Salze und gegebenenfalls übliche Trägerstoffe undioder Verdünnungsmittel mischt.

8. Verbindungen gemäß Anspruch 1 der Formeln IIa, IIIa und Va 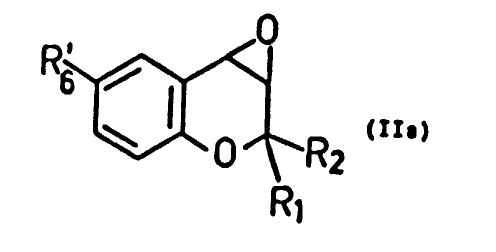 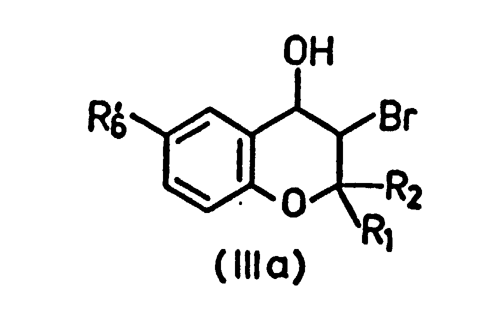 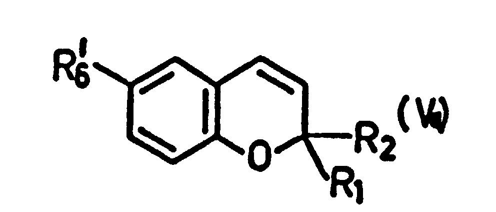 mit der vorstehend angegebenen Bedeutung von R₁, R₂, R₆',
ausgenommen Verbindungen der Formel (IIa),
bei denen zugleich R₁ und R₂ beide Methyl und R₆' Trifluormethoxy oder Trifluorethoxy bedeuten.

## Claims (Claims for the following Contracting State(s): AT, BE, CH, DE, FR, GB, IT, LI, NL, SE)

1. Benzopyran derivatives of the general formula I: 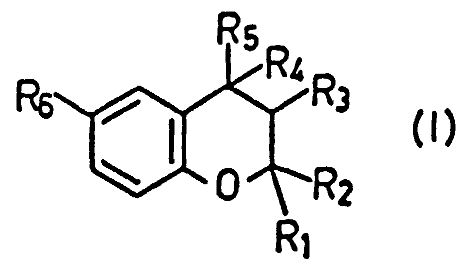 in which
R₁ and R₂, which can be identical or different, are hydrogen, C₁₋₆-alkyl, branched C₃₋₆-alkyl, C₃₋₇-cycloalkyl or, together with the carbon atom by which they are enclosed, C₃₋₇-spiroalkyl,
R₃ is hydroxyl, C₁₋₈-alkoxy, formyloxy, C₁₋₈-alkylcarbonyloxy, C₁₋₈-alkoxycarbonyloxy, C₁₋₈-monoalkylaminocarbonyloxy or C₁₋₈-dialkylaminocarbonyloxy, it being possible for the C₁₋₈-alkyl or -alkoxy groups to be either linear or branched, and
R₄ is hydrogen or
R₃ and R₄ together form a bond,
R₅ is a heterocycle of the formula A: 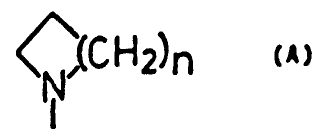
n being 1, 2, 3 or 4,
or a heterocycle of the formula B: 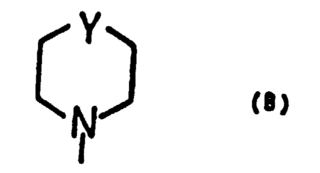
Y being oxygen, sulphur, unsubstituted amino -NH- or substituted amino -NR₇- and R₇ being linear C₁₋₉-alkyl, branched C₃₋₇-alkyl, C₃₋₇-cycloalkyl, linear or branched C₁₋₉-alkyl substituted by C₃₋₇-cycloalkyl, C₁₋₈-alkylcarbonyl, C₁₋₈-alkoxycarbonyl, benzyl, triphenylmethyl, phenyl, benzyloxycarbonyl, phenylcarbonyl or benzylcarbonyl,
or R₅ is a heterocycle of the formula C: 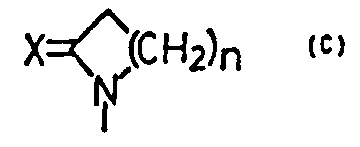
X being oxygen or sulphur and n being 1, 2, 3 or 4,
or a heterocycle of the formula D: 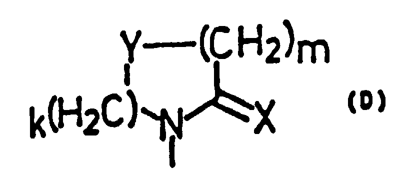 m being 0, 1 or 2 and k being 1, 2 or 3, with the proviso that (m + k) is 1, 2 or 3, and X and Y being defined as indicated for the formulae B and C,
or a heterocycle of the formula E: 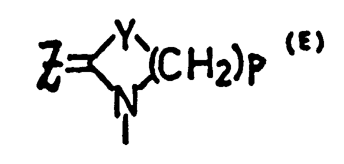
Z being cyanimino N-CN, cis- or trans-nitromethylidene (c/t)-CH-NO₂ or nitroimino N-NO₂, Y being defined as indicated above and p being 2 or 3, and
R₆, depending on R₅, represents the two classes of substituents R₆' and R₆'', wherein, if R₅ is a heterocycle A, B, C or D, R₆ represents the class of substituents R₆' and
R₆' is difluoromethoxy, trifluoromethoxy, trifluoroethoxy, tetrafluoroethoxy, difluoromethylthio, difluoromethylsulphinyl, difluoromethylsulphonyl, trifluoromethylthio, trifluoromethylsulphinyl, trifluoromethylsulphonyl, trifluoroethylthio, trifluoroethylsulphinyl or trifluoroethylsulphonyl,
or, if R₅ is the heterocycle E, R₆ represents the above-mentioned class of substituents R₆' or the class of substituents R₆'' and
R₆'' is cyano, nitro, C₁₋₆-alkyl, C₃₋₈-cycloalkyl, formyl or C₁₋₆-alkylcarbonyl, the heterocycle R₅ being in the trans position to the radical R₃ when R₃ and R₄ together are not a bond but R₄ is hydrogen, and their salts and acid addition salts, tautomers and optical isomers,
with the exception of compounds of the formula (I) in which simultaneously R₁ and R₂ are both methyl, R₃ is hydroxyl, R₅ is a heterocyclic radical of the formula (C), X is oxygen, n is 2, 3 or 4 and R₆ is trifluoromethoxy or trifluoroethoxy,
and with the exception of compounds of the formula (I) in which simultaneously R₁ and R₂ are C₁₋₆-alkyl or branched c₃₋₆-alkyl, R₃ is hydroxyl, R₄ is hydrogen, or R₃ and R₄ together are a bond, R₅ is a heterocycle of the formula (E), Y being sulphur, amino or substituted amino, R₇ being C₁₋₆-alkyl or branched C₃₋₆-alkyl and Z being cyanimino, and R₆ is cyano.

2. Compounds of the formula Ia: 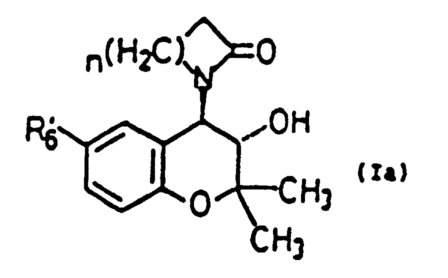 wherein n is the number 2 or 3 and R₆' is difluoromethoxy, trifluoromethylthio, difluoromethylthio, trifluoromethylsulphonyl, difluoromethylsulphonyl or tetrafluoroethoxy.

3. Compounds of the formula Ia': 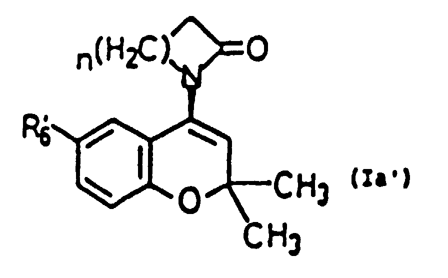 wherein n is the number 2 or 3 and R₆' is difluoromethoxy, trifluoromethoxy, trifluoromethylthio, difluoromethylthio, trifluoromethylsulphonyl, difluoromethylsulphonyl, trifluoroethoxy or tetrafluoroethoxy.

4. Compound according to Claim 1, selected from the group comprising 6-trifluoromethylthio-3,4-dihydro-2,2-dimethyl-trans-4-(2-oxo-1-pyrrolidinyl)-2H-benzo(b)pyran-3-ol, 6-trifluoromethylsulphonyl-3,4-dihydro-2,2-dimethyl-trans-4-(2-oxo-1-pyrrolidinyl)-2H-benzo(b)pyran-3-ol, 6-trifluoromethylsulphonyl-3,4-dihydro-2,2-dimethyl-trans-4-(2-oxo-1-piperidinyl)-2H-benzo(b)pyran-3-ol, 6-difluoromethoxy-3,4-dihydro-2,2-dimethyl-trans-4-(2-oxo-1-piperidinyl)-2H-benzo(b)pyran-3-ol, 6-trifluoromethylthio-2,2-dimethyl-4-(2-oxo-1-pyrrolidinyl)-2H-benzo(b)pyran, 6-trifluoromethylsulphonyl-2,2-dimethyl-4-(2-oxo-1-pyrrolidinyl)-2H-benzo(b)pyran, 6-trifluoromethylthio-2,2-dimethyl-4-(2-oxo-1-piperidinyl)-2H-benzo(b)pyran, 6-trifluoromethylsulphonyl-2,2-dimethyl-4-(2-oxo-1-piperidinyl)-2H-benzo(b)pyran, 6-trifluoromethoxy-2,2-dimethyl-4-(2-oxo-1-piperIdinyl)-2H-benzo(b)pyran and 6-trifluoromethoxy-2,2-dimethyl-4-(2-oxo-1-pyrrolidinyl)-2H-benzo(b)pyran.

5. 6-Trifluoromethylthio-3,4-dihydro-2,2-dimethyl-trans-4-(2-oxo-1-piperidinyl)-2H-benzo(b)pyran-3-ol.

6. Process for the preparation of the compounds according to Claim 1, characterized in that
a) oxiranes of the general formula IIa, in which R₁, R₂ and R₆' are defined as indicated above, are reacted with anions C⁻ and D⁻ of heterocycles of the general formulae H-C and H-D in which X is oxygen and Y, k, m and n are defined as indicated above, with the restriction that for those heterocycles H-D in which m is not equal to 0, Y is not unsubstituted amino, to give compounds of the general formula I in which R₁, R₂ and R₆' are defined as indicated above, R₃ is hydroxyl, R₄ is hydrogen and R₅ is the heterocyclic radicals C and D in which X is oxygen and Y, k, m and n are defined as indicated above, with the exception that Y is not unsubstituted amino if m is not equal to 0, the substituents R₃ and R₅ of the compounds of the formula I being in a trans arrangement relative to one another, and in that
b) compounds of the invention according to a) in which Y is substituted amino and m is 1 or 2 are converted by hydrolysis, hydrogenolysis or dealkylation to compounds of the formula I according to a) in which Y is then also unsubstituted amino if m is not equal to 0, R₃ and R₅ again being in a trans arrangement relative to one another, and in that
c) oxiranes of the general formula IIa according to a) are reacted with amines of the general formulae H-A and H-B to give compounds of the formula I in which R₁, R₂ and R₆' are defined as indicated above, R₅ is A or B, n and Y being defined as indicated above, R₄ is hydrogen and R₃ is hydroxyl, R₃ and R₅ being in a trans arrangement relative to one another, and in that
d) oxiranes of the general formulae IIa and IIb, in which R₁, R₂, R₆' and R₆'' are defined as indicated above, are reacted with anions E⁻ of heterocycles of the general formula H-E, in which Y, Z and p are defined as indicated above, to give compounds of the general formula I in which R₁, R₂, R₆' and R₆'' are defined as indicated above, R₃ is hydroxyl, R₄ is hydrogen and R₅ is E; Y, Z and p being defined as indicated above, R₃ and R₅ being in a trans arrangement relative to one another, and in that
e) compounds of the invention of the formula I according to a) and b) are reacted with thionating reagents, e.g. preferably Lawesson's reagent, in an inert solvent, e.g. toluene, to give compounds of the formula I in which R₁, R₂, R₃, R₄ and R₆' are defined as under a) and R₅ is the radicals C and D, Y, k, m and n being defined as indicated above, except that X is sulphur, and in that
f) compounds of the invention of the formula I according to a), d) and e), as well as compounds of the invention according to c) in which Y is not unsubstituted amino, are converted by reaction with alkyl halides, mesylates, brosylates or tosylates, acyl halides, anhydrides or imidazolides, alkylcarbonyl halides or anhydrides, mono- or dialkylaminocarbonyl halides, phosgene or alkyl isocyanates to compounds of the general formula I in which R₁, R₂, R₄, R₆', R₆'' and R₅ are defined according to a), c), d) and e), with the restriction that Y in the radical B is not unsubstituted amino, and R₃ is C₁₋₆-alkoxy, formyloxy, C₁₋₈-alkylcarbonyloxy, C₁₋₈-monoalkylamino or C₁₋₈-dialkylamino, and in that
g) those compounds of the invention of the formula I according to f) in which Y is substituted amino, especially benzyl-substituted amino, is converted by appropriate procedures, especially hydrogenolysis, to compounds of the formula I according to f) in which Y is unsubstituted amino, and in that
h) compounds of the invention of the formula I according to a), b), d) and e) are reacted in the presence of a dehydrating agent, e.g. sodium hydride, in an inert solvent, e.g. tetrahydrofuran, to give compounds of the general formula I in which R₁, R₂, R₅, R₆' and R₆'' are defined as indicated above and R₃ and R₄ together form a bond, and in that
i) oxiranes of the general formula IIa or IIb, in which R₁, R₂ and R₆' or R₆'' are defined as indicated above, are reacted in the presence of at least two equivalents of a base to give compounds of the invention according to a) and d) in the first instance, these are not isolated but converted in situ to compounds of the invention according to h) by prolonging the reaction time and especially by raising the reaction temperature, preferably to 40°C, and these are isolated by processes which are conventional per se, and in that
j) compounds of the invention according to a), b), c), d), f), g) and h) in which R₆' is difluoromethylthio, trifluoromethylthio or 2,2,2-trifluoroethylthio and simultaneously X and Y are other than sulphur are converted with suitable oxidizing agents, preferably potassium peroxomonosulphate or hydrogen peroxide/glacial acetic acid, to those compounds of the invention according to a), b), c), d), g) and h) in which R₆' is difluoromethylsulphinyl, trifluoromethylsulphinyl, 2,2,2-trifluoroethylsulphinyl or, in particular, trifluoromethylsulphonyl, 2,2,2-trifluoroethylsulphonyl or difluoromethylsulphonyl, and, if mixtures of sulphinyl and sulphonyl compounds are obtained, these are separated into the pure components by processes which are conventional per se.

7. Pharmaceutical preparation, characterized in that it contains one or more of the compounds according to Claim 1 or their biocompatible salts and, if appropriate, conventional excipients and/or diluents.

8. Compounds of the formulae IIa, IIIa and Va: 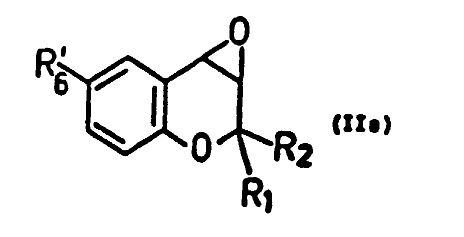 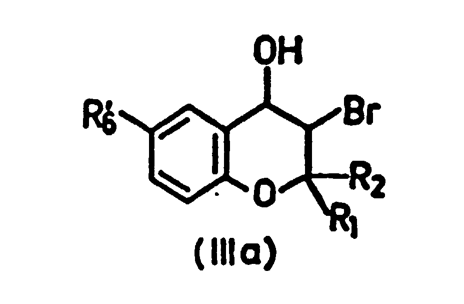 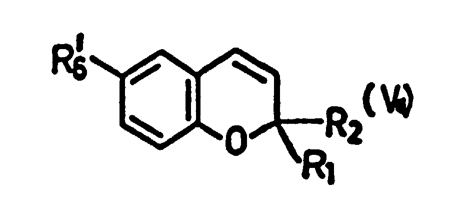 in which R₁, R₂ and R₆' are defined as indicated above,
with the exception of compounds of the formula (IIa) in which simultaneously R₁ and R₂ are both methyl and R₆' is trifluoromethoxy or trifluoroethoxy.

9. Compounds according to Claim 1, characterized in that R₃ and R₄ together form a bond to give a double bond between the C₃ and C₄ positions of the benzopyran skeleton.

## Claims (Claims for the following Contracting State(s): ES)

1. Process for the preparation of benzopyran derivatives of the general formula I: 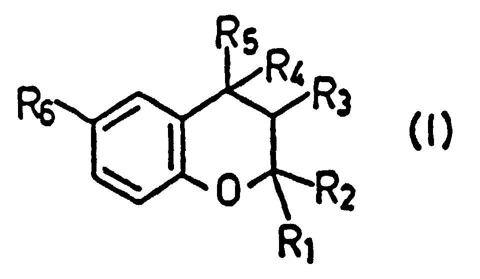 in which
R₁ and R₂, which can be identical or different, are hydrogen, C₁₋₆-alkyl, branched C₃₋₆-alkyl, C₃₋₇-cycloalkyl or, together with the carbon atom by which they are enclosed, C₃₋₇-spiroalkyl,
R₃ is hydroxyl, C₁₋₈-alkoxy, formyloxy, C₁₋₈-alkylcarbonyloxy, C₁₋₈-alkoxycarbonyloxy, C₁₋₈-monoalkylaminocarbonyloxy or C₁₋₈-dialkylaminocarbonyloxy, it being possible for the C₁₋₈-alkyl or -alkoxy groups to be either linear or branched, and
R₄ is hydrogen or
R₃ and R₄ together form a bond,
R₅ is a heterocycle of the formula A: 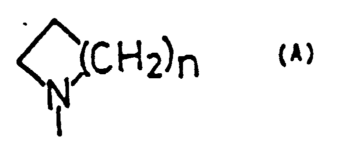
n being 1, 2, 3 or 4,
or a heterocycle of the formula B: 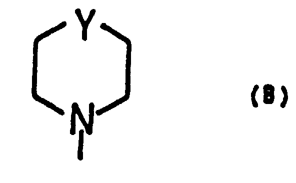
Y being oxygen, sulphur, unsubstituted amino -NH- or substituted amino -NR₇- and R₇ being linear C₁₋₉-alkyl, branched C₃₋₇-alkyl, C₃₋₇-cycloalkyl, linear or branched C₁₋₉-alkyl substituted by C₃₋₇-cycloalkyl, C₁₋₈-alkylcarbonyl, C₁₋₈-alkoxycarbonyl, benzyl, triphenylmethyl, phenyl, benzyloxycarbonyl, phenylcarbonyl or benzylcarbonyl,
or R₅ is a heterocycle of the formula C: 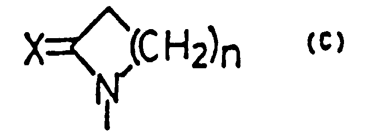
X being oxygen or sulphur and n being 1, 2, 3 or 4,
or a heterocycle of the formula D: 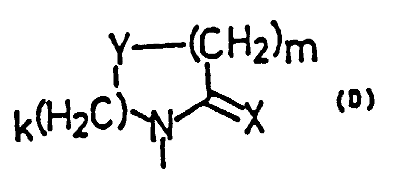
m being 0, 1 or 2 and k being 1, 2 or 3, with the proviso that (m + k) is 1, 2 or 3, and X and Y being defined as indicated for the formulae B and C,
or a heterocycle of the formula E: 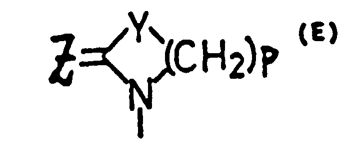
Z being cyanimino N-CN, cis- or trans-nitromethylidene (c/t)-CH-NO₂ or nitroimino N-NO₂, Y being defined as indicated above and p being 2 or 3, and
R₆, depending on R₅, represents the two classes of substituents R₆' and R₆'', wherein, if R₅ is a heterocycle A, B, C or D, R, represents the class of substituents R₆' and
R₆' is difluoromethoxy, trifluoromethoxy, trifluoroethoxy, tetrafluoroethoxy, difluoromethylthio, difluoromethylsulphinyl, difluoromethylsulphonyl, trifluoromethylthio, trifluoromethylsulphinyl, trifluoromethylsulphonyl, trifluoroethylthio, trifluoroethylsulphinyl or trifluoroethylsulphonyl,
or, if R₅ is the heterocycle E, R₆ represents the above-mentioned class of substituents R₆' and the class of substituents R₆'' and
R₆'' is cyano, nitro, C₁₋₆-alkyl, C₃₋₈-cycloalkyl, formyl or C₁₋₆-alkylcarbonyl, the heterocycle R₅ being in the trans position to the radical R₃ when R₃ and R₄ together are not a bond but R₄ is hydrogen, and their salts and acid addition salts, tautomers and optical isomers,
with the exception of compounds of the formula (I) in which simultaneously R₁ and R₂ are both methyl, R₃ is hydroxyl, R₅ is a heterocyclic radical of the formula (C), X is oxygen, n is 2, 3 or 4 and R₆ is trifluoromethoxy or trifluoroethoxy,
and with the exception of compounds of the formula (I) in which simultaneously R₁ and R₂ are C₁₋₆-alkyl or branched C₃₋₆-alkyl, R₃ is hydroxyl, R₄ is hydrogen, or R₃ and R₄ together are a bond, R₅ is a heterocycle of the formula (E), Y being sulphur, amino or substituted amino, R₇ being C₁₋₆-alkyl or branched C₃₋₆-alkyl and Z being cyanimino, and R₆ is cyano,
characterized in that
a) oxiranes of the general formula IIa, in which R₁, R₂ and R₆' are defined as indicated above, are reacted with anions C⁻ and D⁻ of heterocycles of the general formulae H-C and H-D in which X is oxygen and Y, k, m and n are defined as indicated above, with the restriction that for those heterocycles H-D in which m is not equal to 0, Y is not unsubstituted amino, to give compounds of the general formula I in which R₁, R₂ and R₆' are defined as indicated above, R₃ is hydroxyl, R₄ is hydrogen and R₅ is the heterocyclic radicals C and D in which X is oxygen and Y, k, m and n are defined as indicated above, with the exception that Y is not unsubstituted amino if m is not equal to 0, the substituents R₃ and R₅ of the compounds of the formula I being in a trans arrangement relative to one another, and in that
b) compounds of the invention according to a) in which Y is substituted amino and m is 1 or 2 are converted by hydrolysis, hydrogenolysis or dealkylation to compounds of the formula I according to a) in which Y is then also unsubstituted amino if m is not equal to 0, R₃ and R₅ again being in a trans arrangement relative to one another, and in that
c) oxiranes of the general formula IIa according to a) are reacted with amines of the general formulae H-A and H-B to give compounds of the formula I in which R₁, R₂ and R₆' are defined as indicated above, R₅ is A or B, n and Y being defined as indicated above, R₄ is hydrogen and R₃ is hydroxyl, R₃ and R₅ being in a trans arrangement relative to one another, and in that
d) oxiranes of the general formulae IIa and IIb, in which R₁, R₂, R₆' and R₆'' are defined as indicated above, are reacted with anions E⁻ of heterocycles of the general formula H-E, in which Y, Z and p are defined as indicated above, to give compounds of the general formula I in which R₁, R₂, R₆' and R₆'' are defined as indicated above, R₃ is hydroxyl, R, is hydrogen and R₅ is E; Y, Z and p being defined as indicated above, R₃ and R₅ being in a trans arrangement relative to one another, and in that
e) compounds of the invention of the formula I according to a) and b) are reacted with thionating reagents, e.g. preferably Lawesson's reagent, in an inert solvent, e.g. toluene, to give compounds of the formula I in which R₁, R₂, R₃, R₄ and R₆' are defined as under a) and Rs is the radicals C and D; Y, k, m and n being defined as indicated above, except that X is sulphur, and in that
f) compounds of the invention of the formula I according to a), d) and e), as well as compounds of the invention according to c) in which Y is not unsubstituted amino, are converted by reaction with alkyl halides, mesylates, brosylates or tosylates, acyl halides, anhydrides or imidazolides, alkylcarbonyl halides or anhydrides, mono- or dialkylaminocarbonyl halides, phosgene or alkyl isocyanates to compounds of the general formula I in which R₁, R₂, R₄, R₆', R₆'' and R₅ are defined according to a), c), d) and e), with the restriction that Y in the radical B is not unsubstituted amino, and R₃ is C₁₋₆-alkoxy, formyloxy, C₁₋₈-alkylcarbonyloxy, C₁₋₈-monoalkylamino or C₁₋₈-dialkylamino, and in that
g) those compounds of the invention of the formula I according to f) in which Y is substituted amino, especially benzyl-substituted amino, is converted by appropriate procedures, especially hydrogenolysis, to compounds of the formula I according to f) in which Y is unsubstituted amino, and in that
h) compounds of the invention of the formula I according to a), b), d) and e) are reacted in the presence of a dehydrating agent, e.g. sodium hydride, in an inert solvent, e.g. tetrahydrofuran, to give compounds of the general formula I in which R₁, R₂, R₅, R₆' and R₆'' are defined as indicated above and R₃ and R₄ together form a bond, and in that
i) oxiranes of the general formula IIa or IIb, in which R₁, R₂ and R₆' or R₆'' are defined as indicated above, are reacted in the presence of at least two equivalents of a base to give compounds of the invention according to a) and d) in the first instance, these are not isolated but converted in situ to compounds of the invention according to h) by prolonging the reaction time and especially by raising the reaction temperature, preferably to 40°C, and these are isolated by processes which are conventional per se, and in that
j) compounds of the invention according to a), b), c), d), f), g) and h) in which R₆' is difluoromethylthio, trifluoromethylthio or 2,2,2-trifluoroethylthio and simultaneously X and Y are other than sulphur are converted with suitable oxidizing agents, preferably Oxone or hydrogen peroxide/glacial acetic acid, to compounds of the invention according to a), b), c), d), g) and h) in which R₆' is difluoromethylsulphinyl, trifluoromethylsulphinyl, 2,2,2-trifluoroethylsulphinyl or, in particular, trifluoromethylsulphonyl, 2,2,2-trifluoroethylsulphonyl or difluoromethylsulphonyl, and, if mixtures of sulphinyl and sulphonyl compounds are obtained, these are separated into the pure components by processes which are conventional per se, and/or
k) if desired, resulting compounds are converted to salts or acid addition salts, and/or
l) if desired, resulting compounds are resolved into their optical antipodes.

2. Process according to Claim 1 for the preparation of compounds of the formula Ia: 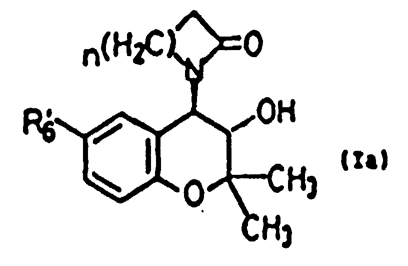 wherein n is the number 2 or 3 and R₆' is difluoromethoxy, trifluoromethylthio, difluoromethylthio, trifluoromethylsulphonyl, difluoromethylsulphonyl or tetrafluoroethoxy.

3. Process according to Claim 1 for the preparation of compounds of the formula Ia': 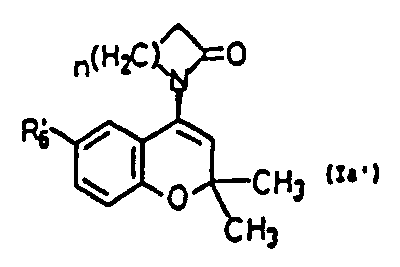 wherein n is the number 2 or 3 and R₆' is difluoromethoxy, trifluoromethoxy, trifluoromethylthio, difluoromethylthio, trifluoromethylsulphonyl, difluoromethylsulphonyl, trifluoroethoxy or tetrafluoroethoxy.

4. Process according to Claim 1 for the preparation of 6-trifluoromethylthio-3,4-dihydro-2,2-dimethyl-trans-4-(2-oxo-1-piperidinyl)-2H-benzo(b)pyran-3-ol.

5. Process according to Claim 1 wherein R₃ and R₄ together form a bond to give a double bond between the C₃ and C₄ positions of the benzopyran skeleton.

6. Use of a compound I according to Claim 1 for obtaining a pharmaceutical preparation for the prophylaxis and treatment of high blood pressure, cardiac insufficiency, angina pectoris, occlusive arterial disease, cerebral and peripheral circulatory disturbances, asthma, cerebral insufficiency, migraine, vertigo, diseases of the inner ear or auditory apparatus, raised intraocular pressure, glaucoma, diminished visual acuity and acute and chronic heart diseases.

7. Process for obtaining a pharmaceutical preparation, characterized in that one or more of the compounds of the formula I according to Claim 1 or their biocompatible salts and, if appropriate, conventional excipients and/or diluents are mixed.

## Claims (Claims for the following Contracting State(s): GR)

1. Process for the preparation of benzopyran derivatives of the general formula I: 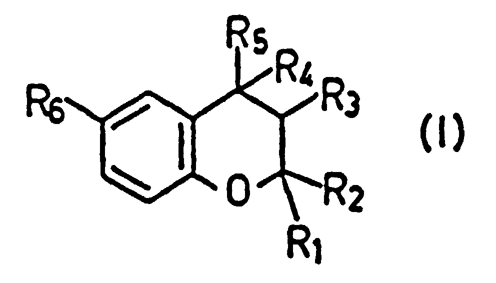 in which
R₁ and R₂, which can be identical or different, are hydrogen, C₁₋₆-alkyl, branched C₃₋₆-alkyl, C₃₋₇-cycloalkyl or, together with the carbon atom to which they are attached, C₃₋₇-spiroalkyl,
R₃ is hydroxyl, C₁₋₈-alkoxy, formyloxy, C₁₋₈-alkylcarbonyloxy, C₁₋₈-alkoxycarbonyloxy, C₁₋₈-monoalkylaminocarbonyloxy or C₁₋₈-dialkylaminocarbonyloxy, it being possible for the C₁₋₈-alkyl or -alkoxy groups to be either linear or branched,
R₄ is hydrogen or
R₃ and R₄ together form a bond,
R₅ is a heterocycle of the formula A: 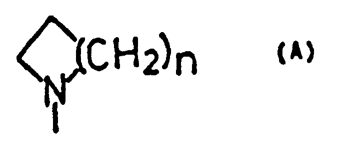
n being 1, 2, 3 or 4,
or a heterocycle of the formula B: 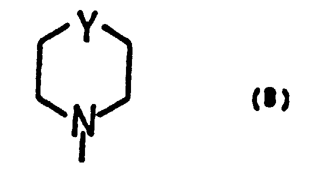
Y being oxygen, sulphur, unsubstituted amino -NH- or substituted amino -NR₇- and R₇ being linear C₁₋₉-alkyl, branched C₃₋₇-alkyl, C₃₋₇-cycloalkyl, linear or branched C₁₋₉-alkyl substituted by C₃₋₇-cycloalkyl, C₁₋₈-alkylcarbonyl, C₁₋₈-alkoxycarbonyl, benzyl, triphenylmethyl, phenyl, benzyloxycarbonyl, phenylcarbonyl or benzylcarbonyl,
or R₅ is a heterocycle of the formula C: 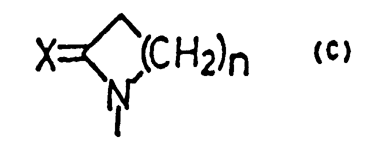
X being oxygen or sulphur and n being 1, 2, 3 or 4,
or a heterocycle of the formula D: 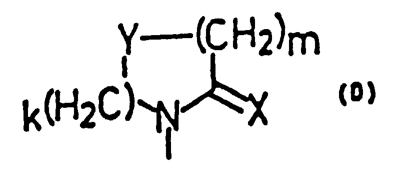
m being 0, 1 or 2 and k being 1, 2 or 3, with the proviso that (m + k) is 1, 2 or 3, and X and Y being defined as indicated for the formulae B and C,
or a heterocycle of the formula E: 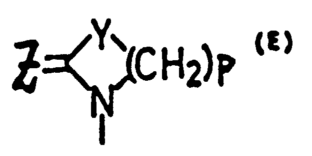
Z being cyanimino N-CN, cis- or trans-nitromethylidene (c/t)-CH-NO₂ or nitroimino N-NO₂, Y being defined as indicated above and p being 2 or 3, and
R₆, depending on R₅, represents the two classes of substituents R₆' and R₆'', wherein, if R₅ is a heterocycle A, B, C or D, R₆ represents the class of substituents R₆' and
R₆' is difluoromethoxy, trifluoromethoxy, trifluoroethoxy, tetrafluoroethoxy, difluoromethylthio, difluoromethylsulphinyl, difluoromethylsulphonyl, trifluoromethylthio, trifluoromethylsulphinyl, trifluoromethylsulphonyl, trifluoroethylthio, trifluoroethylsulphinyl or trifluoroethylsulphonyl,
or, if R₅ is the heterocycle E, R₆ represents the above-mentioned class of substituents R₆' and the class of substituents R₆'' and
R₆'' is cyano, nitro, C₁₋₆-alkyl, C₃₋₈-cycloalkyl, formyl or C₁₋₆-alkylcarbonyl,
the heterocycle R₅ being in the trans position to the radical R₃ when R₃ and R₄ together are not a bond but R₄ is hydrogen,
and their salts and acid addition salts, tautomers and optical isomers,
with the exception of compounds of the formula (I) in which simultaneously R₁ and R₂ are both methyl, R₃ is hydroxyl, R₅ is a heterocyclic radical of the formula (C), X is oxygen, n is 2, 3 or 4 and R, is trifluoromethoxy or trifluoroethoxy,
and with the exception of compounds of the formula (I) in which simultaneously R₁ and R₂ are C₁₋₆-alkyl or branched C₃₋₆-alkyl, R₃ is hydroxyl, R₄ is hydrogen, or R₃ and R₄ together are a bond, R₅ is a heterocycle of the formula (E), Y being sulphur, amino or substituted amino, R₇ being C₁₋₆-alkyl or branched C₃₋₆-alkyl and Z being cyanimino, and R₆ is cyano,
characterized in that
a) oxiranes of the general formula IIa, in which R₁, R₂ and R₆' are defined as indicated above, are reacted with anions C⁻ and D⁻ of heterocycles of the general formulae H-C and H-D in which X is oxygen and Y, k, m and n are defined as indicated above, with the restriction that for heterocycles H-D in which m is not equal to 0, Y is not unsubstituted amino, to give compounds of the general formula I in which R₁, R₂ and R₆' are defined as indicated above, R₃ is hydroxyl, R₄ is hydrogen and R₅ is the heterocyclic radicals C and D in which X is oxygen and Y, k, m and n are defined as indicated above, with the exception that Y is not unsubstituted amino if m is not equal to 0, the substituents R₃ and R₅ of the compounds of the formula I being in a trans arrangement relative to one another, and in that
b) compounds of the invention according to a) in which Y is substituted amino and m is 1 or 2 are converted by hydrolysis, hydrogenolysis or dealkylation to compounds of the formula I according to a) in which Y is then also unsubstituted amino if m is not equal to 0, R₃ and R₅ again being in a trans arrangement relative to one another, and in that
c) oxiranes of the general formula IIa according to a) are reacted with amines of the general formulae H-A and H-B to give compounds of the formula I in which R₁, R₂ and R₆' are defined as indicated above, R₅ is A or B, n and Y being defined as indicated above, R₄ is hydrogen and R₃ is hydroxyl, R₃ and R₅ being in a trans arrangement relative to one another, and in that
d) oxiranes of the general formulae IIa and IIb, in which R₁, R₂, R₆' and R₆'' are defined as indicated above, are reacted with anions E⁻ of heterocycles of the general formula H-E, in which Y, Z and p are defined as indicated above, to give compounds of the general formula I in which R₁, R₂, R₆' and R₆'' are defined as indicated above, R₃ is hydroxyl, R₄ is hydrogen and R₅ is E, Y, Z and p being defined as indicated above, R₃ and R₅ being in a trans arrangement relative to one another, and in that
e) compounds of the invention of the formula I according to a) and b) are reacted with thionating reagents, e.g. preferably Lawesson's reagent, in an inert solvent, e.g. toluene, to give compounds of the formula I in which R₁, R₂, R₃, R₄ and R₆' are defined as under a) and R₅ is the radicals C and D, Y, k, m and n being defined as indicated above, except that X is sulphur, and in that
f) compounds of the invention of the formula I according to a), d) and e), as well as compounds of the invention according to c) in which Y is not unsubstituted amino, are converted by reaction with alkyl halides, mesylates, brosylates or tosylates, acyl halides, anhydrides or imidazolides, alkylcarbonyl halides or anhydrides, mono- or di-alkylaminocarbonyl halides, phosgene or alkyl isocyanates to compounds of the general formula I in which R₁, R₂, R₄, R₆', R₆" and R₅ are defined according to a), c), d) and e), with the restriction that Y in the radical B is not unsubstituted amino, and R₃ is C₁₋₆-alkoxy, formyloxy, C₁₋₈-alkylcarbonyloxy, C₁₋₈-monoalkylamino or C₁₋₈-dialkylamino, and in that
g) compounds of the invention of the formula I according to f) in which Y is substituted amino, especially benzyl-substituted amino, is converted by appropriate procedures, especially hydrogenolysis, to compounds of the formula I according to f) in which Y is unsubstituted amino, and in that
h) compounds of the invention of the formula I according to a), b), d) and e) are reacted in the presence of a dehydrating agent, e.g. sodium hydride, in an inert solvent, e.g. tetrahydrofuran, to give compounds of the general formula I in which R₁, R₂, R₅, R₆' and R₆" are defined as indicated above and R₃ and R₄ together form a bond, and in that
i) oxiranes of the general formula IIa or IIb, in which R₁, R₂ and R₆' or R₆" are defined as indicated above, are reacted in the presence of at least two equivalents of a base to give compounds of the invention according to a) and d) in the first instance, these are not isolated but converted in situ to compounds of the invention according to h) by prolonging the reaction time and especially by raising the reaction temperature, preferably to 40°C, and these are isolated by processes which are conventional per se, and in that
j) compounds of the invention according to a), b), c), d), f), g) and h) in which R₆' is difluoromethylthio, trifluoromethylthio or 2,2,2-trifluoroethylthio and simultaneously X and Y are other than sulphur are converted with suitable oxidizing agents, preferably Oxone® or hydrogen peroxide/glacial acetic acid, to compounds of the invention according to a), b), c), d), g) and h) in which R₆' is difluoromethylsulphinyl, trifluoromethylsulphinyl, 2,2,2-trifluoroethylsulphinyl or, in particular, trifluoromethylsulphonyl, 2,2,2-trifluoroethylsulphonyl or difluoromethylsulphonyl, and, if mixtures of sulphinyl and sulphonyl compounds are obtained, these are separated into the pure components by processes which are conventional per se, and/or
k) if desired, resulting compounds are converted to salts or acid addition salts, and/or
l) if desired, resulting compounds are resolved into their optical antipodes.

2. Process according to Claim 1 for the preparation of compounds of the formula Ia: 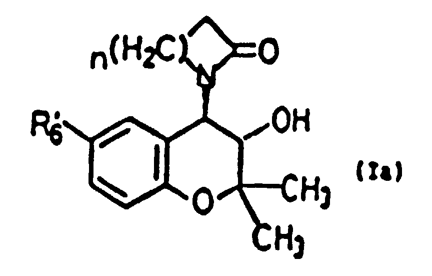 wherein n is the number 2 or 3 and R₆' is difluoromethoxy, trifluoromethylthio, difluoromethylthio, trifluoromethylsulphonyl, difluoromethylsulphonyl or tetrafluoroethoxy.

3. Process according to Claim 1 for the preparation of compounds of the formula Ia': 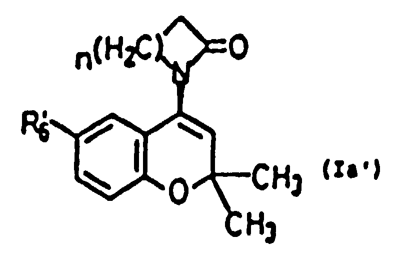 wherein n is the number 2 or 3 and R₆' is difluoromethoxy, trifluoromethoxy, trifluoromethylthio, difluoromethylthio, trifluoromethylsulphonyl, difluoromethylsulphonyl, trifluoroethoxy or tetrafluoroethoxy.

4. Process according to Claim 1 for the preparation of 6-trifluoromethylthio-3,4-dihydro-2,2-dimethyl-trans-4-(2-oxo-1-piperidinyl)-2H-benzo(b)pyran-3-ol.

5. Process according to Claim 1 wherein R₃ and R₄ together form a bond to give a double bond between the C₃ and C₄ positions of the benzopyran skeleton.

6. Use of a compound I according to Claim 1 for obtaining a pharmaceutical preparation for the prophylaxis and treatment of high blood pressure, cardiac insufficiency, angina pectoris, occlusive arterial disease, cerebral and peripheral circulatory disturbances, asthma, cerebral insufficiency, migraine, vertigo, diseases of the inner ear or auditory apparatus, raised intraocular pressure, glaucoma, diminished visual acuity and acute and chronic heart diseases.

7. Process for obtaining a pharmaceutical preparation, characterized in that one or more of the compounds of the formula I according to Claim 1 or their biocompatible salts and, if appropriate, conventional excipients and/or diluents are mixed.

8. Compounds according to Claim 1 of the formulae IIa, IIIa and Va: 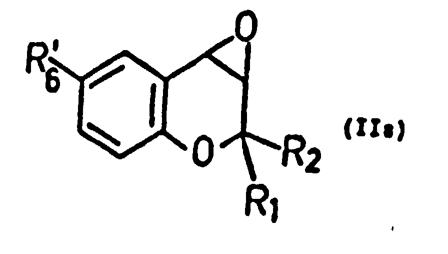 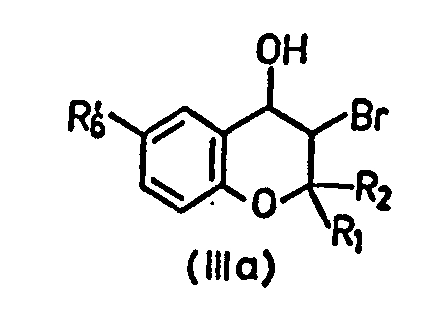 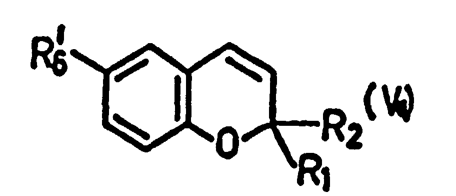 in which R₁, R₂ and R₆' are defined as indicated above,
with the exception of compounds of the formula (IIa) in which simultaneously R₁ and R₂ are both methyl and R₆' is trifluoromethoxy or trifluoroethoxy.

## Revendications (Revendications pour l'(les) Etat(s) contractant(s) suivant(s): AT, BE, CH, DE, FR, GB, IT, LI, NL, SE)

1. Dérivés benzopyraniques répondant à la formule générale I, 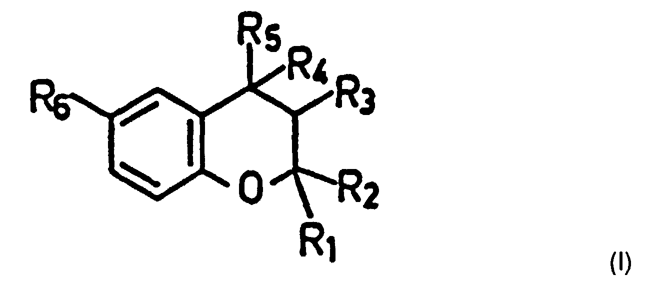 dans laquelle
R₁ et R₂, qui peuvent être identiques ou différents, représentent un atome d'hydrogène, un groupe alkyle en C₁₋₆, un groupe alkyle ramifié en C₃₋₆, un groupe cycloalkyle en C₃₋₇ ou
ensemble avec l'atome de carbone qu'ils entourent, ils signifient un groupe spiroalkyle en C₃₋₇,
R₃ représente un groupe hydroxyle, un groupe alcoxy en C₁₋₈, un groupe formyloxy, un groupe (alkyle en C₁₋₈)carbonyloxy, un groupe (alcoxy en C₁₋₈)carbonyloxy, un groupe mono(alkyle en C₁₋₈)aminocarbonyloxy ou un groupe di(alkyle en C₁₋₈)aminocarbonyloxy, où les groupes alkyles ou alcoxy en C₁₋₈ peuvent être tant linéaires que ramifiés, et
R₄ représente un atome d'hydrogène ou
R₃ et R₄ forment ensemble une liaison,
R₅ représente un hétérocycle répondant à la formule A 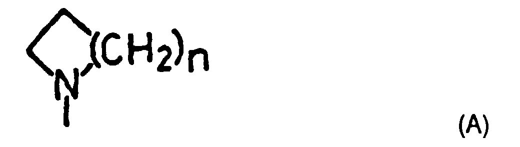
où n vaut 1, 2, 3 ou 4,
ou un hétérocycle répondant à la formule B 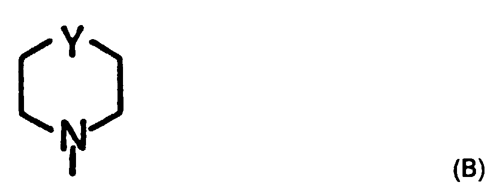
où Y représente un atome d'oxygène, un atome de soufre, un groupe amino -NH- non-substitué, un groupe amino -NR₇- substitué, et R₇ représente un groupe alkyle en C₁₋₉ linéaire, un groupe alkyle en C₃₋₇ ramifié, un groupe cycloalkyle en C₃₋₇, un groupe alkyle en C₁₋₉ linéaire ou ramifié substitué par un groupe cycloalkyle en C₃₋₇, un groupe alkylcarbonyle en C₁₋₈, un groupe alcoxycarbonyle en C₁₋₈, un groupe benzyle, un groupe triphénylméthyle, un groupe phényle, un groupe benzyloxycarbonyle, un groupe phénylcarbonyle ou un groupe benzylcarbonyle,
ou R₅ représente un hétérocycle répondant à la formule C 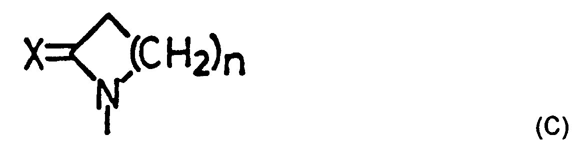
où X représente un atome d'oxygène ou un atome de soufre et n vaut 1, 2, 3 ou 4,
ou un hétérocycle répondant à la formule D 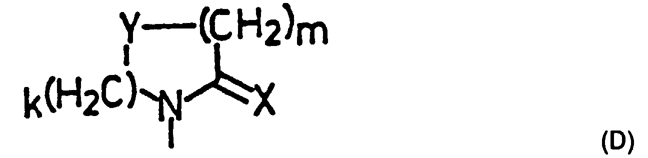
où m vaut 0, 1 ou 2 et k vaut 1, 2 ou 3, mais de telle manière que (m + k) vaut 1, 2 ou 3 et qu'en outre X et Y présentent la signification indiquée pour les formules B et C,
ou un hétérocycle répondant à la formule E 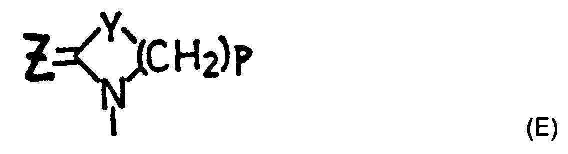
où Z représente un groupe cyanimino N-CN, un groupe nitrométhylidène cis ou trans (c/t) CH-NO₂ ou un groupe nitroimino N-NO₂, Y présente la signification indiquée ci-dessus et p vaut 2 ou 3, et
R₆ représente, en fonction de R₅, les deux classes de substituants R₆' et R₆", où si R₅ représente un hétérocycle A, B, C ou D, R₆ représente la classe de substituants R₆' et
R₆' représente un groupe difluorométhoxy, un groupe trifluorométhoxy, un groupe trifluoroéthoxy, un groupe tétrafluoroéthoxy, un groupe difluorométhylthio, un groupe difluorométhylsulfinyle, un groupe difluorométhylsulfonyle, un groupe trifluorométhylthio, un groupe trifluorométhylsulfinyle, un groupe trifluorométhylsulfonyle, un groupe trifluoroéthylthio, un groupe trifluoroéthylsulfinyle ou un groupe trifluoroéthylsulfonyle,
ou, si R₅ représente l'hétérocycle E, R₆ représente la classe de substituants précédente R₆' et la classe de substituants R₆" et
R₆" représente un groupe cyano, un groupe nitro, un groupe alkyle en C₁₋₆, un groupe cycloalkyle en C₃₋₈, un groupe formyle et un groupe (alkyle en C₁₋₆)carbonyle, l'hétérocycle R₅ se trouvant en position trans par rapport au résidu R₃, si R₃ et R₄ ne signifient pas ensemble une liaison, mais si R₄ représente un atome d'hydrogène, de même que leurs sels et leurs sels d'addition acide, leurs tautomères et leurs isomères optiques,
à l'exception des composés répondant à la formule (I), pour lesquels R₁ et R₂ représentent en même temps un groupe méthyle, R₃ représente un groupe hydroxyle, R₅ représente un résidu hétérocyclique répondant à la formule (C), X représente un atome d'oxygène, n vaut 2, 3 ou 4 et R₆ représente un groupe trifluorométhoxy ou un groupe trifluoroéthoxy,
et à l'exception des composés répondant à la formule (I), dans lesquels R₁ et R₂ représentent en même temps un groupe alkyle en C₁₋₆ ou un groupe alkyle en C₃₋₆ ramifié, R₃ représente un groupe hydroxyle, R₄ représente un atome d'hydrogène ou R₃ et R₄ représentent ensemble une liaison, R₅ représente un hétérocycle répondant à la formule (E), dans laquelle *Y* représente un atome de soufre, un groupe amino ou un groupe amino substitué avec R₇ signifiant un groupe alkyle en C₁₋₆ ou un groupe alkyle en C₃₋₆ ramifié et Z représentant un groupe cyanimino, et R₆ représente un groupe cyano.

2. Composés répondant à la formule Ia : 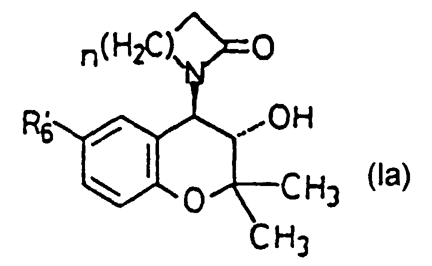 où n représente le nombre 2 ou 3 et R₆' représente un groupe difluorométhoxy, un groupe trifluorométhylthio, un groupe difluorométhylthio, un groupe trifluorométhylsulfonyle, un groupe difluorométhylsulfonyle et un groupe tétrafluoroéthoxy.

3. Composés répondant à la formule Ia' : 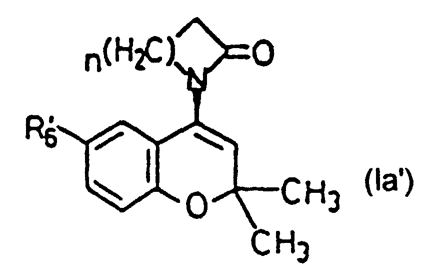 où n représente le nombre 2 ou 3 et R₆' représente un groupe difluorométhoxy, un groupe trifluorométhoxy, un groupe trifluorométhylthio, un groupe difluorométhylthio, un groupe trifluorométhylsulfonyle, un groupe difluorométhylsulfonyle, un groupe trifluoroéthoxy et un groupe tétrafluoroéthoxy.

4. Composé selon la revendication 1, choisi parmi le groupe comprenant le 6-trifluorométhylthio-3,4-dihydro-2,2-diméthyl-trans-4-(2-oxo-1-pyrrolidinyl)-2H-benzo(b)pyran-3-ol, le 6-trifluorométhylsulfonyl-3,4-dihydro-2,2-diméthyl-trans-4-(2-oxo-1-pyrrolidinyl)-2H-benzo(b)pyran-3-ol, le 6-trifluorométhylsulfonyl-3,4-dihydro-2,2-diméthyl-trans-4-(2-oxo-1-pipéridinyl)-2H-benzo(b)pyran-3-ol, le 6-difluorométhoxy-3,4-dihydro-2,2-diméthyl-trans-4-(2-oxo-1-pipéridinyl)-2H-benzo(b)pyran-3-ol, le 6-trifluorométhylthio-2,2-diméthyl-4-(2-oxo-1-pyrrolidinyl)-2H-benzo(b)pyrane, le 6-trifluorométhylsulfonyl-2,2-diméthyl-4-(2-oxo-1-pyrrolidinyl)-2H-benzo(b)pyrane, le 6-trifluorométhylthio-2,2-diméthyl-4-(2-oxo-1-pipéridinyl)-2H-benzo(b)pyrane, le 6-trifluorométhylsulfonyl-2,2-diméthyl-4-(2-oxo-1-pipéridinyl)-2H-benzo(b)pyrane, le 6-trifluorométhoxy-2,2-diméthyl-4-(2-oxo-1-pipéridinyl)-2H-benzo(b)pyrane, le 6-trifluorométhoxy-2,2-diméthyl-4-(2-oxo-1-pyrrolidinyl)-2H-benzo[b]pyrane.

5. 6-Trifluorométhylthio-3,4-dihydro-2,2-diméthyl-trans-4-(2-oxo-1-pipéridinyl)-2H-benzo(b)pyran-3-ol.

6. Procédé de préparation des composés selon la revendication 1, caractérisé en ce que
a) on convertit des oxirannes répondant à la formule générale IIa, dans lesquels R₁, R₂ et R₆' ont la signification indiquée ci-dessus avec des anions C⁻ et D⁻ d'hétérocycles répondant à la formule générale H-C et H-D, dans lesquels X présente la signification d'un atome d'oxygène et Y, k, m, n présentent la signification indiquée ci-dessus, avec la limitation que pour les hétérocycles H-D pour lesquels m est différent de 0, Y ne représente pas un groupe amino non-substitué, en composés répondant à la formule générale I, dans lesquels R₁, R₂ et R₆' présentent la signification indiquée ci-dessus, R₃ représente un groupe hydroxyle, R₄ représente un atome d'hydrogène et R₅ représente les résidus hétérocycliques C et D, dans lesquels X représente un atome d'oxygène et Y, k, m, n présentent la signification indiquée ci-dessus, sauf que Y ne peut pas représenter un groupe amino non-substitué, si m est différent de 0, les substituants R₃ et R₅ des composés répondant à la formule I étant disposés de manière trans l'un par rapport à l'autre, et en ce que
b) on transforme les composés conformes à la présente invention selon le point a) pour lesquels Y représente un groupe amino substitué et m vaut 1 ou 2, par hydrolyse, par hydrogénolyse ou par désalkylation en composés répondant à la formule I selon le point a), dans lesquels Y représente un groupe amino non-substitué, même dans les cas où m est différent de 0, R₃ et R₅ étant de nouveau disposés de manière trans l'un par rapport à l'autre, et en ce que
c) on convertit les oxirannes répondant à la formule générale IIa selon le point a) avec des amines répondant à la formule générale H-A et H-B en composés répondant à la formule I, dans lesquels R₁, R₂ et R₆' présentent la signification indiquée ci-dessus et R₅ représente A ou B, avec la signification indiquée ci-dessus pour n et Y, R₄ représente un atome d'hydrogène et R₃ représente un groupe hydroxyle, R₃ et R₅ étant disposés de manière trans l'un par rapport à l'autre, et en ce que
d) on convertit les oxirannes répondant à la formule générale IIa et IIb, dans lesquels R₁, R₂, R₆' et R₆" présentent la signification indiquée ci-dessus, avec des anions E⁻ d'hétérocycles répondant à la formule générale H-E, dans lesquels Y, Z et p ont la signification indiquée ci-dessus, en composés répondant à la formule générale I, dans lesquels R₁, R₂, R₆' et R₆" présentent la signification indiquée ci-dessus, R₃ représente un groupe hydroxyle, R₄ représente un atome d'hydrogène et R₅ représente E avec la signification indiquée pour Y, Z et p, R₃ et R₅ étant disposés de manière trans l'un par rapport à l'autre, et en ce que
e) on convertit les composés conformes à la présente invention répondant à la formule I selon les points a) et b) avec des réactifs de sulfuration, par exemple de préférence avec le réactif de Lawesson dans un solvant inerte, tel que par exemple le toluène, en composés répondant à la formule I, dans lesquels R₁, R₂, R₃, R₄ et R₆' sont tels que définis au point a), R₅ représente les résidus C, D avec la signification indiquée pour Y, k, m, n, X représentant cependant un atome de soufre, et en ce que
f) on transforme les composés conformes à la présente invention répondant à la formule I selon les points a), d) et e), de même que les composés conformes à la présente invention selon le point c) pour lesquels Y ne représente pas un groupe amino non-substitué, par conversion avec des halogénures d'alkyle, des mésylates d'alkyle, des brosylates d'alkyle, des tosylates d'alkyle, des halogénures d'acyle, des anhydrides d'acyle, des acylimidazolides, des halogénures d'alkylcarbonyle ou des anhydrides d'alkylcarbonyle, des halogénures de mono- ou de dialkylaminocarbonyle, du phosgène ou des isocyanates d'alkyle en composés répondant à la formule générale I, dans lesquels R₁, R₂, R₄, R₆', R₆" et R₅ sont tels que définis conformément aux points a), c), d) et e), avec la limitation que Y dans le résidu B ne représente pas un groupe amino non-substitué, et R₃ représente un groupe alcoxy en C₁₋₆, un groupe formyloxy, un groupe (alkyle en C₁₋₈)carbonyloxy, un groupe mono(alkyle en C₁₋₈)amino, un groupe di(alkyle en C₁₋₈)amino, et en ce que
g) on transforme les composés conformes à la présente invention répondant à la formule I selon le point f) pour lesquels Y représente un groupe amino substitué, en particulier substitué par un groupe benzyle par des mesures appropriées, en particulier par hydrogénolyse, en composés répondant à la formule I selon le point f), pour lesquels Y représente un groupe amino non-substitué, et en ce que
h) on convertit les composés conformes à la présente invention répondant à la formule I selon les points a), b), d) et e) en présence d'un agent de déshydratation, tel que par exemple l'hydrure de sodium dans un solvant inerte, tel que par exemple le tétrahydrofurane, en composés répondant à la formule générale I, dans laquelle R₁, R₂, R₅, R₆' et R₆" présentent la signification indiquée ci-dessus et R₃ ensemble avec R₄ forment une liaison, et en ce que
i) on convertit d'abord les oxirannes répondant aux formules générales IIa, respectivement llb, dans lesquels R₁, R₂ et R₆', respectivement R₆" présentent la signification indiquée ci-dessus, en présence d'au moins deux équivalents d'une base, en composés conformes à la présente invention selon les points a) et d), sans cependant les isoler, mais en les transformant par allongement du temps de réaction et en particulier par augmentation de la température de réaction, de préférence à 40 °C, in situ en composés conformes à la présente invention selon le point h) et en les isolant suivant un procédé courant, et en ce que
j) on transforme les composés conformes à la présente invention selon les points a), b), c), d), f), g) et h) dans lesquels R₆' présente la signification d'un groupe difluorométhylthio, d'un groupe trifluorométhylthio et d'un groupe 2,2,2-trifluoroéthylthio et dans lesquels X et Y présentent simultanément une autre signification que l'atome de soufre avec des agents d'oxydation appropriés, de préférence du peroxomonosulfate de potassium, respectivement du peroxyde d'hydrogène/acide acétique glacial, en composés conformes à la présente invention selon les points a), b), c), d), g) et h) dans lesquels R₆' présente la signification d'un groupe difluorométhylsulfinyle, d'un groupe trifluorométhylsulfinyle, d'un groupe 2,2,2-trifluoroéthylsulfinyle et en particulier d'un groupe trifluorométhylsulfonyle, d'un groupe 2,2,2-trifluoroéthylsulfonyle, d'un groupe difluorométhylsulfonyle, respectivement en ce qu'on sépare les mélanges résultants éventuels de composés sulfinyliques et sulfonyliques par des procédés courants en composants purs.

7. Préparation pharmaceutique, caractérisée en ce qu'elle contient un ou plusieurs composés selon la revendication 1, ou leurs sels physiologiquement acceptables et éventuellement des supports et/ou excipients courants.

8. Composés répondant aux formules IIa, IIIa et Va 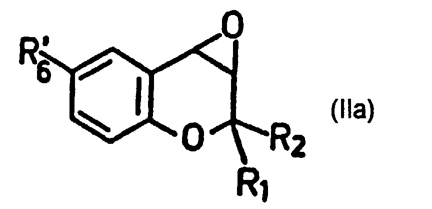 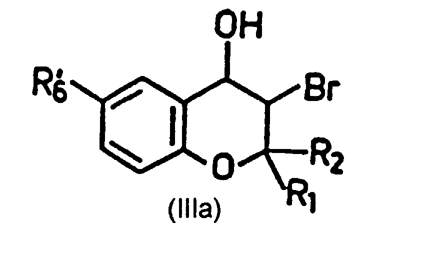 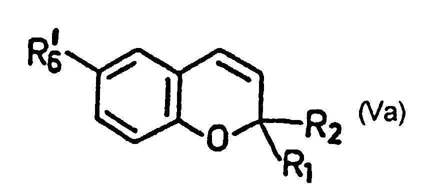 avec les significations indiquées ci-dessus pour R₁, R₂ et R₆', à l'exception des composés répondant à la formule (IIa) pour lesquels R₁ et R₂ représentent en même temps un groupe méthyle et R₆' représente un groupe trifluorométhoxy ou un groupe trifluoroéthoxy.

9. Composés selon la revendication 1, caractérisés en ce que R₃ et R₄ forment ensemble une liaison, de telle manière qu'entre la position C₃ et C₄ du squelette benzopyranique se trouve une double liaison.

## Revendications (Revendications pour l'(les) Etat(s) contractant(s) suivant(s): ES)

1. Procédé pour la préparation de dérivés benzopyraniques répondant à la formule générale I, 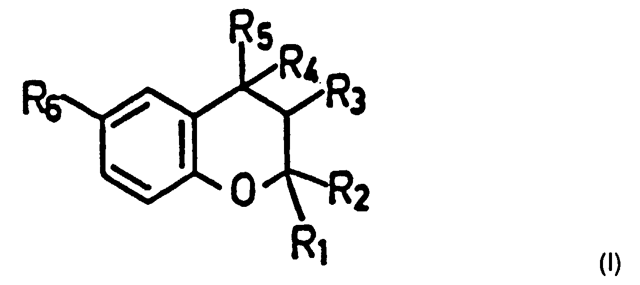 dans laquelle
R₁ et R₂, qui peuvent être identiques ou différents, représentent un atome d'hydrogène, un groupe alkyle en C₁₋₆, un groupe alkyle ramifié en C₃₋₆, un groupe cycloalkyle en C₃₋₇ ou
ensemble avec l'atome de carbone qu'ils entourent, ils signifient un groupe spiroalkyle en C₃₋₇,
R₃ représente un groupe hydroxyle, un groupe alcoxy en C₁₋₈, un groupe formyloxy, un groupe (alkyle en C₁₋₈)carbonyloxy, un groupe (alcoxy en C₁₋₈)carbonyloxy, un groupe mono(alkyle en C₁₋₈)aminocarbonyloxy ou un groupe di(alkyle en C₁₋₈)aminocarbonyloxy, où les groupes alkyles ou alcoxy en C₁₋₈ peuvent être tant linéaires que ramifiés, et
R₄ représente un atome d'hydrogène ou
R₃ et R₄ forment ensemble une liaison,
R₅ représente un hétérocycle répondant à la formule A 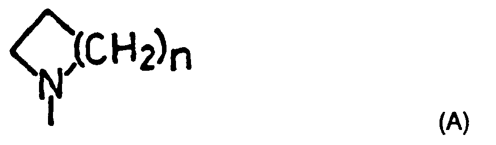
où n vaut 1, 2, 3 ou 4,
ou un hétérocycle répondant à la formule B 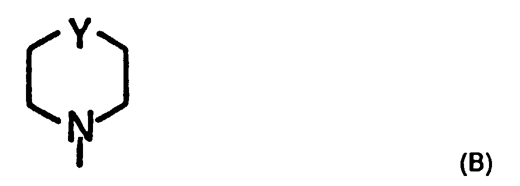
où Y représente un atome d'oxygène, un atome de soufre, un groupe amino -NH- non-substitué, un groupe amino -NR₇- substitué, et R₇ représente un groupe alkyle en C₁₋₉ linéaire, un groupe alkyle en C₃₋₇ ramifié, un groupe cycloalkyle en C₃₋₇, un groupe alkyle en C₁₋₉ linéaire ou ramifié substitué par un groupe cycloalkyle en C₃₋₇, un groupe alkylcarbonyle en C₁₋₈, un groupe alcoxycarbonyle en C₁₋₈, un groupe benzyle, un groupe triphénylméthyle, un groupe phényle, un groupe benzyloxycarbonyle, un groupe phénylcarbonyle ou un groupe benzylcarbonyle,
ou R₅ représente un hétérocycle répondant à la formule C 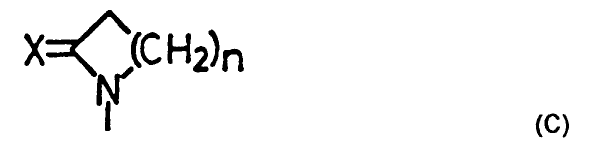
où X représente un atome d'oxygène ou un atome de soufre et n vaut 1, 2, 3 ou 4,
ou un hétérocycle répondant à la formule D 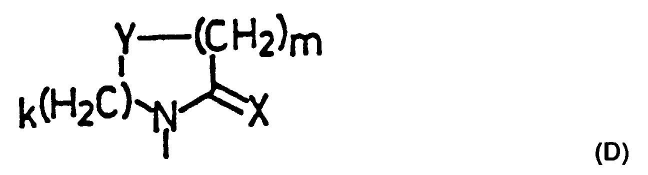 où m vaut 0, 1 ou 2 et k vaut 1, 2 ou 3, mais de telle manière que (m + k) vaut 1, 2 ou 3 et qu'en outre X et Y présentent la signification indiquée pour les formules B et C,
ou un hétérocycle répondant à la formule E 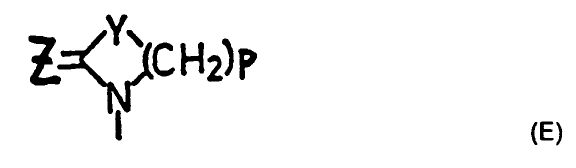
où Z représente un groupe cyanimino N-CN, un groupe nitrométhylidène cis ou trans (c/t) CH-NO₂ ou un groupe nitroimino N-NO₂, Y présente la signification indiquée ci-dessus et p vaut 2 ou 3, et
R₆ représente, en fonction de R₅, les deux classes de substituants R₆' et R₆", où si R₅ représente un hétérocycle A, B, C ou D, R₆ représente la classe de substituants R₆' et
R₆' représente un groupe difluorométhoxy, un groupe trifluorométhoxy, un groupe trifluoroéthoxy, un groupe tétrafluoroéthoxy, un groupe difluorométhylthio, un groupe difluorométhylsulfinyle, un groupe difluorométhylsulfonyle, un groupe trifluorométhylthio, un groupe trifluorométhylsulfinyle, un groupe trifluorométhylsulfonyle, un groupe trifluoroéthylthio, un groupe trifluoroéthylsulfinyle ou un groupe trifluoroéthylsulfonyle,
ou, si R₅ représente l'hétérocycle E, R₆ représente la classe de substituants précédente R₆' et la classe de substituants R₆" et
R₆" représente un groupe cyano, un groupe nitro, un groupe alkyle en C₁₋₆, un groupe cycloalkyle en C₃₋₈, un groupe formyle et un groupe (alkyle en C₁₋₆)carbonyle, l'hétérocycle R₅ se trouvant en position trans par rapport au résidu R₃, si R₃ et R₄ ne signifient pas ensemble une liaison, mais si R₄ représente un atome d'hydrogène, de même que leurs sels et leurs sels d'addition acide, leurs tautomères et leurs isomères optiques,
à l'exception des composés répondant à la formule (I), pour lesquels R₁ et R₂ représentent en même temps un groupe méthyle, R₃ représente un groupe hydroxyle, R₅ représente un résidu hétérocyclique répondant à la formule (C), X représente un atome d'oxygène, n vaut 2, 3 ou 4 et R₆ représente un groupe trifluorométhoxy ou un groupe trifluoroéthoxy,
et à l'exception des composés répondant à la formule (I), dans lesquels R₁ et R₂ représentent en même temps un groupe alkyle en C₁₋₆ ou un groupe alkyle en C₃₋₆ ramifié, R₃ représente un groupe hydroxyle, R₄ représente un atome d'hydrogène ou R₃ et R₄ représentent ensemble une liaison, R₅ représente un hétérocycle répondant à la formule (E), dans laquelle Y représente un atome de soufre, un groupe amino ou un groupe amino substitué avec R₇ signifiant un groupe alkyle en C₁₋₆ ou un groupe alkyle en C₃₋₆ ramifié et Z représentant un groupe cyanimino, et R₆ représente un groupe cyano,
caractérisé en ce que
a) on convertit des oxirannes répondant à la formule générale IIa, dans lesquels R₁, R₂ et R₆' ont la signification indiquée ci-dessus avec des anions C⁻ et D⁻ d'hétérocycles répondant à la formule générale H-C et H-D, dans lesquels X présente la signification d'un atome d'oxygène et Y, k, m, n présentent la signification indiquée ci-dessus, avec la limitation que pour les hétérocycles H-D pour lesquels m est différent de 0, Y ne représente pas un groupe amino non-substitué, en composés répondant à la formule générale I, dans lesquels R₁, R₂ et R₆' présentent la signification indiquée ci-dessus, R₃ représente un groupe hydroxyle, R₄ représente un atome d'hydrogène et R₅ représente les résidus hétérocycliques C et D, dans lesquels X représente un atome d'oxygène et Y, k, m, n présentent la signification indiquée ci-dessus, sauf que Y ne peut pas représenter un groupe amino non-substitué, si m est différent de 0, les substituants R₃ et R₅ des composés répondant à la formule I étant disposés de manière trans l'un par rapport à l'autre, et en ce que
b) on transforme les composés conformes à la présente invention selon le point a) pour lesquels Y représente un groupe amino substitué et m vaut 1 ou 2, par hydrolyse, par hydrogénolyse ou par désalkylation en composés répondant à la formule I selon le point a), dans lesquels Y représente un groupe amino non-substitué, même dans les cas où m est différent de 0, R₃ et R₅ étant de nouveau disposés de manière trans l'un par rapport à l'autre, et en ce que
c) on convertit les oxirannes répondant à la formule générale IIa selon le point a) avec des amines répondant à la formule générale H-A et H-B en composés répondant à la formule I, dans lesquels R₁, R₂ et R₆' présentent la signification indiquée ci-dessus et R₅ représente A ou B, avec la signification indiquée ci-dessus pour n et Y, R₄ représente un atome d'hydrogène et R₃ représente un groupe hydroxyle, R₃ et R₅ étant disposés de manière trans l'un par rapport à l'autre, et en ce que
d) on convertit les oxirannes répondant à la formule générale IIa et IIb, dans lesquels R₁, R₂, R₆' et R₆" présentent la signification indiquée ci-dessus, avec des anions E⁻ d'hétérocycles répondant à la formule générale H-E, dans lesquels Y, Z et p ont la signification indiquée ci-dessus, en composés répondant à la formule générale I, dans lesquels R₁, R₂, R₆' et R₆" présentent la signification indiquée ci-dessus, R₃ représente un groupe hydroxyle, R₄ représente un atome d'hydrogène et R₅ représente E avec la signification indiquée pour Y, Z et p, R₃ et R₅ étant disposés de manière trans l'un par rapport à l'autre, et en ce que
e) on convertit les composés conformes à la présente invention répondant à la formule I selon les points a) et b) avec des réactifs de sulfuration, par exemple de préférence avec le réactif de Lawesson dans un solvant inerte, tel que par exemple le toluène, en composés répondant à la formule I, dans lesquels R₁, R₂, R₃, R₄ et R₆' sont tels que définis au point a), R₅ représente les résidus C, D avec la signification indiquée pour Y, k, m, n, X représentant cependant un atome de soufre, et en ce que
f) on transforme les composés conformes à la présente invention répondant à la formule I selon les points a), d) et e), de même que les composés conformes à la présente invention selon le point c) pour lesquels Y ne représente pas un groupe amino non-substitué, par conversion avec des halogénures d'alkyle, des mésylates d'alkyle, des brosylates d'alkyle, des tosylates d'alkyle, des halogénures d'acyle, des anhydrides d'acyle, des acylimidazolides, des halogénures d'alkylcarbonyle ou des anhydrides d'alkylcarbonyle, des halogénures de mono- ou de dialkylaminocarbonyle, du phosgène ou des isocyanates d'alkyle en composés répondant à la formule générale I, dans lesquels R₁, R₂, R₄, R₆', R₆" et R₅ sont tels que définis conformément aux points a), c), d) et e), avec la limitation que Y dans le résidu B ne représente pas un groupe amino non-substitué, et R₃ représente un groupe alcoxy en C₁₋₆, un groupe formyloxy, un groupe (alkyle en C₁₋₈)carbonyloxy, un groupe mono(alkyle en C₁₋₈)amino, un groupe di(alkyle en C₁₋₈)amino, et en ce que
g) on transforme les composés conformes à la présente invention répondant à la formule I selon le point f) pour lesquels Y représente un groupe amino substitué, en particulier substitué par un groupe benzyle par des mesures appropriées, en particulier par hydrogénolyse, en composés répondant à la formule I selon le point f), pour lesquels Y représente un groupe amino non-substitué, et en ce que
h) on convertit les composés conformes à la présente invention répondant à la formule I selon les points a), b), d) et e) en présence d'un agent de déshydratation, tel que par exemple l'hydrure de sodium dans un solvant inerte, tel que par exemple le tétrahydrofurane, en composés répondant à la formule générale I, dans laquelle R₁, R₂, R₅, R₆' et R₆" présentent la signification indiquée ci-dessus et R₃ ensemble avec R₄ forment une liaison, et en ce que
i) on convertit d'abord les oxirannes répondant aux formules générales IIa, respectivement llb, dans lesquels R₁, R₂ et R₆', respectivement R₆" présentent la signification indiquée ci-dessus, en présence d'au moins deux équivalents d'une base, en composés conformes à la présente invention selon les points a) et d), sans cependant les isoler, mais en les transformant par allongement du temps de réaction et en particulier par augmentation de la température de réaction, de préférence à 40 °C, in situ en composés conformes à la présente invention selon le point h) et en les isolant suivant un procédé courant, et en ce que
j) on transforme les composés conformes à la présente invention selon les points a), b), c), d), f), g) et h) dans lesquels R₆' présente la signification d'un groupe difluorométhylthio, d'un groupe trifluorométhylthio et d'un groupe 2,2,2-trifluoroéthylthio et dans lesquels X et Y présentent simultanément une autre signification que l'atome de soufre avec des agents d'oxydation appropriés, de préférence de l'Oxone®, respectivement du peroxyde d'hydrogène/acide acétique glacial, en composés conformes à la présente invention selon les points a), b), c), d), g) et h) dans lesquels R₆' présente la signification d'un groupe difluorométhylsulfinyle, d'un groupe trifluorométhylsulfinyle, d'un groupe 2,2,2-trifluoroéthylsulfinyle et en particulier d'un groupe trifluorométhylsulfonyle, d'un groupe 2,2,2-trifluoroéthylsulfonyle, d'un groupe difluorométhylsulfonyle, respectivement en ce qu'on sépare les mélanges résultants éventuels de composés sulfinyliques et sulfonyliques par des procédés courants en composants purs, et/ou en ce qu'on
k) on convertit, si on le souhaite, les composés obtenus en sels ou en sels d'addition acide, et/ou en ce que
I) on sépare, si on le souhaite, les composés obtenus en leurs antipodes optiques.

2. Procédé selon la revendication 1, pour la préparation de composés répondant à la formule Ia : 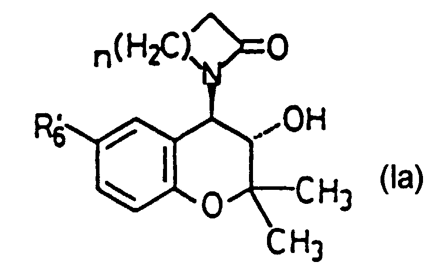 où n représente le nombre 2 ou 3 et R₆' représente un groupe difluorométhoxy, un groupe trifluorométhylthio, un groupe difluorométhylthio, un groupe trifluorométhylsulfonyle, un groupe difluorométhylsulfonyle et un groupe tétrafluoroéthoxy.

3. Procédé selon la revendication 1, pour la préparation de composés répondant à la formule la': 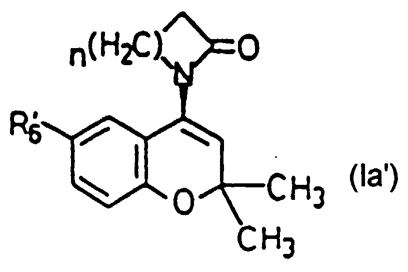 où n représente le nombre 2 ou 3 et R₆' représente un groupe difluorométhoxy, un groupe trifluorométhoxy, un groupe trifluorométhylthio, un groupe difluorométhylthio, un groupe trifluorométhylsulfonyle, un groupe difluorométhylsulfonyle, un groupe trifluoroéthoxy et un groupe tétrafluoroéthoxy.

4. Procédé selon la revendication 1, pour la préparation de 6-trifluorométhylthio-3,4-dihydro-2,2-diméthyl-trans-4-(2-oxo-1-pipéridinyl)-2H-benzo(b)pyran-3-ol.

5. Procédé selon la revendication 1, caractérisé en ce que R₃ et R₄ forment ensemble une liaison, de telle manière qu'entre la position C₃ et C₄ du squelette benzopyranique se trouve une double liaison.

6. Utilisation d'un composé I selon la revendication 1, pour la préparation d'une préparation pharmaceutique pour la prophylaxie et le traitement de l'hypertension, de l'insuffisance cardiaque, de l'angine de poitrine, des occlusions artérielles, des troubles de la circulation sanguine périphérique et cérébrale, de l'asthme, de l'insuffisance cérébrale, de la migraine, du vertige, des maladies de l'oreille interne, respectivement de l'appareil auditif, de l'hypertension intraoculaire, du glaucome, de l'asthénopie et des maladies cardiaques aiguës et chroniques.

7. Procédé pour la préparation d'une préparation pharmaceutique, caractérisé en ce qu'on mélange un ou plusieurs composés selon la revendication 1, ou leurs sels physiologiquement acceptables et éventuellement des supports et/ou excipients courants.

## Revendications (Revendications pour l'(les) Etat(s) contractant(s) suivant(s): GR)

1. Procédé pour la préparation de dérivés benzopyraniques répondant à la formule générale I, 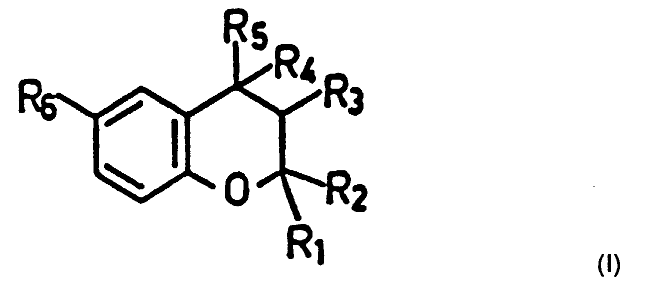 dans laquelle
R₁ et R₂, qui peuvent être identiques ou différents, représentent un atome d'hydrogène, un groupe alkyle en C₁₋₆, un groupe alkyle ramifié en C₃₋₆, un groupe cycloalkyle en C₃₋₇ ou
ensemble avec l'atome de carbone qu'ils entourent, ils signifient un groupe spiroalkyle en C₃₋₇,
R₃ représente un groupe hydroxyle, un groupe alcoxy en C₁₋₈, un groupe formyloxy, un groupe (alkyle en C₁₋₈)carbonyloxy, un groupe (alcoxy en C₁₋₈)carbonyloxy, un groupe mono(alkyle en C₁₋₈)aminocarbonyloxy ou un groupe di(alkyle en C₁₋₈)aminocarbonyloxy, où les groupes alkyles ou alcoxy en C₁₋₈ peuvent être tant linéaires que ramifiés, et
R₄ représente un atome d'hydrogène ou
R₃ et R₄ forment ensemble une liaison,
R₅ représente un hétérocycle répondant à la formule A 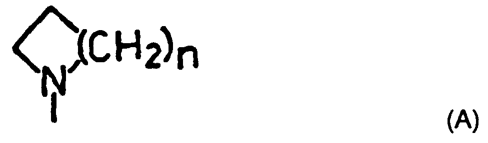
où n vaut 1, 2, 3 ou 4,
ou un hétérocycle répondant à la formule B 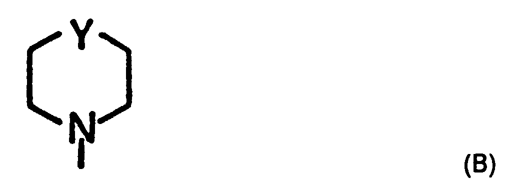
où Y représente un atome d'oxygène, un atome de soufre, un groupe amino -NH- non-substitué, un groupe amino -NR₇- substitué, et R₇ représente un groupe alkyle en C₁₋₉ linéaire, un groupe alkyle en C₃₋₇ ramifié, un groupe cycloalkyle en C₃₋₇, un groupe alkyle en C₁₋₉ linéaire ou ramifié substitué par un groupe cycloalkyle en C₃₋₇, un groupe alkylcarbonyle en C₁₋₈, un groupe alcoxycarbonyle en C₁₋₈, un groupe benzyle, un groupe triphénylméthyle, un groupe phényle, un groupe benzyloxycarbonyle, un groupe phénylcarbonyle ou un groupe benzylcarbonyle,
ou R₅ représente un hétérocycle répondant à la formule C 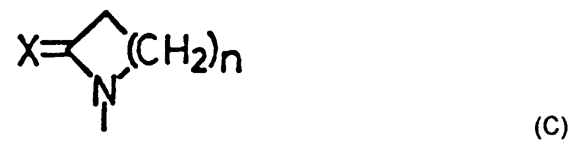
où X représente un atome d'oxygène ou un atome de soufre et n vaut 1, 2, 3 ou 4,
ou un hétérocycle répondant à la formule D 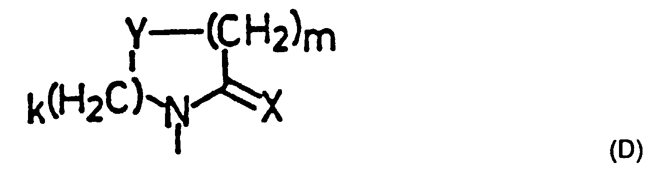
où m vaut 0, 1 ou 2 et k vaut 1, 2 ou 3, mais de telle manière que (m + k) vaut 1, 2 ou 3 et qu'en outre X et Y présentent la signification indiquée pour les formules B et C,
ou un hétérocycle répondant à la formule E 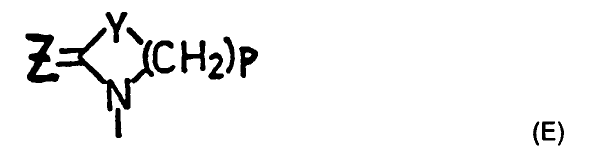
où Z représente un groupe cyanimino N-CN, un groupe nitrométhylidène cis ou trans (c/t) CH-NO₂ ou un groupe nitroimino N-NO₂, Y présente la signification indiquée ci-dessus et p vaut 2 ou 3, et
R₆ représente, en fonction de R₅, les deux classes de substituants R₆' et R₆", où si R₅ représente un hétérocycle A, B, C ou D, R₆ représente la classe de substituants R₆' et
R₆' représente un groupe difluorométhoxy, un groupe trifluorométhoxy, un groupe trifluoroéthoxy, un groupe tétrafluoroéthoxy, un groupe difluorométhylthio, un groupe difluorométhylsulfinyle, un groupe difluorométhylsulfonyle, un groupe trifluorométhylthio, un groupe trifluorométhylsulfinyle, un groupe trifluorométhylsulfonyle, un groupe trifluoroéthylthio, un groupe trifluoroéthylsulfinyle ou un groupe trifluoroéthylsulfonyle,
ou, si R₅ représente l'hétérocycle E, R₆ représente la classe de substituants précédente R₆' et la classe de substituants R₆" et
R₆" représente un groupe cyano, un groupe nitro, un groupe alkyle en C₁₋₆, un groupe cycloalkyle en C₃₋₈, un groupe formyle et un groupe (alkyle en C₁₋₆)carbonyle, l'hétérocycle R₅ se trouvant en position trans par rapport au résidu R₃, si R₃ et R₄ ne signifient pas ensemble une liaison, mais si R₄ représente un atome d'hydrogène, de même que leurs sels et leurs sels d'addition acide, leurs tautomères et leurs isomères optiques,
à l'exception des composés répondant à la formule (I), pour lesquels R₁ et R₂ représentent en même temps un groupe méthyle, R₃ représente un groupe hydroxyle, R₅ représente un résidu hétérocyclique répondant à la formule (C), X représente un atome d'oxygène, n vaut 2, 3 ou 4 et R₆ représente un groupe trifluorométhoxy ou un groupe trifluoroéthoxy,
et à l'exception des composés répondant à la formule (I), dans lesquels R₁ et R₂ représentent en même temps un groupe alkyle en C₁₋₆ ou un groupe alkyle en C₃₋₆ ramifié, R₃ représente un groupe hydroxyle, R₄ représente un atome d'hydrogène ou R₃ et R₄ représentent ensemble une liaison, R₅ représente un hétérocycle répondant à la formule (E), dans laquelle Y représente un atome de soufre, un groupe amino ou un groupe amino substitué avec R₇ signifiant un groupe alkyle en C₁₋₆ ou un groupe alkyle en C₃₋₆ ramifié et Z représentant un groupe cyanimino, et R₆ représente un groupe cyano,
caractérisé en ce que
a) on convertit des oxirannes répondant à la formule générale IIa, dans lesquels R₁, R₂ et R₆' ont la signification indiquée ci-dessus avec des anions C⁻ et D⁻ d'hétérocycles répondant à la formule générale H-C et H-D, dans lesquels X présente la signification d'un atome d'oxygène et Y, k, m, n présentent la signification indiquée ci-dessus, avec la limitation que pour les hétérocycles H-D pour lesquels m est différent de 0, Y ne représente pas un groupe amino non-substitué, en composés répondant à la formule générale I, dans lesquels R₁, R₂ et R₆' présentent la signification indiquée ci-dessus, R₃ représente un groupe hydroxyle, R₄ représente un atome d'hydrogène et R₅ représente les résidus hétérocycliques C et D, dans lesquels X représente un atome d'oxygène et Y, k, m, n présentent la signification indiquée ci-dessus, sauf que Y ne peut pas représenter un groupe amino non-substitué, si m est différent de 0, les substituants R₃ et R₅ des composés répondant à la formule I étant disposés de manière trans l'un par rapport à l'autre, et en ce que
b) on transforme les composés conformes à la présente invention selon le point a) pour lesquels Y représente un groupe amino substitué et m vaut 1 ou 2, par hydrolyse, par hydrogénolyse ou par désalkylation en composés répondant à la formule I selon le point a), dans lesquels Y représente un groupe amino non-substitué, même dans les cas où m est différent de 0, R₃ et R₅ étant de nouveau disposés de manière trans l'un par rapport à l'autre, et en ce que
c) on convertit les oxirannes répondant à la formule générale IIa selon le point a) avec des amines répondant à la formule générale H-A et H-B en composés répondant à la formule I, dans lesquels R₁, R₂ et R₆' présentent la signification indiquée ci-dessus et R₅ représente A ou B, avec la signification indiquée ci-dessus pour n et Y, R₄ représente un atome d'hydrogène et R₃ représente un groupe hydroxyle, R₃ et R₅ étant disposés de manière trans l'un par rapport à l'autre, et en ce que
d) on convertit les oxirannes répondant à la formule générale IIa et IIb, dans lesquels R₁, R₂, R₆' et R₆" présentent la signification indiquée ci-dessus, avec des anions E⁻ d'hétérocycles répondant à la formule générale H-E, dans lesquels Y, Z et p ont la signification indiquée ci-dessus, en composés répondant à la formule générale I, dans lesquels R₁, R₂, R₆' et R₆" présentent la signification indiquée ci-dessus, R₃ représente un groupe hydroxyle, R₄ représente un atome d'hydrogène et R₅ représente E avec la signification indiquée pour Y, Z et p, R₃ et R₅ étant disposés de manière trans l'un par rapport à l'autre, et en ce que
e) on convertit les composés conformes à la présente invention répondant à la formule I selon les points a) et b) avec des réactifs de sulfuration, par exemple de préférence avec le réactif de Lawesson dans un solvant inerte, tel que par exemple le toluène, en composés répondant à la formule I, dans lesquels R₁, R₂, R₃, R₄ et R₆' sont tels que définis au point a), R₅ représente les résidus C, D avec la signification indiquée pour Y, k, m, n, X représentant cependant un atome de soufre, et en ce que
f) on transforme les composés conformes à la présente invention répondant à la formule I selon les points a), d) et e), de même que les composés conformes à la présente invention selon le point c) pour lesquels Y ne représente pas un groupe amino non-substitué, par conversion avec des halogénures d'alkyle, des mésylates d'alkyle, des brosylates d'alkyle, des tosylates d'alkyle, des halogénures d'acyle, des anhydrides d'acyle, des acylimidazolides, des halogénures d'alkylcarbonyle ou des anhydrides d'alkylcarbonyle, des halogénures de mono- ou de dialkylaminocarbonyle, du phosgène ou des isocyanates d'alkyle en composés répondant à la formule générale I, dans lesquels R₁, R₂, R₄, R₆', R₆" et R₅ sont tels que définis conformément aux points a), c), d) et e), avec la limitation que Y dans le résidu B ne représente pas un groupe amino non-substitué, et R₃ représente un groupe alcoxy en C₁₋₆, un groupe formyloxy, un groupe (alkyle en C₁₋₈)carbonyloxy, un groupe mono(alkyle en C₁₋₈)amino, un groupe di(alkyle en C₁₋₈)amino, et en ce que
g) on transforme les composés conformes à la présente invention répondant à la formule I selon le point f) pour lesquels Y représente un groupe amino substitué, en particulier substitué par un groupe benzyle par des mesures appropriées, en particulier par hydrogénolyse, en composés répondant à la formule I selon le point f), pour lesquels Y représente un groupe amino non-substitué, et en ce que
h) on convertit les composés conformes à la présente invention répondant à la formule I selon les points a), b), d) et e) en présence d'un agent de déshydratation, tel que par exemple l'hydrure de sodium dans un solvant inerte, tel que par exemple le tétrahydrofurane, en composés répondant à la formule générale I, dans laquelle R₁, R₂, R₅, R₆' et R₆" présentent la signification indiquée ci-dessus et R₃ ensemble avec R₄ forment une liaison, et en ce que
i) on convertit d'abord les oxirannes répondant aux formules générales IIa, respectivement IIb, dans lesquels R₁, R₂ et R₆', respectivement R₆" présentent la signification indiquée ci-dessus, en présence d'au moins deux équivalents d'une base, en composés conformes à la présente invention selon les points a) et d), sans cependant les isoler, mais en les transformant par allongement du temps de réaction et en particulier par augmentation de la température de réaction, de préférence à 40 °C, in situ en composés conformes à la présente invention selon le point h) et en les isolant suivant un procédé courant, et en ce que
j) on transforme les composés conformes à la présente invention selon les points a), b), c), d), f), g) et h) dans lesquels R₆' présente la signification d'un groupe difluorométhylthio, d'un groupe trifluorométhylthio et d'un groupe 2,2,2-trifluoroéthylthio et dans lesquels X et Y présentent simultanément une autre signification que l'atome de soufre avec des agents d'oxydation appropriés, de préférence de l'Oxone®, respectivement du peroxyde d'hydrogène/acide acétique glacial, en composés conformes à la présente invention selon les points a), b), c), d), g) et h) dans lesquels R₆' présente la signification d'un groupe difluorométhylsulfinyle, d'un groupe trifluorométhylsulfinyle, d'un groupe 2,2,2-trifluoroéthylsulfinyle et en particulier d'un groupe trifluorométhylsulfonyle, d'un groupe 2,2,2-trifluoroéthylsulfonyle, d'un groupe difluorométhylsulfonyle, respectivement en ce qu'on sépare les mélanges résultants éventuels de composés sulfinyliques et sulfonyliques par des procédés courants en composants purs, et/ou en ce qu'on
k) on convertit, si on le souhaite, les composés obtenus en sels ou en sels d'addition acide, et/ou en ce que
I) on sépare, si on le souhaite, les composés obtenus en leurs antipodes optiques.

2. Procédé selon la revendication 1, pour la préparation de composés répondant à la formule Ia : 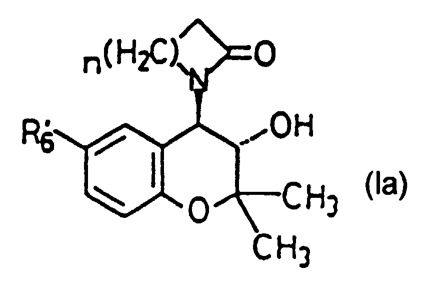 où n représente le nombre 2 ou 3 et R₆' représente un groupe difluorométhoxy, un groupe trifluorométhylthio, un groupe difluorométhylthio, un groupe trifluorométhylsulfonyle, un groupe difluorométhylsulfonyle et un groupe tétrafluoroéthoxy.

3. Procédé selon la revendication 1, pour la préparation de composés répondant à la formule la': 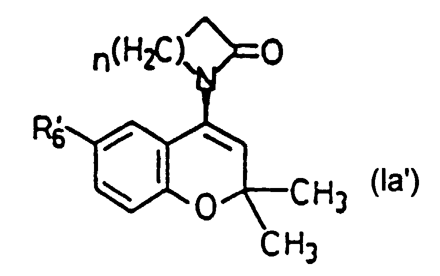 où n représente le nombre 2 ou 3 et R₆' représente un groupe difluorométhoxy, un groupe trifluorométhoxy, un groupe trifluorométhylthio, un groupe difluorométhylthio, un groupe trifluorométhylsulfonyle, un groupe difluorométhylsulfonyle, un groupe trifluoroéthoxy et un groupe tétrafluoroéthoxy.

4. Procédé selon la revendication 1, pour la préparation de 6-trifluorométhylthio-3,4-dihydro-2,2-diméthyl-trans-4-(2-oxo-1-pipéridinyl)-2H-benzo(b)pyran-3-ol.

5. Procédé selon la revendication 1, caractérisé en ce que R₃ et R₄ forment ensemble une liaison, de telle manière qu'entre la position C₃ et C₄ du squelette benzopyranique se trouve une double liaison.

6. Utilisation d'un composé I selon la revendication 1, pour la préparation d'une préparation pharmaceutique pour la prophylaxie et le traitement de l'hypertension, de l'insuffisance cardiaque, de l'angine de poitrine, des occlusions artérielles, des troubles de la circulation sanguine périphérique et cérébrale, de l'asthme, de l'insuffisance cérébrale, de la migraine, du vertige, des maladies de l'oreille interne, respectivement de l'appareil auditif, de l'hypertension intraoculaire, du glaucome, de l'asthénopie et des maladies cardiaques aiguës et chroniques.

7. Procédé pour la préparation d'une préparation pharmaceutique, caractérisé en ce qu'on mélange un ou plusieurs composés selon la revendication 1, ou leurs sels physiologiquement acceptables et éventuellement des supports et/ou excipients courants.

8. Composés selon la revendication 1, répondant aux formules IIa, Illa et Va 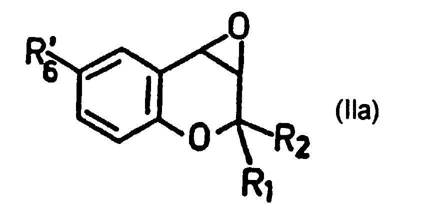 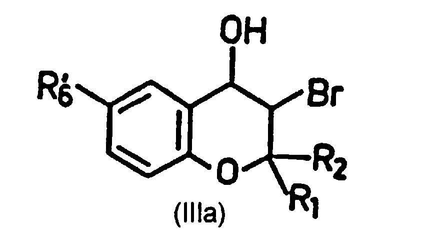 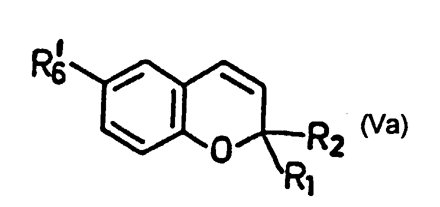 avec les significations indiquées ci-dessus pour R₁, R₂ et R₆', à l'exception des composés répondant à la formule (IIa) pour lesquels R₁ et R₂ représentent en même temps un groupe méthyle et R₆' représente un groupe trifluorométhoxy ou un groupe trifluoroéthoxy.
